# EUROPÄISCHE PATENTSCHRIFT

(11) **EP 2 054 394 B1**
(45) Veröffentlichungstag und Bekanntmachung des Hinweises auf die Patenterteilung: **18.11.2009**
(21) Anmeldenummer: 08760876.6
(22) Anmeldetag: 11.06.2008
(51) Int. Cl.: C07D 241/08, A01N 43/60

(54) **PIPERAZINVERBINDUNGEN MIT HERBIZIDER WIRKUNG**
PIPERAZINE COMPOUNDS WITH A HERBICIDAL ACTION
COMPOSÉS PIPÉRAZINIQUES À ACTION HERBICIDE

(30) Priorität: 12.06.2007 EP 07110125
(43) Veröffentlichungstag der Anmeldung: 06.05.2009
(73) Patentinhaber: BASF SE, 67056 Ludwigshafen (DE)
(72) Erfinder: HUPE, Eike, 68161 Mannheim (DE); SEITZ, Thomas, 68519 Viernheim (DE); WITSCHEL, Matthias, 67098 Bad Dürkheim (DE); SONG, Dschun, 68167 Mannheim (DE); MOBERG, William Karl, 67454 Hassloch (DE); PARRA RAPADO, Liliana, 77654 Offenburg (DE); STELZER, Frank, 68309 Mannheim (DE); VESCOVI, Andrea, 68167 Mannheim (DE); NEWTON, Trevor William, 67435 Neustadt (DE); REINHARD, Robert, 67117 Limburgerhof (DE); SIEVERNICH, Bernd, 67454 Hassloch (DE); GROSSMANN, Klaus, 67141 Neuhofen (DE); EHRHARDT, Thomas, 67346 Speyer (DE); RACK, Michael, 69214 Eppelheim (DE); KIBLER, Elmar, 67454 Hassloch (DE)
(86) Internationale Anmeldenummer: PCT/EP2008/057328
(87) Internationale Veröffentlichungsnummer: WO 2008/152072

(56) Entgegenhaltungen:
- WO-A-2007/077201
- US-A1- 2003 171 379
- KING R R ET AL: "Herbicidal Properties of the Thaxtomin Group of Phytotoxins" JOURNAL OF AGRICULTURAL AND FOOD CHEMISTRY, AMERICAN CHEMICAL SOCIETY. WASHINGTON, US, Bd. 49, 18. April 2001 (2001-04-18), Seiten 2298-2301, XP002370714 ISSN: 0021-8561
- DAVID L. COFFEN, DAVID A. KATONAK, NELS R. NELSON, FRED D. SANCILIO: "A short synthesis of aromatic analogues of the aranotins" J. ORG. CHEM., Bd. 42, Nr. 6, 1977, Seiten 948-952, XP002500804
- MANFRED AUGUSTIN: "Über 2,5-Diketopiperazine II. Die Umsetzung des 2,5-Diketopiperazins mit Aldehyden und Nitrosoverbindungen" J. PRAKT. CHEM., Bd. 4, Nr. 32, 1966, Seiten 158-166, XP002500805
- WANG L X ET AL: "A RAPID AND EFFECTIVE METHOD FOR THE DEACYLATION OF 1,4-DIACETYL-2,5-PIPERAZINEDIONE AND ITS DERIVATIVES" CHINESE CHEMICAL LETTERS, INSTITUTE OF MATERIA MEDICA, CN, Bd. 4, Nr. 8, 1. Januar 1993 (1993-01-01), Seite 687/688, XP008061016 ISSN: 1001-8417

## Beschreibung

Die vorliegende Erfindung betrifft Piperazinverbindungen der im Folgenden definierten allgemeinen Formel I und deren Verwendung als Herbizide. Die Erfindung betrifft außerdem Mittel für den Pflanzenschutz und ein Verfahren zur Bekämpfung von unerwünschtem Pflanzenwuchs.

Bei den von dem Pflanzenpathogen S. scabies produzierten Thaxtominen A und B (King R. R. et al., J. Agric. Food Chem. (1992) 40, 834-837) handelt es sich um Naturstoffe mit einem zentralen Piperazin-2,5-dion-Ring, der in der 3-Position einen 4-Nitro-indol-3-ylmethyl-Rest und in der 2-Position einen gegebenenfalls durch OH substituierten Benzyl-Rest trägt. Aufgrund ihrer pflanzenschädigenden Wirkung wurde auch die Verwendungsmöglichkeit dieser Verbindungsklasse als Herbizide untersucht (King R. R. et al., J. Agric. Food Chem. (2001) 49, 2298-2301).

Die EP-A 181152 und EP-A 243122 beschreiben strukturell ähnliche Piperazinverbindungen und ihre Verwendung als Antagonisten des Platelet Activating Factor.

Die WO 99/48889, WO 01/53290 und WO 2005/011699 beschreiben 2,5-Diketopiperazinverbindungen, die in der 3- bzw. 6-Position einen über eine Methylen- oder Methingruppe gebundenen 4-Imidazolyl-Rest und in der anderen 3- bzw. 6-Position einen Benzyl- oder Benzylidenrest aufweisen. Bei diesen Verbindungen handelt es sich um Antitumor-Wirkstoffe.

Die US 2003/0171379 A1 beschreibt die Verwendung von Mactanamid, einem fungistatischen Diketopiperazin der Formel A, 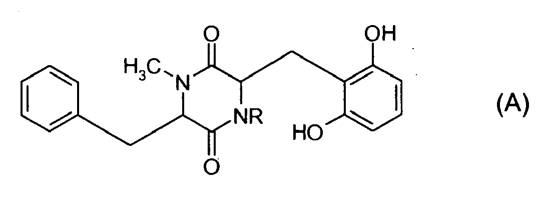 worin R für H oder Methyl steht, als entzündungshemmenden Wirkstoff in der Medizin.

Die ältere Patentanmeldung PCT/EP2006/070271 (= WO 2007/077201) beschreibt 2,5-Diketopiperazin-Verbindungen, die in der 3-Position und der 6-Position jeweils einen über eine Methylengruppe verknüpften Aryl- oder Hetarylrest aufweisen.

Eine Aufgabe der vorliegenden Erfindung ist die Bereitstellung von Verbindungen mit herbizider Wirkung. Insbesondere sollen Verbindungen zur Verfügung gestellt werden, die eine hohe herbizide Wirkung, insbesondere bereits bei niedrigen Aufwandmengen, aufweisen und deren Verträglichkeit gegenüber Kulturpflanzen für eine kommerzielle Verwertung hinreichend ist.

Diese und weitere Aufgaben werden durch die im Folgenden definierten Verbindungen der Formel I und durch ihre landwirtschaftlich geeigneten Salze gelöst.

Gegenstand der vorliegenden Erfindung sind somit Piperazinverbindungen der allgemeinen Formel I 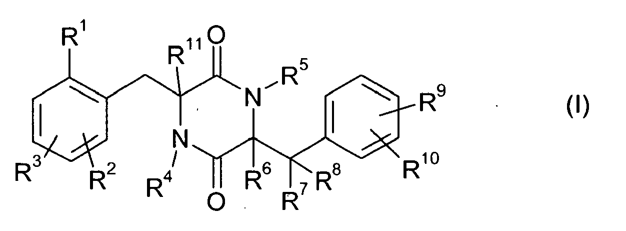 worin
- R¹: ausgewählt ist unter Halogen, Cyano, Nitro, Z-C(=O)-R¹², Phenyl und einem 5- oder 6-gliedrigen heterocyclischen Rest, der 1, 2, 3 oder 4 Heteroatom, ausge- wählt unter O, N und S als Ringatome aufweist, worin Phenyl und der heterocyc- lische Rest unsubstituiert sind oder 1, 2, 3 oder 4 Substituenten R^{1a} aufweisen können, die unabhängig voneinander unter Halogen, CN, NO₂, C₁-C₄-Alkyl, C₁- C₄-Haloalkyl, C₁-C₄-Alkoxy und C₁-C₄-Haloalkoxy ausgewählt sind, und worin Z für eine kovalente Bindung oder eine CH₂-Gruppe steht; R¹² Wasserstoff, C₁-C₆-Alkyl, C₃-C₆-Cycloalkyl, C₂-C₆-Alkenyl, C₅-C₆- Cycloalkenyl, C₂-C₆-Alkinyl, Hydroxy, C₁-C₆-Alkoxy, C₃-C₆-Alkenyloxy, C₃- C₆-Alkinyloxy, Amino, C₁-C₆-Alkylamino, [Di-(C₁-C₆)-alkyl]amino, C₁-C₆- Alkoxyamino, C₁-C₆-Alkylsulfonylamino, C₁-C₆-Alkylaminosulfonylamino, [Di-(C₁-C₆)-alkylamino]sulfonylamino, C₃-C₆-Alkenylamino, C₃-C₆- Alkinylamino, N-(C₂-C₆-Alkenyl)-N-(C₁-C₆-alkyl)-amino, N-(C₂-C₆-Alkinyl)-N- (C₁-C₆-alkyl)-amino, N-(C₁-C₆-Alkoxy)-N-(C₁-C₆-alkyl)-amino, N-(C₂-C₆- Alkenyl)-N-(C₁-C₆-alkoxy)-amino, N-(C₂-C₆-Alkinyl)-N-(C₁-C₆-alkoxy)-amino, Phenyl, Phenoxy oder Phenylamino bedeutet; wobei die Alkylteile in den unter R¹² aufgeführten Resten teileweise oder vollständig halogeniert sein können und die Phenylteile in den unter R¹² aufgeführten Resten 1, 2, 3 oder 4 Susbstituenten R^{12a} tragen können, die unter Halogen, CN, NO₂, C₁-C₄-Alkyl, C₁-C₄-Haloalkyl, C₁-C₄-Alkoxy und C₁-C₄-Haloalkoxy ausgewählt sind;
- R²: Wasserstoff, Halogen, Nitro, Cyano, C₁-C₄-Alkyl, C₁-C₄-Haloalkyl, C₂-C₄-Alkenyl, C₁-C₄-Alkoxy, C₁-C₄-Haloalkoxy, Benzyl oder eine Gruppe S(O)ₙR²¹ bedeutet, worin R²¹ fürC₁-C₄-Alkyl oder C₁-C₄-Haloalkyl steht und n für 0, 1 oder 2 steht;
- R³: Wasserstoff oder Halogen bedeutet;
- R⁴: C₁-C₄-Alkyl, C₃-C₄-Alkenyl oder C₃-C₄-Alkinyl bedeutet;
- R⁵: Wasserstoff, C₁-C₄-Alkyl, C₃-C₄-Alkenyl, C₃-C₄-Alkinyl oder eine Gruppe C(=O)⁵¹ bedeutet, worin R⁵¹ für Wasserstoff, C₁-C₄-Alkyl, C₁-C₄-Haloalkyl, C₁- C₄-Alkoxy oder C₁-C₄-Haloalkoxy steht;
- R⁶: für C₁-C₄-Alkyl, C₁-C₄-Hydroxyalkyl oder C₁-C₄-Haloalkyl steht;
- R⁷, R⁸: unabhängig voneinander für Wasserstoff, OH, C₁-C₄-Alkoxy, C₁-C₄-Haloalkyoxy, C₁-C₄-Alkyl oder C₁-C₄-Haloalkyl stehen;
- R⁹, R¹⁰: unabhängig voneinander ausgewählt sind unter Wasserstoff, Halogen, CN, NO₂, C₁-C₄-Alkyl, C₁-C₄-Haloalkyl, C₂-C₄-Alkenyl, C₁-C₄-Alkoxy und C₁-C₄- Haloalkoxy; und
- R¹¹: Wasserstoff oder C₁-C₄-Alkyl bedeutet;
sowie die landwirtschaftlich geeigneten Salze dieser Verbindungen.

Gegenstand der vorliegenden Erfindung ist auch die Verwendung von Piperazinverbindungen der allgemeinen Formel I oder der landwirtschaftlich brauchbaren Salze von Piperazinverbindungen der Formel I als Herbizide, d.h. zur Bekämpfung von Schadpflanzen.

Gegenstand der vorliegenden Erfindung sind auch Mittel, welche mindestens eine Piperazinverbindung der Formel I oder ein landwirtschaftlich brauchbares Salz von I und für die Formulierung von Pflanzenschutzmitteln übliche Hilfsmittel enthalten.

Gegenstand der vorliegenden Erfindung ist außerdem ein Verfahren zur Bekämpfung von unerwünschtem Pflanzenwuchs, bei dem man eine herbizid wirksame Menge mindestens einer Piperazinverbindung der Formel I oder eines landwirtschaftlich brauchbaren Salzes von I auf Pflanzen, deren Samen und/oder deren Lebensraum einwirken läßt.

Die Erfindung betrifft außerdem Verfahren und Zwischenprodukte zur Herstellung von Verbindungen der Formel I.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Die Verbindungen der Formel I können bezüglich der Anordnung der beiden benzylischen Gruppen in der 3- und der 6-Position cis oder trans-Anordnung aufweisen. Gegenstand der Erfindung sind sowohl die reinen cis-Isomere und trans-Isomere als auch deren Gemische.

Die Verbindungen der Formel I weisen sowohl in der 3-Position als auch in der 6-Position des Piperazinrings jeweils ein Chiralitätszentrum auf. Daher existiert Verbindung I in Form vier verschiedener Konfigurationsisomere (S,S)-I, (R,R)-I, (R,S)-I und (S,R)-I, wobei jeweils 2 dieser Konfigurationsisomere sich zueinander wie Bild und Spiegelbild verhalten, wie in der folgenden Darstellung gezeigt. Die Verbindung I kann daher in Form der reinen Enantiomere, sowie als Enantiomerengemische, z.B. als Gemisch von (S,S)-I mit (R,R)-I oder als Gemisch von (R,S)-I mit (S,R)-I, oder Diastereomerengemische, z.B. als Gemisch aller 4 Diastereomere, vorliegen.

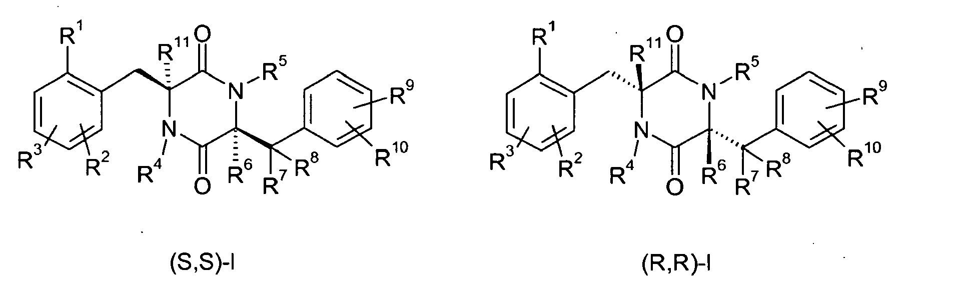

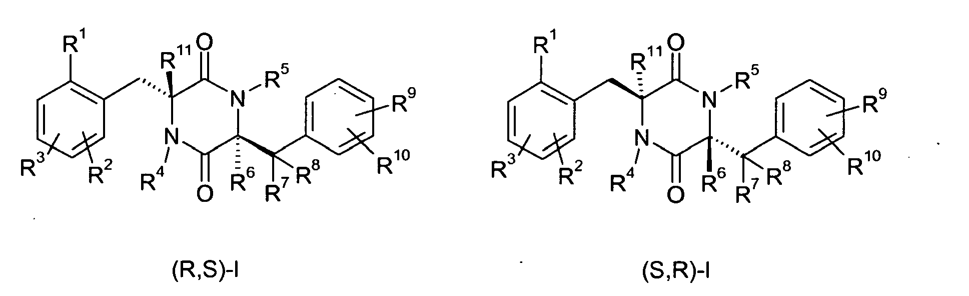

Gegenstand der Erfindung sind sowohl die reinen Enantiomeren oder Diastereomere als auch deren Gemische.

Die Verbindungen der Formel I können auch in Form ihrer landwirtschaftlich brauchbaren Salze vorliegen, wobei es auf die Art des Salzes in der Regel nicht ankommt. Im Allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium oder Kalium, der Erdalkalimetalle, vorzugsweise Calcium oder Magnesium, und der Übergangsmetalle, vorzugsweise Mangan, Kupfer, Zink oder Eisen in Betracht. Ebenso kann als Kation Ammonium verwendet werden, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium. Des Weiteren kommen Phosphoniumionen, Sulfoniumionen, vorzugsweise Tri(C₁-C₄-alkyl)sulfonium oder Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat oder Butyrat.

Die für die Substituenten der erfindungsgemäßen Verbindungen genannten organischen Molekülteile stellen Sammelbegriffe für individuelle Aufzählungen der einzelnen Gruppenmitglieder dar. Sämtliche Kohlenwasserstoffketten, wie Alkyl, Halo(gen)alkyl, Alkenyl, Alkinyl, sowie die Alkylteile und Alkenylteile in Alkoxy, Halo(gen)alkoxy, Alkylamino, Dialkylamino, N-Alkylsulfonylamino, Alkenyloxy, Alkinyloxy, Alkoxyamino, Alkylaminosulfonylamino, Dialkylaminosulfonylamino, Alkenylamino, Alkinylamino, N-(Alkenyl)-N-(alkyl)-amino, N-(Alkinyl)-N-(alkyl)-amino, N-(Alkoxy)-N-(alkyl)-amino, N-(Alkenyl)-N-(alkoxy)-amino oder N-(Alkinyl)-N-(alkoxy)-amino können geradkettig oder verzweigt sein.

Das Präfix Cₙ-Cₘ- gibt die jeweilige Kohlenstoffzahl der Kohlenwasserstoffeinheit an. Sofern nicht anders angegeben tragen halogenierte Substituenten vorzugsweise ein bis fünf gleiche oder verschiedene Halogenatome, insbesondere Fluoratome oder Chloratome.

Die Bedeutung Halogen steht jeweils für Fluor, Chlor, Brom oder lod.

Ferner bedeuten beispielsweise:
Alkyl sowie die Alkylteile beispielsweise in Alkoxy, Alkylamino, Dialkylamino, N-Alkylsulfonylamino, Alkylaminosulfonylamino, Dialkylaminosulfonylamino, N-(Alkenyl)-N-(alkyl)-amino, N-(Alkinyl)-N-(alkyl)-amino, N-(Alkoxy)-N-(alkyl)-amino,: gesättigte, geradkettige oder verzweigte Kohlenwasserstoffreste mit einem oder mehr C-Atomen, z.B. 1 bis 2, 1 bis 4, oder 1 bis 6 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl. In einer erfindungsgemäßen Ausführungsform steht Alkyl für kleine Alkylgruppen wie C₁-C₄-Alkyl. In einer anderen erfindungsgemäßen Ausführungsform steht Alkyl für größere Alkylgruppen wie C₅-C₆-Alkyl.

Halogenalkyl (auch als Haloalkyl bezeichnet): einen Alkylrest wie vorstehend genannt, dessen Wasserstoffatome partiell oder vollständig durch Halogenatome wie Fluor, Chlor, Brom und/oder lod substituiert sind, z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl.

Cycloalkyl sowie die Cycloalkylteile beispielsweise in Cycloalkoxy oder Cycloalkylcarbonyl: monocyclische, gesättigte Kohlenwasserstoffgruppen mit drei oder mehr C-Atomen, z.B. 3 bis 6 Kohlenstoffringgliedern, wie Cyclopropyl, Cyclobutyl, Cyclopentyl und Cyclohexyl.

Alkenyl sowie Alkenylteile beispielsweise in Alkenylamino, Alkenyloxy, N-(Alkenyl)-N-(alkyl)-amino, N-(Alkenyl)-N-(alkoxy)-amino: einfach ungesättigte, geradkettige oder verzweigte Kohlenwasserstoffreste mit zwei oder mehr C-Atomen, z. B. 2 bis 4, 2 bis 6 oder 3 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl.

Cycloalkenyl: monocyclische, einfach ungesättigte Kohlenwasserstoffgruppen mit 5 bis 6, vorzugsweise 5 bis 6 Kohlenstoffringgliedern, wie Cyclopenten-1-yl, Cyclopenten-3-yl, Cyclohexen-1-yl, Cyclohexen-3-yl, Cyclohexen-4-yl.

Alkinyl sowie Alkinylteile beispielsweise in Alkinyloxy, Alkinylamino, N-(Alkinyl)-N-(alkyl)-amino oder N-(Alkinyl)-N-(alkoxy)-amino: geradkettige oder verzweigte Kohlenwasserstoffgruppen mit zwei oder mehr C-Atomen, z. B. 2 bis 4, 2 bis 6, oder 3 bis 6 Kohlenstoffatomen und einer Dreifachbindung in beliebiger Position, z. B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl.

Alkoxy: Alkyl, wie vorstehend definiert, das über ein O-Atom gebunden ist: z. B. Methoxy, Ethoxy, n-Propoxy, 1-Methylethoxy, Butoxy, 1-Methylpropoxy, 2-Methylpropoxy oder 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy.

Aryl: ein- oder mehrkernige aromatische Kohlenwasserstoffreste mit 6 bis 14 Kohlenstoffatomen, wie Phenyl, Naphthyl, Anthracenyl oder Phenanthrenyl, vorzugsweise Phenyl oder Naphthyl.

5- oder 6-gliedrigen heterocyclischen Rest: ein heterocyclischer Rest, der 5 oder 6 Ringatome aufweist wobei 1, 2, 3 oder 4 Ringatome Heteroatome sind, die unter O, S und N ausgewählt sind, wobei der heterocyclische Rest gesättigt, partiell ungesättigt oder aromatisch ist. Beispiele für heterocyclische Reste sind:
C-gebundene, 5-gliedrige, gesättigte Ringe wie Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, Tetrahydropyrazol-3-yl, Tetrahydro-pyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 1,3,2-Dioxathiolan-4-yl;
C-gebundene, 6-gliedrige, gesättigte Ringe wie:
   Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl;
N-gebundene, 5-gliedrige, gesättigte Ringe wie:
   Tetrahydropyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl;
N-gebundene, 6-gliedrige, gesättigte Ringe wie:
   Piperidin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl;
C-gebundene, 5-gliedrige, partiell ungesättigte Ringe wie:
   2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydro-thien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1 H-pyrrol-2-yl, 2,3-Dihydro-1H-pyrrol-3-yl, 2,5-Dihydro-1 H-pyrrol-2-yl, 2,5-Dihydro-1 H-pyrrol-3-yl, 4,5-Dihydro-1 H-pyrrol-2-yl, 4,5-Dihydro-1 H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 4,5-Dihydro-1 H-pyrazol-3-yl, 4,5-Dihydro-1 H-pyrazol-4-yl, 4,5-Dihydro-1H-pyrazol-5-yl, 2,5-Dihyciro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydro-oxazol-2-yl, 4,5-Dihydrooxazol-4-yl, 4,5-Dihydrooxazol-5-yl, 2,5-Dihydro-oxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydro-thiazol-2-yl, 4,5-Dihydrothiazol-4-yl, 4,5-Dihydrothiazol-5-yl, 2,5-Dihydro-thiazol-2-yl, 2,5-Dihydrothiazol-4-yl, 2,5-Dihydrothiazol-5-yl, 2,3-Dihydro-thiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl;
   C-gebundene, 6-gliedrige, partiell ungesättigte Ringe wie: 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydrothiopyran-6-yl, 2H-3,4-Dihydrothiopyran-5-yl, 2H-3,4-Dihydrothiopyran-4-yl, 2H-3,4-Dihydrothiopyran-3-yl, 2H-3,4-Dihydrothiopyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl-, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydro-pyridin-3-yl, 1,2-Dihydropyridin-4-yl, 1,2-Dihydropyridin-5-yl, 1,2-Dihydro-pyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl;
N-gebundene, 5-gliedrige, partiell ungesättigte Ringe wie:
   2,3-Dihydro-1 H-pyrrol-1-yl, 2,5-Dihydro-1 H-pyrrol-1-yl, 4,5-Dihydro-1 H-pyrazol-1-yl, 2,5-Dihydro-1 H-pyrazol-1-yl, 2,3-Dihydro-1 H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1 H-imidazol-1-yl, 2,5-Dihydro-1 H-imidazol-1-yl, 2,3-Dihydro-1 H-imidazol-1-yl, 2,3-Dihydrooxazol-3-yl, 2,3-Dihydrothiazol-3-yl, Oxadiazolin-2-yl, 1,2,4-Δ²-Oxadiazolin-4-yl, 1,2,4-Δ³-Oxadiazolin-2-yl, 1,3,4-Δ²-Oxadiazolin-4-yl, 1,2,4-Δ⁵-Thiadiazolin-2-yl, 1,2,4-Δ³-Thiadiazolin-2-yl, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2,4-Δ³-Triazolin-1-yl, 1,2,4-Δ¹-Triazolin-4-yl;
N-gebundene, 6-gliedrige, partiell ungesättigte Ringe wie:
   1,2,3,4-Tetrahydropyridin-1-yl, 1,2,5,6-Tetrahydropyridin-1-yl, 1,4-Dihydropyridin-1-yl, 1,2-Dihydropyridin-1-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-oxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihdro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;
   C-gebundene, 5-gliedrige, heteroaromatische Ringe wie: 2-Furyl, 3-Furyl, 2-Thienyl, 3-Thienyl, Pyrrol-2-yl, Pyrrol-3-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, [1 H]-Tetrazol-5-yl und [2H]-Tetrazol-5-yl;
C-gebundene, 6-gliedrige, heteroaromatische Ringe wie :
   Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl und 1,2,4-Triazin-6-yl;
N-gebundene, 5-gliedrige, heteroaromatische Ringe wie:
   Pyrrol-1-yl, Pyrazol-1-yl, Imidazol-1-yl, 1,2,3-Triazol-1-yl, 1,2,4-Triazol-1-yl, [1H]-Tetrazol-1-yl und [2H]-Tetrazol-2-yl.

Die vorgenannten Heterocyclen können in der angegebenen Weise substituiert sein. In den vorgenannten Heterocyclen kann ein Schwefelatom zu S=O oder S(=O)₂ oxidiert sein.

Des Weiteren bedeuten:
- Alkenyloxy: Alkenyl wie vorstehend genannt, das über ein Sauerstoffatom gebunden ist;
- Alkinyloxy: Alkinyl wie vorstehend genannt, das über ein Sauerstoffatom gebunden ist;
- Alkylamino: eine Gruppe NHR, worin R für Alkyl, wie zuvor definiert, steht;
- [Dialkyl]amino: eine Gruppe NR'R, worin R und R' für Alkyl, wie zuvor definiert, stehen;
- Alkoxyamino: eine Gruppe NH(OR), worin R für Alkyl, wie zuvor definiert, steht;
- Alkylsulfonylamino: eine Gruppe NHS(O)₂R
- Alkylaminosulfonylamino: eine Gruppe NHS(O)₂NHR, worin R für Alkyl, wie zuvor definiert, steht;
- [Dialkylamino]sulfonylamino: eine Gruppe NHS(O)₂NR'R, worin R und R' für Alkyl, wie zuvor definiert, stehen;
- Alkenylamino: eine Gruppe NHR, worin R für Alkenyl, wie zuvor definiert, steht;
- Alkinylamino: eine Gruppe NHR, worin R für Alkinyl, wie zuvor definiert, steht;
- N-(Alkenyl)-N-(alkyl)-amino: eine Gruppe NR'R, worin R für Alkenyl und R' für Alkyl, wie zuvor definiert, stehen;
- N-(Alkinyl)-N-(alkyl)-amino: eine Gruppe NR'R, worin R für Alkinyl und R' für Alkyl, wie zuvor definiert, stehen;
- N-(Alkoxy)-N-(alkyl)-amino: eine Gruppe NR'R, worin R für Alkyl und R' für Alkoxy, wie zuvor definiert, stehen;
- N-(Alkenyl)-N-(alkoxy)-amino: eine Gruppe NR'R, worin R für Alkenyl und R' für Alkoxy, wie zuvor definiert, stehen; und
- N-(Alkinyl)-N-(alkoxy)-amino: eine Gruppe NR'R, worin R für Alkinyl und R' für Alkoxy, wie zuvor definiert, stehen.

In einer besonderen Ausführungsform haben die Variablen der Verbindungen der Formel I folgende Bedeutungen, wobei diese sowohl für sich allein betrachtet als auch in Kombination miteinander besondere Ausgestaltungen der Verbindungen der Formel I darstellen:
R¹ steht insbesondere für Cyano, Nitro oder für einen 5- oder 6-gliedrigen heteroaromatischen Rest, wie zuvor definiert, der vorzugsweise entweder 1, 2, 3 oder 4 Stickstoffatome oder 1 Sauerstoff oder 1 Schwefelatom und gegebenenfalls 1 oder 2 Stickstoffatome als Ringlieder aufweiset und der unsubstituiert ist oder 1 oder 2 unter R^{1a} ausgewählte Substituenten aufweisen kann.

In einer ersten bevorzugten Ausführungsform der Erfindung steht R¹ für Cyano oder Nitro.

In einer weiteren bevorzugten Ausführungsform der Erfindung steht R¹ für 5- oder 6-gliedrigen heteroaromatischen Rest, wie zuvor definiert, der vorzugsweise entweder 1, 2, 3 oder 4 Stickstoffatome oder 1 Sauerstoff oder 1 Schwefelatom und gegebenenfalls 1 oder 2 Stickstoffatome als Ringlieder aufweist und der unsubstituiert ist oder 1 oder 2 unter R^{1a} ausgewählte Substituenten aufweisen kann. Beispiele für bevorzugte heteroaromatische Reste sind Pyridazin-3-yl, Pyridazin-4-yl, Pyrimidin-2-yl, Pyrimidin-4-yl, Pyrimidin-5-yl, Pyrazin-2-yl, 2-Furyl, 3-Furyl, 2-Thienyl, 3-Thienyl, Pyrazol-1-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-1-yl, Imidazol-2-yl, Imidazol-4-yl, Imidazol-5-yl, Oxazol-2-yl, Oxazol-4-yl, Oxazol-5-yl, Thiazol-2-yl, Thiazol-4-yl und Thiazol-5-yl, insbesondere C-gebundene heteroaromatische Reste wie Pyrazol-3-yl, Imidazol-5-yl, Oxazol-2-yl, Thiazol-2-yl, Thiazol-4-yl, Thiazol-5-yl, Pyridin-2-yl, Pyridin-3-yl, Pyridin-4-yl, Pyrimidin-2-yl, Pyrimidin-4-yl, Pyrimidin-5-yl, Pyridazin-4-yl, Pyrazin-2-yl, [1H]-Tetrazol-5-yl und [2H]-Tetrazol-5-yl, wobei die hier exemplarisch genannten Heterocyclen 1 oder 2 unter R^{1a} ausgewählte Substituenten aufweisen können. Bevorzugte Reste R^{1a} sind insbesondere F, Cl, CN, Nitro, Methyl, Ethyl, Methoxy, Ethoxy, Difluormethoxy, Trifluormethoxy und Trifluormethyl.

Ebenfalls bevorzugt sind Verbindungen der allgemeinen Formel I und deren Salze, worin R¹ für Halogen, insbesondere für Chlor oder Brom, steht.

Der Rest R² steht vorzugsweise für Wasserstoff, Fluor, Chlor, C₁-C₂-Alkyl, C₁-C₂-Fluoralkyl, Ethenyl, C₁-C₂-Alkoxy oder C₁-C₂-Fluoralkoxy, insbesondere für Fluor, Chlor, Methyl, Ethyl, Methoxy, Ethenyl oder Trifluormethoxy. R² steht besonders bevorzugt für Wasserstoff, Fluor oder Chlor.

Unter den Verbindungen der Formel I, in denen R² von Wasserstoff verschieden ist, sind solche Verbindungen bevorzugt, worin R² in ortho-Position zur Bindungsstelle des Phenylrings angeordnet ist.

In einer besonders bevorzugten Ausführungsform steht R² für Halogen, insbesondere Chlor oder Fluor, das in ortho-Position zur Bindungsstelle des Phenylrings angeordnet ist.

Unter den Verbindungen der Formel I, in denen R³ für Halogen steht, sind solche Verbindungen bevorzugt, worin R³ in para-Position zur Gruppe R¹ angeordnet ist.

Unter den Verbindungen der Formel I, in denen R³ für Halogen steht, sind solche Verbindungen bevorzugt, worin R³ für Fluor oder Chlor steht. In einer anderen, ebenfalls bevorzugten Ausführungsform steht R³ für Wasserstoff.

R⁴ steht vorzugsweise für Methyl.

R⁵ steht vorzugsweise für Wasserstoff, Methyl oder Ethyl, speziell Methyl.

Ebenfalls bevorzugt sind Verbindungen der Formel I, worin R⁵ eine Gruppe C(=O)R⁵¹ bedeutet, worin R⁵¹ eine der zuvor genannten Bedeutungen aufweist und insbesondere für Wasserstoff, C₁-C₄-Alkyl, speziell Methyl oder Ethyl, oder für C₁-C₄-Hatoalkyl, speziell C₁-C₂-Fluoralkyl wie Trifluormethyl steht.

R⁶ steht vorzugsweise für C₁-C₃-Alkyl, oder C₁-C₂-Fluoralkyl I, insbesondere für Methyl, Ethyl, n-Propyl oder Trifluormethyl, I und speziell für Methyl oder Ethyl.

Vorzugsweise steht wenigstens einer und insbesondere beide Reste R⁷ und R⁸ für Wasserstoff.

Unter den Verbindungen der Formel I, in denen R⁹ für einen von Wasserstoff verschiedenen Rest steht, sind solche Verbindungen bevorzugt, worin R⁹ in para-Position zur Gruppe CR⁷R⁸ angeordnet ist.

Unter den Verbindungen der Formel I, in denen R⁹ für einen von Wasserstoff verschiedenen Rest steht, sind solche Verbindungen bevorzugt, worin R⁹ für Halogen, insbesondere für Fluor oder Chlor steht. In einer anderen, ebenfalls bevorzugten Ausführungsform steht R⁹ für Wasserstoff.

R¹⁰ steht vorzugsweise für Wasserstoff.

R¹¹ steht vorzugsweise für Wasserstoff.

In der Gruppe C(O)R¹² steht R¹² vorzugsweise für Wasserstoff, C₁-C₄-Alkyl oder C₁-C₄-Haloalkyl.

Unter den Verbindungen der Formel I und deren Salzen sind die Verbindungen der allgemeinen Formel la sowie deren landwirtschaftlich geeigneten Salze bevorzugt: 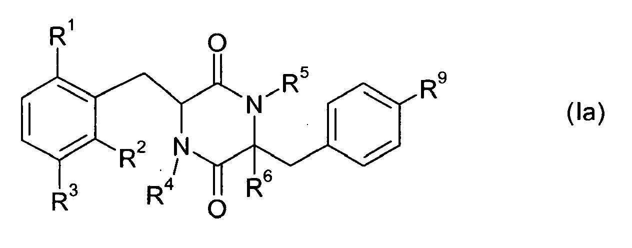 worin R¹, R², R³, R⁴, R⁵, R⁶ und R⁹ eine der zuvor angegebenen Bedeutungen, insbesondere die als bevorzugt angegebenen Bedeutungen aufweisen. Insbesondere haben in Formel la die Reste R¹, R², R³, R⁴, R⁵, R⁶ und R⁹ unabhängig voneinander, vorzugsweise jedoch in Kombination, die nachfolgenden Bedeutungen aufweisen:
- R¹: Cyano oder Nitro;
- R²: Wasserstoff, Fluor, Chlor, C₁-C₂-Alkyl, Ethenyl oder C₁-C₂-Alkoxy, insbesondere Wasserstoff, Fluor oder Chlor;
- R³: Fluor oder Wasserstoff;
- R⁴: Methyl;
- R⁵: Wasserstoff, Methyl oder Ethyl, speziell Methyl;
- R⁶: Methyl oder Ethyl; und
- R⁹: Wasserstoff oder Halogen, insbesondere Wasserstoff oder Fluor.

Wie bereits zuvor erläutert, weisen die Verbindungen der Formel I an den Kohlenstoffatomen der 3- und der 6-Position des Piperazinrings jeweils ein Chiralitätszentrum auf. Bevorzugt sind diejenigen Verbindungen der Formel I, worin die benzylischen Gruppen an der 3- und der 6-Position eine cis-Anordnung bezüglich des Piperazinrings aufweisen, d.h. das S,S-Enantiomer (S,S)-I und das R,R-Enantiomer (R,R)-I sowie deren Gemische. Ebenso bevorzugt sind Gemische der cis-Verbindung(en) mit der(den) trans-Verbindung(en), worin die cis-Verbindung(en) im Überschuss vorliegt(en), insbesondere cis/trans-Gemische mit einem cis/trans-Verhältnis, von wenigstens 2:1 insbesondere wenigstens 5:1.

Eine besonders bevorzugte Ausführungsform der Erfindung betrifft das S,S-Enantiomer der Formel (S,S)-I, sowie Enantiomerengemische und Diastereomerengemische von I, worin das S,S-Enantiomer Hauptbestandteil ist und vorzugsweise wenigstens 70 %, insbesondere wenigstens 80 % und speziell wenigstens 90 % der Verbindung I ausmacht. Ebenso bevorzugt sind die landwirtschaftlich geeigneten Salze der Enantiomere (S,S)-I und Enantiomerengemische und Diastereomerengemische der Salze, worin das S,S-Enantiomer Hauptbestandteil ist und vorzugsweise wenigstens 70 %, insbesondere wenigstens 80 % und speziell wenigstens 90 % der Verbindung I ausmacht. Eine andere, ebenso bevorzugt Ausführungsform betrifft eine racemische Mischung des S,S-Enantiomers (S,S)-I mit dem R,R-Enantiomer (R,R)-I.

Eine weitere Ausführungsform der Erfindung betrifft das R,R-Enantiomer der Formel (R,R)-I, sowie Enantiomerengemische und Diastereomerengemische von I, worin das R,R-Enantiomer Hauptbestandteil ist und vorzugsweise wenigstens 70 %, insbesondere wenigstens 80 % und speziell wenigstens 90 % der Verbindung I ausmacht. Eine weitere Ausführungsform der Erfindung betrifft die landwirtschaftlich geeigneten Salze der Enantiomere (R,R)-I und Enantiomerengemische und Diastereomerengemische der Salze, worin das R,R-Enantiomer Hauptbestandteil ist und vorzugsweise wenigstens 70 %, insbesondere wenigstens 80 % und speziell wenigstens 90 % der Verbindung I ausmacht.

Bevorzugt sind insbesondere die reinen Enantiomere der im Folgenden angegebenen Formel (S,S)-Ia, worin R¹, R², R³, R⁴, R⁵, R⁶ und R⁹ eine der zuvor angegebenen Bedeutungen, insbesondere eine der als bevorzugt oder als besonders bevorzugt angegebenen Bedeutungen aufweisen, sowie Enantiomerengemische und Diastereomerengemische von la, worin das S,S-Enantiomer Hauptbestandteil ist und vorzugsweise wenigstens 70 %, insbesondere wenigstens 80 % und speziell wenigstens 90 % der Verbindung la ausmacht. Ebenso bevorzugt sind die landwirtschaftlich geeigneten Salze der Enantiomere (S,S)-Ia und Enantiomerengemische und Diastereomerengemische der Salze, worin das Salz des S,S-Enantiomers Hauptbestandteil ist und vorzugsweise wenigstens 70 %, insbesondere wenigstens 80 % und speziell wenigstens 90 % des Salzes von la ausmacht. Eine andere, ebenso bevorzugt Ausführungsform betrifft eine racemische Mischung des S,S-Enantiomers (S,S)-Ia mit dem R,R-Enantiomer (R,R)-Ia.

Eine weitere Ausführungsform der Erfindung betrifft die reinen Enantiomere der im Folgenden angegebenen Formel (R,R)-Ia, worin R¹, R², R³, R⁴, R⁵, R⁶ und R⁹ eine der zuvor angegebenen Bedeutungen, insbesondere eine der als bevorzugt oder als besonders bevorzugt angegebenen Bedeutungen aufweisen, sowie Enantiomerengemische und Diastereomerengemische von la, worin das R,R-Enantiomer Hauptbestandteil ist und vorzugsweise wenigstens 70 %, insbesondere wenigstens 80 % und speziell wenigstens 90 % der Verbindung la ausmacht. Eine weitere Ausführungsform der Erfindung betrifft die landwirtschaftlich geeigneten Salze der Enantiomere (R,R)-Ia und Enantiomerengemische und Diastereomerengemische der Salze, worin das Salz des R,R-Enantiomers Hauptbestandteil ist und vorzugsweise wenigstens 70 %, insbesondere wenigstens 80 % und speziell wenigstens 90 % des Salzes von la ausmacht.

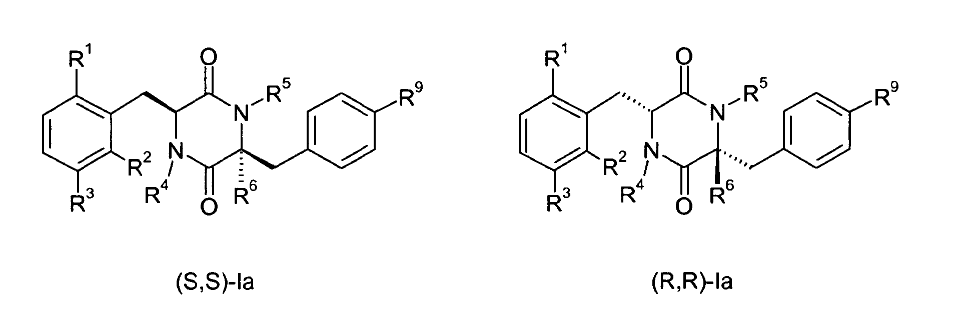

Insbesondere haben in Formel (S,S)-Ia bw. (R,R)-Ia die Reste R¹, R², R³, R⁴, R⁵, R⁶ und R⁹ unabhängig voneinander, vorzugsweise jedoch in Kombination, die nachfolgenden Bedeutungen:
- R¹: Cyano oder Nitro;
- R²: Wasserstoff, Fluor, Chlor, C₁-C₂-Alkyl, Ethenyl oder C₁-C₂-Alkoxy, insbesondere Wasserstoff, Fluor oder Chlor;
- R³: Fluor oder Wasserstoff;
- R⁴: Methyl;
- R⁵: Wasserstoff, Methyl oder Ethyl, speziell Methyl;
- R⁶: Methyl oder Ethyl; und
- R⁹: Wasserstoff oder Halogen, insbesondere Wasserstoff oder Fluor.

Beispiele für erfindungsgemäß bevorzugte Verbindungen sind die im Folgenden genannten Verbindungen sowie deren Salze:
2-[5-Benzyl-1,4,5-trimethyl-3,6-dioxopiperazin-2-ylmethyl]-benzonitril,
2-[5-Benzyl-1,4,5-trimethyl-3,6-dioxopiperazin-2-ylmethyl]-3-fluorbenzonitril,
2-[5-Benzyl-1,4,5-trimethyl-3,6-dioxopiperazin-2-ylmethyl]-3-methoxybenzonitril,
2-[5-Benzyl-1,4,5-trimethyl-3,6-dioxopiperazin-2-ylmethyl]-3,4-difluorbenzonitril,
2-[5-Benzyl-1,4,5-trimethyl-3,6-dioxopiperazin-2-ylmethyl]-3-methylbenzonitril,
2-[5-Benzyl-1,4,5-trimethyl-3,6-dioxopiperazin-2-ylmethyl]-3-ethenylbenzonitril, I,
2-[5-Benzyl-1,5-dimethyl-3,6-dioxopiperazin-2-ylmethyl]-benzonitril,
2-[5-Benzyl-1,5-dimethyl-3,6-dioxopiperazin-2-ylmethyl]-3-fluorbenzonitril,
2-[5-Benzyl-1,5-dimethyl-3,6-dioxopiperazin-2-ylmethyl]-3-methoxybenzonitril,
2-[5-Benzyl-1,5-dimethyl-3,6-dioxopiperazin-2-ylmethyl]-3,4-difluorbenzonitril,
2-[5-Benzyl-1,5-dimethyl-3,6-dioxopiperazin-2-ylmethyl]-3-methylbenzonitril,
2-[5-Benzyl-1,5-dimethyl-3,6-dioxopiperazin-2-ylmethyl]-3-ethenylbenzonitril,
2-[5-Benzyl-5-ethyl-1,4-dimethyl-3,6-dioxopiperazin-2-ylmethyl]-benzonitril
2-[5-Benzyl-5-ethyl-1,4-dimethyl-3,6-dioxopiperazin-2-ylmethyl]-3-fluorbenzonitril
2-[5-Benzyl-5-ethyl-1,4-dimethyl-3,6-dioxopiperazin-2-ylmethyl]-3-methoxybenzonitril
2-[5-Benzyl-5-ethyl-1,4-dimethyl-3,6-dioxopiperazin-2-ylmethyl]-3,4-difluorbenzonitril,
2-[5-Benzyl-5-ethyl-1,4-dimethyl-3,6-dioxopiperazin-2-ylmethyl]-3-methylbenzonitril,
2-[5-Benzyl-5-ethyl-1,4-dimethyl-3,6-dioxopiperazin-2-ylmethyl]-3-ethenylbenzonitril,
2-[5-Benzyl-5-ethyl-1-methyl-3,6-dioxopiperazin-2-ylmethyl]-benzonitril,
2-[5-Benzyl-5-ethyl-1-methyl-3,6-dioxopiperazin-2-ylmethyl]-3-fluorbenzonitril,
2-[5-Benzyl-5-ethyl-1-methyl-3,6-dioxopiperazin-2-ylmethyl]-3-methoxybenzonitril,
2-[5-Benzyl-5-ethyl-1-methyl-3,6-dioxopiperazin-2-ylmethyl]-3,4-difluorbenzonitril,
2-[5-Benzyl-5-ethyl-1-methyl-3,6-dioxopiperazin-2-ylmethyl]-3-methylbenzonitril,
2-[5-Benzyl-5-ethyl-1-methyl-3,6-dioxopiperazin-2-ylmethyl]-3-ethenylbenzonitril,
2-[5-(4-Fluorbenzyl)-1,4,5-trimethyl-3,6-dioxopiperazin-2-ylmethyl]-benzonitril,
2-[5-(4-Fluorbenzyl)-1,4,5-trimethyl-3,6-dioxopiperazin-2-ylmethyl]-3-fluorbenzonitril,
2-[5-(4-Fluorbenzyl)-1,4,5-trimethyl-3,6-dioxopiperazin-2-ylmethyl]-3-methoxybenzonitril,
2-[5-(4-Fluorbenzyl)-1,4,5-trimethyl-3,6-dioxopiperazin-2-ylmethyl]-3,4-difluorbenzonitril,
2-[5-(4-Fluorbenzyl)-1,4,5-trimethyl-3,6-dioxopiperazin-2-ylmethyl]-3-methylbenzonitril,
2-[5-(4-Fluorbenzyl)-1,4,5-trimethyl-3,6-dioxopiperazin-2-ylmethyl]-3-ethenylbenzonitril,
2-[5-(4-Fluorbenzyl)-1,5-dimethyl-3,6-dioxopiperazin-2-ylmethyl]-benzonitril,
2-[5-(4-Fluorbenzyl)-1,5-dimethyl-3,6-dioxopiperazin-2-ylmethyl]-3-fluorbenzonitril,
2-[5-(4-Fluorbenzyl)-1,5-dimethyl-3,6-dioxopiperazin-2-ylmethyl]-3-methoxybenzonitril,
2-[5-(4-Fluorbenzyl)-1,5-dimethyl-3,6-dioxopiperazin-2-ylmethyl]-3,4-difluorbenzonitril,
2-[5-(4-Fluorbenzyl)-1,5-dimethyl-3,6-dioxopiperazin-2-ylmethyl]-3-methylbenzonitril,
2-[5-(4-Fluorbenzyl)-1,5-dimethyl-3,6-dioxopiperazin-2-ylmethyl]-3-ethenylbenzonitril,
2-[5-(4-Fluorbenzyl)-5-ethyl-1,4-dimethyl-3,6-dioxopiperazin-2-ylmethyl]-benzonitril
2-[5-(4-Fluorbenzyl)-5-ethyl-1,4-dimethyl-3,6-dioxopiperazin-2-ylmethyl]-3-fluorbenzonitril
2-[5-(4-Fluorbenzyl)-5-ethyl-1,4-dimethyl-3,6-dioxopiperazin-2-ylmethyl]-3-methoxybenzonitril
2-[5-(4-Fluorbenzyl)-5-ethyl-1,4-dimethyl-3,6-dioxopiperazin-2-ylmethyl]-3,4-difluorbenzonitril,
2-[5-(4-Fluorbenzyl)-5-ethyl-1,4-dimethyl-3,6-dioxopiperazin-2-ylmethyl]-3-methylbenzonitril,
2-[5-(4-Fluorbenzyl)-5-ethyl-1,4-dimethyl-3,6-dioxopiperazin-2-ylmethyl]-3-ethenylbenzonitril,
2-[5-(4-Fluorbenzyl)-5-ethyl-1-methyl-3,6-dioxopiperazin-2-ylmethyl]-benzonitril, 2-[5-(4-Fluorbenzyl)-5-ethyl-1-methyl-3,6-dioxopiperazin-2-ylmethyl]-3-fluorbenzonitril,
2-[5-(4-Fluorbenzyl)-5-ethyl-1-methyl-3,6-dioxopiperazin-2-ylmethyl]-3-methoxybenzonitril,
2-[5-(4-Fluorbenzyl)-5-ethyl-1-methyl-3,6-dioxopiperazin-2-ylmethyl]-3,4-difluorbenzonitril,
2-[5-(4-Fluorbenzyl)-5-ethyl-1-methyl-3,6-dioxopiperazin-2-ylmethyl]-3-methylbenzonitril,
2-[5-(4-Fluorbenzyl)-5-ethyl-1-methyl-3,6-dioxopiperazin-2-ylmethyl]-3-ethenylbenzonitril,
3-Benzyl-6-(2-nitrobenzyl)-1,3,4-trimethylpiperazin-2,5-dion,
3-Benzyl-6-(2-fluor-6-nitrobenzyl)-1,3,4-trimethylpiperazin-2,5-dion,
3-Benzyl-6-(2,3-difluor-6-nitrobenzyl)-1,3,4-trimethylpiperazin-2,5-dion,
3-Benzyl-6-(2-methoxy-6-nitrobenzyl)-1,3,4-trimethylpiperazin-2,5-dion,
3-Benzyl-6-(2-methyl-6-nitrobenzyl)-1,3,4-trimethylpiperazin-2,5-dion,
3-Benzyl-6-(2-ethenyl-6-nitrobenzyl)-1,3,4-trimethylpiperazin-2,5-dion,
3-Benzyl-6-(2-nitrobenzyl)-1,3-dimethylpiperazin-2,5-dion,
3-Benzyl-6-(2-fluor-6-nitrobenzyl)-1,3-dimethylpiperazin-2,5-dion,
3-Benzyl-6-(2,3-difluor-6-nitrobenzyl)-1,3-dimethylpiperazin-2,5-dion,
3-Benzyl-6-(2-methoxy-6-nitrobenzyl)-1,3-dimethylpiperazin-2,5-dion,
3-Benzyl-6-(2-methyl-6-nitrobenzyl)-1,3-dimethylpiperazin-2,5-dion,
3-Benzyl-6-(2-ethenyl-6-nitrobenzyl)-1,3-dimethylpiperazin-2,5-dion,
3-Benzyl-6-(2-nitrobenzyl)-3-ethyl-1,4-dimethylpiperazin-2,5-dion,
3-Benzyl-6-(2-fluor-6-nitrobenzyl)-3-ethyl-1,4-dimethylpiperazin-2,5-dion,
3-Benzyl-6-(2,3-difluor-6-nitrobenzyl)-3-ethyl-1,4-dimethylpiperazin-2,5-dion,
3-Benzyl-6-(2-methoxy-6-nitrobenzyl)-3-ethyl-1,4-dimethylpiperazin-2,5-dion,
3-Benzyl-6-(2-methyl-6-nitrobenzyl)-3-ethyl-1,4-dimethylpiperazin-2,5-dion,
3-Benzyl-6-(2-ethenyl-6-nitrobenzyl)-3-ethyl-1,4-dimethylpiperazin-2,5-dion,
3-Benzyl-6-(2-nitrobenzyl)-3-ethyl-1-methylpiperazin-2,5-dion,
3-Benzyl-6-(2-fluor-6-nitrobenzyl)-3-ethyl-1-methylpiperazin-2,5-dion,
3-Benzyl-6-(2,3-difluor-6-nitrobenzyl)-3-ethyl-1-methylpiperazin-2,5-dion,
3-Benzyl-6-(2-methoxy-6-nitrobenzyl)-3-ethyl-1-methylpiperazin-2,5-dion,
3-Benzyl-6-(2-methyl-6-nitrobenzyl)-3-ethyl-1-methylpiperazin-2,5-dion,
3-Benzyl-6-(2-ethenyl-6-nitrobenzyl)-3-ethyl-1-methylpiperazin-2,5-dion,
3-(4-Fluorbenzyl)-6-(2-nitrobenzyl)-1,3,4-trimethylpiperazin-2,5-dion,
3-(4-Fluorbenzyl)-6-(2-fluor-6-nitrobenzyl)-1,3,4-trimethylpiperazin-2,5-dion,
3-(4-Fluorbenzyl)-6-(2,3-difluor-6-nitrobenzyl)-1,3,4-trimethylpiperazin-2,5-dion,
3-(4-Fluorbenzyl)-6-(2-methoxy-6-nitrobenzyl)-1,3,4-trimethylpiperazin-2,5-d ion,
3-(4-Fluorbenzyl)-6-(2-methyl-6-nitrobenzyl)-1,3,4-trimethylpiperazin-2,5-dion,
3-(4-Fluorbenzyl)-6-(2-ethenyl-6-nitrobenzyl)-1,3,4-trimethylpiperazin-2,5-dion,
3-(4-Fluorbenzyl)-6-(2-nitrobenzyl)-1,3-dimethylpiperazin-2,5-dion,
3-(4-Fluorbenzyl)-6-(2-fluor-6-nitrobenzyl)-1,3-dimethylpiperazin-2,5-dion,
3-(4-Fluorbenzyl)-6-(2,3-difluor-6-nitrobenzyl)-1,3-dimethylpiperazin-2,5-dion,
3-(4-Fluorbenzyl)-6-(2-methoxy-6-nitrobenzyl)-1,3-dimethylpiperazin-2,5-dion,
3-(4-Fluorbenzyl)-6-(2-methyl-6-nitrobenzyl)-1,3-dimethylpiperazin-2,5-dion,
3-(4-Fluorbenzyl)-6-(2-ethenyl-6-nitrobenzyl)-1,3-dimethylpiperazin-2,5-dion,
3-(4-Fluorbenzyl)-6-(2-nitrobenzyl)-3-ethyl-1,4-dimethylpiperazin-2,5-dion,
3-(4-Fluorbenzyl)-6-(2-fluor-6-nitrobenzyl)-3-ethyl-1,4-dimethylpiperazin-2,5-dion,
3-(4-Fluorbenzyl)-6-(2,3-difluor-6-nitrobenzyl)-3-ethyl-1,4-dimethylpiperazin-2,5-dion,
3-(4-Fluorbenzyl)-6-(2-methoxy-6-nitrobenzyl)-3-ethyl-1,4-dimethylpiperazin-2,5-dion,
3-(4-Fluorbenzyl)-6-(2-methyl-6-nitrobenzyl)-3-ethyl-1,4-dimethylpiperazin-2,5-dion,
3-(4-Fluorbenzyl)-6-(2-ethenyl-6-nitrobenzyl)-3-ethyl-1,4-dimethylpiperazin-2,5-dion,
3-(4-Fluorbenzyl)-6-(2-nitrobenzyl)-3-ethyl-1-methylpiperazin-2,5-dion,
3-(4-Fluorbenzyl)-6-(2-fluor-6-nitrobenzyl)-3-ethyl-1-methylpiperazin-2,5-dion,
3-(4-Fluorbenzyl)-6-(2,3-difluor-6-nitrobenzyl)-3-ethyl-1-methylpiperazin-2,5-dion,
3-(4-Fluorbenzyl)-6-(2-methoxy-6-nitrobenzyl)-3-ethyl-1-methylpiperazin-2,5-dion,
3-(4-Fluorbenzyl)-6-(2-methyl-6-nitrobenzyl)-3-ethyl-1-methylpiperazin-2,5-dion und
3-(4-Fluorbenzyl)-6-(2-ethenyl-6-nitrobenzyl)-3-ethyl-1-methylpiperazin-2,5-dion.

Unter den hier beispielhaft genannten Verbindungen und ihren Salzen sind solche Verbindungen und Salze bevorzugt, worin die benzylischen Gruppen in der 3- und der 6-Position des Piperazinrings cis zueinander angeordnet sind, und Gemische der cis-Verbindung(en) mit der(den) trans-Verbindung(en) worin die cis-Verbindung(en) im Überschuss vorliegt(en), insbesondere cis/trans-Gemische mit einem cis/trans-Verhältnis, von wenigstens 2:1 insbesondere wenigstens 5:1.

Unter den hier beispielhaft genannten Verbindungen und ihren Salzen sind jeweils das S,S-Enantiomer und dessen Salze bevorzugt. Ebenso bevorzugt sind Enantiomerengemische und Diastereomerengemische der hier exemplarisch genannten Verbindungen I und ihrer Salze, worin das S,S-Enantiomer Hauptbestandteil ist und vorzugsweise wenigstens 70 %, insbesondere wenigstens 80 % und speziell wenigstens 90 % der jeweiligen Verbindung ausmacht. Eine andere, ebenso bevorzugte Ausführungsform betrifft racemische Mischungen des jeweiligen S,S-Enantiomers mit dem jeweiligen R,R-Enantiomer der hier exemplarisch aufgeführten Verbindungen.

Die erfindungsgemäßen Verbindungen können nach Standardverfahren der organischen Chemie hergestellt werden, beispielsweise einem Verfahren (im Folgenden Verfahren A) welches die folgenden Schritte umfasst:
i) Bereitstellung einer Verbindung der allgemeinen Formel II 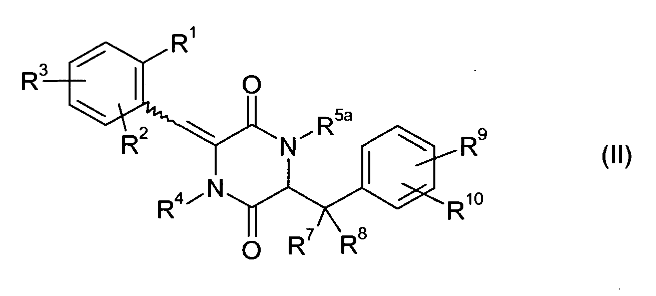 worin R¹, R², R³, R⁴, R⁷, R⁸, R⁹ und R¹⁰ die zuvor genannten Bedeutungen aufweisen, und R^{5a} eine der für R⁵ angegebene, von Wasserstoff verschiedene Bedeutung aufweist oder für eine Schutzgruppe steht;
ii) Umsetzung der Verbindung II mit einem Alkylierungsmittel der Formel R⁶-X worin R⁶ die zuvor angegebenen Bedeutungen aufweist und X für eine nucleophil verdrängbare Abgangsgruppe steht, in Gegenwart einer Base; gegebenenfalls Entfernen der Schutzgruppe, wenn R^{5a} für einen Schutzgruppe steht;
   und
iii) Hydrieren der in Schritt ii) erhaltenen Verbindung, wobei man eine Verbindung der Formel I erhält, worin R¹¹ für Wasserstoff steht;
   oder
iia) Hydrieren der Verbindung II;
   und
iiia) Umsetzung der in Schritt iia) erhaltenen Verbindung mit einem Alkylierungsmittel der Formel R⁶-X, worin R⁶ die zuvor angegebenen Bedeutungen aufweist und X für eine nucleophil verdrängbare Abgangsgruppe steht, in Gegenwart einer Base und gegebenenfalls Entfernen der Schutzgruppe, wenn R^{5a} für einen Schutzgruppe steht, wobei man eine Verbindung der Formel I erhält, worin R¹¹ für Wasserstoff steht;
iv) und gegebenenfalls Umsetzung der Verbindung der Formel I, worin R¹¹ für Wasserstoff steht mit einem Alkylierungsmittel R¹¹-X worin R¹¹ für C₁-C₄-Alkyl steht und X für eine nucleophil verdrängbare Abgangsgruppe steht, in Gegenwart einer Base.

Die Alkylierung in Schritt ii) bzw. iiia) können in Analogie zu Standardverfahren der Alkylierung durchgeführt werden, beispielsweise nach den von I.O. Donkor et al., Bioorg. Med. Chem. Lett. 11 (19) (2001), 2647-2649, B.B. Snider et al., Tetrahedron 57 (16) (2001), 3301-3307, I. Yasuhiro et al., Heterocycles, 45, 1997, 1151, J. Am. Chem. Soc. 105, 1983, 3214, J. Am. Chem. Soc. 124(47) (2002), 14017-14019, Chem. Commun. 1998, 659 oder M. Falorni et al., Europ. J. Org. Chem. (8) (2000), 1669-1675 beschriebenen Methoden.

Hierzu wird in den Schritten ii) die Piperazinverbindung der Formel II mit einem geeigneten Alkylierungsmittel, im Folgenden Verbindung X-R⁶ umgesetzt, wobei man eine Piperazinverbindung der Formel I erhält (siehe z.B. J. Am. Chem. Soc. 105, 1983, 3214).

In den Alkylierungsmitteln X-R⁶ kann X Halogen, insbesondere Chlor, Brom oder lod oder O-SO₂-R^{m} mit Rₘ in der Bedeutung von C₁-C₄-Alkyl oder Aryl, welche gegebenenfalls durch Halogen, C₁-C₄-Alkyl oder Halo-C₁-C₄-alkyl substituiert sind, bedeuten.

Die Umsetzung erfolgt üblicherweise bei Temperaturen im Bereich von -78°C bis zum Siedepunkt der Reaktionsmischung, bevorzugt von -50°C bis 65°C, insbesondere bevorzugt von -30°C bis 65°C. In der Regel wird die Umsetzung in einem Lösungsmittel vorgenommen, bevorzugt in einem inerten organischen Lösungsmittel.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Dichlormethan, Dichlorethan, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol, tert.-Butanol, Wasser, Dimethylsulfoxid, N-Methylpyrrolidon, Dimethylformamid und Dimethylacetamid sowie Morpholin und N-Methylmorpholin und deren Mischungen. Bevorzugte Lösungsmittel sind Toluol, Dichlormethan, Tetrahydrofuran, N-Methylpyrrolidon oder Dimethylformamid und deren Mischungen.

In der Regel wird die Alkylierung der Verbindung II in Schritt ii) bzw. iiia) mit dem Alkylierungsmittel in Gegenwart einer Base durchgeführt. Geeignete Basen sind anorganische Verbindungen wie Alkalimetall- und Erdalkalimetallhydroxide wie Lithiumhydroxid, Natriumhydroxid, Kaliumhydroxid oder Calciumhydroxid, wässrige Lösung von Ammoniak, Alkalimetall- oder Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, beispielsweise Lithiumdiisopropylamid, Natriumamid und Kaliumamid, Alkalimetall- und Erdalkalimetallcarbonate wie Lithiumcarbonat, Kaliumcarbonat, Cäsiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin, 2-Hydroxypyridin und N-Methylpiperidin, Pyridin, substituierte Pyridine wie Collidin, Lutidin und 4-Dimethylaminopyridin sowie bicyclische Amine. Die Basen werden im Allgemeinen äquimolar eingesetzt. Sie können auch im Überschuss oder selbst als Lösungsmittel verwendet werden. In einer bevorzugten Ausführungsform wird die Base in äquimolarer Menge oder im Wesentlichen äquimolarer Menge zugesetzt. In einer weiteren bevorzugten Ausführungsform wird Natriumhydrid als Base verwendet.

Geeignete Schutzgruppen, die als Gruppe R^{5a} in Betracht kommt, sind insbesondere die zuvor genannten Reste C(O)R⁵¹, z.B. der Acetylrest. Die Einführung dieser Schutzgruppen kann in Analogie zu bekannten Verfahren der Schutzgruppenchemie erfolgen, z.B. durch Umsetzung mit Anhydriden der Formel (R⁵¹C(O))₂O, z.B. nach der in Green, Wuts, Protective Groups in Organic Synthesis, 3rd ed. 1999, John Wiley and Sons, S. 553 beschriebenen Methode.

Die Entfernung einer Schutzgruppe R^{5a} kann in Analogie zu bekannten Verfahren der Schutzgruppenchemie erfolgen (siehe Green, Wuts, Protective Groups in Organic Synthesis, 3rd ed. 1999, John Wiley and Sons, S. 553).

Die Hydrierung in Schritt iii) bzw. in Schritt iia) kann in Analogie zu bekannten Verfahren zur Reduktion von C=C-Doppelbindungen erfolgen (siehe z.B. J. March, Advanced Organic Chemsistry, 3rd ed. John Wiley & Sons 1985, S. 690-700, sowie Peptide Chemistry 17, 1980, S. 59-64, Tetrahedron Lett. 46, 1979, S. 4483-4486.

Häufig erfolgt die Hydrierung durch Umsetzung mit Wasserstoff in Gegenwart von Übergangsmetallkatalysatoren, z.B. Katalysatoren, die Pt, Pd, Rh oder Ru als aktive Metallspezies enthalten. Geeignet sind sowohl heterogene Katalysatoren wie Pd- oder Pt-Trägerkatalysatoren, z.B. Pd auf Aktivkohle, weiterhin PtO₂, sowie Homogenkatalysatoren. Der Einsatz stereoselektiver Katalysatoren erlaubt eine enantioselektive Hydrierung der Doppelbindung (siehe Peptide Chemistry 17, 1980, S. 59-64, Tetrahedron Lett. 46, 1979, S. 4483-4486).

Die Hydrierung von II kann sowohl nach der Alkylierung von II, d.h. in Schritt iii), als auch vor der Alkylierung von II, d.h. in Schritt iia), durchgeführt werden.

Gegebenenfalls kann im Anschluss an das Entfernen der Schutzgruppe in Schritt ii) bzw. iiia), bei der eine Verbindung I mit R⁵ = H erhalten wird, ein neuer, von Wasserstoff verschiedener Rest R⁵ durch Alkylierung oder durch Acylierung eingeführt werden. Eine anschließende Alkylierung oder Acylierung zur Einführung des Restes R⁵ kann nach Standardverfahren der organischen Chemie erfolgen, z.B. auf die zuvor für die Schritte ii) bzw. iiia) angegebene Methoden.

Die Herstellung der Verbindung I, worin R¹¹ für C₁-C₄-Alkyl steht, erfolgt zweckmäßigerweise durch Umsetzung der Verbindung der Formel I, worin R¹¹ für Wasserstoff steht, mit einem Alkylierungsmittel R¹¹-X, worin R¹¹ für C₁-C₄-Alkyl steht und X für eine nucleophil verdrängbare Abgangsgruppe, z.B. für Halogen, insbesondere Chlor, Brom oder lod oder für O-SO₂-R^{m} mit R^{m} in der Bedeutung von C₁-C₄-Alkyl oder Aryl, welche gegebenenfalls durch Halogen, C₁-C₄-Alkyl oder Halo-C₁-C₄-alkyl substituiert sind, steht, in Gegenwart einer Base. Bezüglich der für diese Umsetzung erforderlichen Reaktionsbedingungen gilt das für die Alkylierung in Schritt ii) bzw. iiia) Gesagte in analoger Weise.

Verbindungen der Formel II sind im Übrigen bekannt, z.B. aus der PCT/EP 2007/050067 (=WO 2007/077247), auf deren Inhalt hiermit vollumfänglich Bezug genommen wird.

Die Herstellung der Verbindungen II kann beispielsweise durch Umsetzung eines Benzaldehyds der Formel II mit einer Piperazinverbindung IV im Sinne einer Aldolkondensation erfolgen, wie im folgenden Schema skizziert ist:

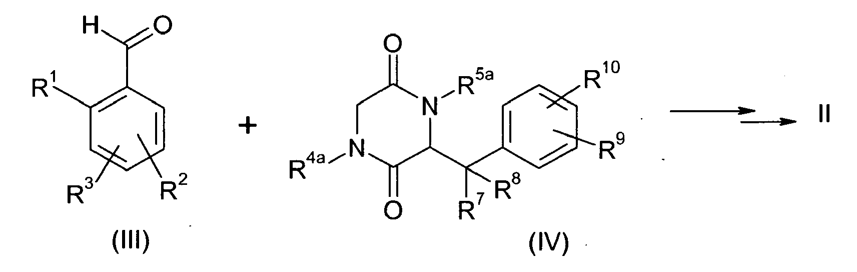

In den Formeln III und IV haben die Variablen R¹, R², R³, R^{5a}, R⁷, R⁸, R⁹ und R¹⁰ die für Formel II angegebenen Bedeutungen. R^{4a} steht für eine Schutzgruppe, C₁-C₄-Alkyl, C₃-C₄-Alkenyl oder C₃-C₄-Alkinyl. Geeignete Schutzgruppen für die Stickstoffatome des Piperazinrings sind insbesondere Acylgruppen, z.B. Gruppen der Formel C(O)R⁵², worin R⁵² eine der für R⁵¹ angegebenen Bedeutungen hat und insbesondere für C₁-C₄-Alkyl z.B. Methyl steht.

Solche Aldolkondensationen können analog zu den in J. Org. Chem. 2000, 65 (24), 8402-8405, Synlett 2006, 677, J. Heterocycl. Chem. 1988, 25, 591 beschriebenen Verfahren durchgeführt werden, worauf hiermit in vollem Umfang Bezug genommen wird.

Die Aldolkondensation erfolgt typischerweise in Gegenwart geeigneter Basen. Geeignete Basen sind solche, die üblicherweise bei Aldolkondensationen eingesetzt werden. Vorzugsweise verwendet man ein Alkalimetall- oder Erdalkalimetallcarbonat als Base, z.B. Natriumcarbonat, Kaliumcarbonat oder Cäsiumcarbonat oder deren Gemische.

Vorzugsweise erfolgt die Umsetzung in einem inerten, vorzugsweise aprotischen organischen Lösungsmittel. Beispiele für geeignete Lösungsmittel sind insbesondere Dichlormethan, Dichlorethan, Chlorbenzol, Ether wie Diethylether, Diisopropylether, tert.-Butylmethylether, Dioxan, Anisol und Tetrahydrofuran, Nitrile wie Acetonitril und Propionitril, sowie Dimethylsulfoxid, Dimethylformamid, N-Methylpyrrolidon und Dimethylacetamid. Bevorzugte Lösungsmittel sind insbesondere unter Dimethylformamid, N-Methylpyrrolidon und Dimethylacetamid ausgewählt.

Die für die Aldolkondensation erforderlichen Temperaturen liegen in der Regel im Bereich von 0°C bis hin zur Siedetemperatur des verwendeten Lösungsmittels und insbesondere im Bereich von 10 bis 80°C.

Für die Umsetzung III mit IV hat es sich als vorteilhaft erwiesen, wenn in der Verbindung IV die Reste R^{4a} und R^{5a} für eine Acylgruppe, z.B. eine Gruppe der Formel R⁵²C(O)- stehen, worin R⁵² eine der für R⁵¹ angegebenen Bedeutungen hat und insbesondere für C₁-C₄-Alkyl z.B. Methyl steht.

Die Einführung dieser Schutzgruppen in die Verbindung IV kann in Analogie zu bekannten Verfahren der Schutzgruppenchemie erfolgen, z.B. durch Umsetzung der entsprechenden NH-freien Verbindung (Verbindung der Formel IV mit R^{4a}, R^{5a} = H) mit Anhydriden der Formel (R⁵²C(O))₂O, z.B. nach der in Green, Wuts, Protective Groups in Organic Synthesis, 3rd ed. 1999, John Wiley and Sons, S. 553 beschriebenen Methode. Die Entfernung einer Schutzgruppe R^{4a}, R^{5a} kann in Analogie zu bekannten Verfahren der Schutzgruppenchemie erfolgen.

Sofern in der Verbindung IV die Reste R^{4a} und R^{5a} für eine Schutzgruppe, z.B. eine Acylgruppe stehen, wird man im Anschluss an die Aldolkondensation diese Reste entfernen, wobei man eine Verbindung der Formel II' erhält, 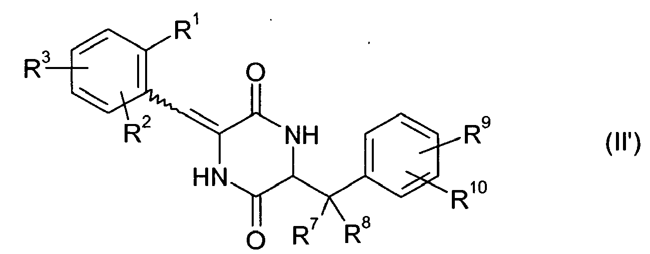 worin R¹, R², R³, R⁷, R⁸, R⁹ und R¹⁰ die für Formel I zuvor genannten Bedeutungen aufweisen. Die Entfernung der Schutzgruppen erfolgt in der Regel durch Hydrolyse, wobei der Rest R^{4a} häufig bereits unter den Bedingungen einer Aldolkondensation abgespalten wird. In die dabei erhaltene Verbindung II' wird anschließend der Rest R⁴ durch Alkylierung und gegebenenfalls der Rest R⁵ durch eine Alkylierung oder Acylierung eingeführt.

Die Alkylierung der Verbindung der Formel II' zur Einführung der Reste R⁴ und R⁵ bzw. R^{5a} kann in Analogie zu den für Schritt ii) bzw. Schritt iiia) angegebenen Methoden erfolgen, z.B. nach den in Heterocycles, 45, 1997, 1151, und Chem. Commun. 1998, 659 beschriebenen Methoden.

Hierzu wird die Piperazinverbindung der Formel II' mit einem geeigneten Alkylierungsmittel, im Folgenden Verbindung X¹-R⁴ und X¹-R⁵ bzw. X¹-R^{5a} umgesetzt. In den Alkylierungsmitteln X¹-R⁴, X¹-R⁵ bzw. X¹-R^{5a} kann X¹ Halogen oder O-SO₂-R^{m} mit R^{m} in der Bedeutung von C₁-C₄-Alkyl oder Aryl, welche gegebenenfalls durch Halogen, C₁-C₄-Alkyl oder Halo-C₁-C₄-alkyl substituiert sind, bedeuten. In den Alkylierungsmitteln X¹-R⁴, X¹-R⁵ bzw. X¹-R^{5a} stehen R⁴, R⁵ und R^{5a} unabhängig voneinander für C₁-C₄-Alkyl, C₃-C₄-Alkenyl oder C₃-C₄-Alkinyl.

Die Umsetzung der Verbindung II mit dem/den Alkylierungsmittel(n) X¹-R⁴, X¹-R⁵ bzw. X¹-R^{5a} erfolgt vorzugsweise in Gegenwart einer Base. Bezüglich Temperaturen, Basen und Lösungsmitteln gilt das für Schritt ii) bzw. iiia) Gesagte in analoger Weise.

Wenn R⁴ und R^{5a} in Formel II bzw. R⁴ und R⁵ in Formel I identisch sind, kann die Umsetzung von II' mit X¹-R⁴ und X¹-R⁵ bzw. X¹-R^{5a} gleichzeitig oder sukzessive in beliebiger Reihenfolge durchgeführt werden. Wenn die Reste R⁴, R⁵ und R⁶ identisch sind, kann die Umsetzung von. II'mit X¹-R⁴ und X¹-R⁵ bzw. X¹-R^{5a} gleichzeitig mit dem Schritt ii) des erfindungsgemäßen Verfahrens durchgeführt werden.

Sofern der Rest R⁵ in Formel I für eine Acylgruppe steht, wird dieser Rest vor oder im Anschluss an die Hydrierung von II durch eine Acylierung eingeführt. Hierzu wird die Verbindung I mit R⁵ = H mit einem Acylierungsmittel, im Folgenden Verbindung X²-R⁵, umgesetzt. Im Acylierungsmittel R⁵-X² steht R⁵ für einen Rest C(O)R⁵¹, worin R⁵¹ die zuvor genannten Bedeutungen aufweist. X² steht in der Regel für Halogen, z.B. Chlor oder für eine Gruppe O-C(O)-R⁵¹. Die Umsetzung kann analog zur Umsetzung von II mit den Alkylierungsmitteln X¹-R⁴ bzw. X¹-R⁵ erfolgen.

Die bei der Alkylierung von Verbindung II in Schritt ii) anfallende Verbindung der Formel IIa kann auch in Analogie zur Herstellung von II durch Umsetzung der Benzaldehyd-Verbindung III mit einer Verbindung IVa hergestellt werden:

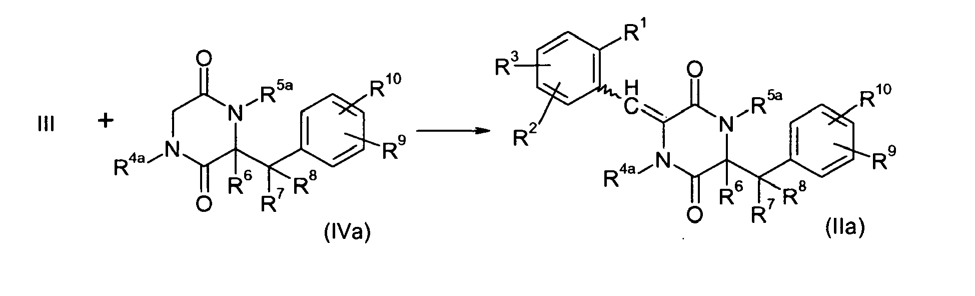

Hierbei haben R¹, R², R³, R^{4a}, R^{5a}, R⁶, R⁷, R⁸, R⁹ und R¹⁰ die zuvor genannten Bedeutungen, insbesondere eine der als bevorzugt genannten Bedeutungen aufweisen. Bevorzugte Reste R^{4a} und R^{5a} in Formel IVa sind die zuvor genannten Acylgruppen der Formel R⁵²C(O), worin R⁵² eine der für R⁵¹ angegebenen Bedeutungen hat und insbesondere für C₁-C₄-Alkyl z.B. Methyl steht.

Sofern R^{4a} und/oder R^{5a} in Formel IVa für eine Schutzgruppe, z.B. eine Acylgruppe der Formel R⁵²C(O), stehen, wird man die Schutzgruppen R^{4a} und/oder R^{5a} vorzugsweise vor der Hydrierung in Schritt iii) des erfindungsgemäßen Verfahrens entfernen. Auf diese Weise erhält man eine Verbindung IIa, worin R^{4a} und gegebenenfalls R^{5a} für Wasserstoff stehen.

Diese Verbindung IIa, worin R^{4a} für Wasserstoff steht, wird vor oder nach der Hydrierung mit einem Alkylierungsmittel der Formel R⁴-X¹, vorzugsweise in Gegenwart einer Base, umgesetzt. Sofern R^{5a} für Wasserstoff steht, wird die Verbindung IIa gegebenenfalls mit einem Alkylierungsmittel der Formel R⁵-X¹ oder einem Acylierungsmittel R⁵-X² vorzugsweise in Gegenwart einer Base, umgesetzt. Für die Umsetzung von Verbindung IIa mit den Alkylierungsmitteln bzw. Acylierurigsmitteln gilt das zuvor für die Umsetzung von II' mit dem/den Alkylierungsmittel(n) bzw. Acylierungsmittel(n) Gesagte analog.

Der Aldehyd III ist entweder kommerziell erhältlich oder kann gemäß bekannten Verfahren zur Herstellung von Aldehyden synthetisiert werden.

Die Verbindungen der Formel IV bzw. IVa können durch intramolekulare Cyclisierung von Verbindungen der allgemeinen Formel V bzw. Va in Analogie zu weiteren literaturbekannten Verfahren, beispielsweise nach T. Kawasaki et al., Org. Lett. 2(19) (2000), 3027-3029, Igor L. Rodionov et al., Tetrahedron 58(42) (2002), 8515-8523 oder A. L. Johnson et al., Tetrahedron 60 (2004), 961-965 hergestellt werden.

Gegebenenfalls erfolgt im Anschluss an die Cyclisierung die Einführung einer von Wasserstoff verschiedenen Gruppe R^{4a} bzw. R^{5a}, wenn R^{4a} bzw. R^{5b} in den Formeln V bzw. Va für Wasserstoff stehen.

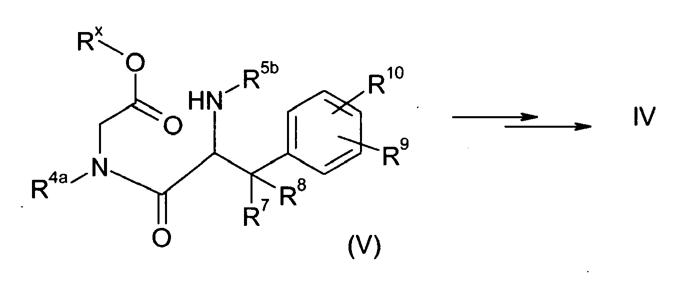

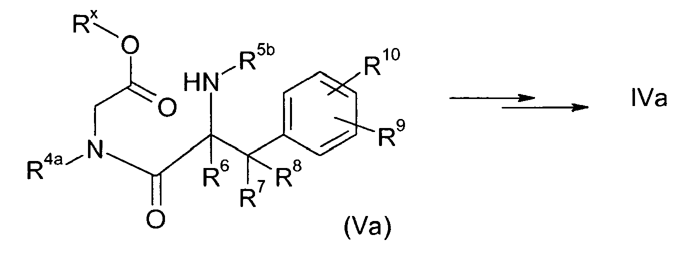

In den Formeln V und Va haben die Variablen R^{4a}, R⁶, R⁷, R⁸, R⁹ und R¹⁰ die zuvor genannten Bedeutungen. R^{5b} steht für Wasserstoff, C₁-C₄-Alkyl, C₃-C₄-Alkenyl oder C₃-C₄-Alkinyl. R^{x} ist hierbei z.B. C₁-C₆-Alkyl, insbesondere Methyl, Ethyl oder Phenyl-C₁-C₆-alkyl, z.B. Benzyl.

Die Cyclisierung der Verbindungen der Formel V bzw. Va kann in Gegenwart einer Base erfolgen. Die Reaktion erfolgt dann in der Regel bei Temperaturen im Bereich von 0°C bis zum Siedepunkt der Reaktionsmischung, bevorzugt von 10°C bis 50°C, insbesondere bevorzugt von 15°C bis 35°C. Die Umsetzung kann in einem Lösungsmittel durchgeführt werden, bevorzugt in einem inerten organischen Lösungsmittel.

Geeignete inerte organische Lösungsmittel umfassen aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Dichlormethan, Dichlorethan, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol, tert.-Butanol, Wasser sowie Dimethylsulfoxid, Dimethylformamid und Dimethylacetamid sowie Morpholin und N-Methylmorpholin. Es können auch Gemische der genannten Lösungsmittel verwendet werden. Bevorzugtes Lösungsmittel ist ein Tetrahydrofuran - Wasser Gemisch mit einem Mischungsverhältnis von 1 : 10 bis 10 : 1.

Geeignete Basen sind z.B. anorganische Verbindungen wie Alkalimetall- und Erdalkalimetallhydroxide wie Lithiumhydroxid, Natriumhydroxid, Kaliumhydroxid oder Calciumhydroxid, wässrige Lösung von Ammoniak, Alkalimetall- oder Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, beispielsweise Lithiumdiisopropylamid, Natriumamid und Kaliumamid, Alkalimetall- und Erdalkalimetallcarbonate wie Lithiumcarbonat, Kaliumcarbonat, Cäsiumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin, 2-Hydroxypyridin und N-Methylpiperidin, Pyridin, substituierte Pyridine wie Collidin, Lutidin und 4-Dimethylaminopyridin sowie bicyclische Amine. Selbstverständlich kann auch eine Mischung verschiedener Basen verwendet werden. Bevorzugt sind insbesondere Kaliumtert-butanolat, 2-Hydroxypyridin oder eine wässrige Lösung von Ammoniak oder einer Mischung dieser Basen. Bevorzugt wird nur eine dieser Basen verwendet. In einer besonders bevorzugten Ausführungsform wird die Reaktion in Gegenwart einer wässrigen Lösung von Ammoniak, die beispielsweise von 10 bis 50 w/v %ig sein kann, durchgeführt. In einer anderen besonders bevorzugten Ausführungsform erfolgt die Cyclisierung in einer Mischung umfassend n-Butanol oder ein Butanolisomerengemisch (z.B. ein Gemisch aus n-Butanol mit 2-Butanol und/oder Isobutanol) und N-Methyl-Morpholin, vorzugsweise unter Rückflussbedingungen.

Die Cyclisierung von V bzw. Va kann auch unter sauerer Katalyse, in Gegenwart aktivierender Verbindungen oder thermisch erfolgen. Die Umsetzung von V in Gegenwart einer Säure erfolgt üblicherweise bei Temperaturen im Bereich von 10°C bis zum Siedepunkt der Reaktionsmischung, bevorzugt von 50°C bis zum Siedepunkt, insbesondere bevorzugt beim Siedepunkt unter Rückfluss. In der Regel wird die Umsetzung in einem Lösungsmittel vorgenommen, bevorzugt in einem inerten organischen Lösungsmittel.

Als Lösungsmittel kommen prinzipiell diejenigen in Betracht, die auch für die basische Cyclisierung verwendet werden können, insbesondere Alkohole. In einer bevorzugten Ausführungsform wird die Reaktion in n-Butanol oder einem Gemisch verschiedener Butanolisomere (z.B. ein Gemisch aus n-Butanol mit 2-Butanol und/oder Isobutanol) durchgeführt.

Als Säuren für die Cyclisierung von V bzw. Va kommen grundsätzlich sowohl Brönstedt- als auch Lewis-Säuren in Betracht. Insbesondere können anorganische Säuren, z.B. Halogenwasserstoffsäuren wie Fluorwasserstoffsäure, Salzsäure, Bromwasserstoffsäure, anorganische Oxosäuren wie Schwefelsäure und Perchlorsäure, weiterhin anorganische Lewis-Säuren wie Bortrifluorid, Aluminiumtrichlorid, Eisen-IIIchlorid, Zinn-IV-chlorid, Titan-IV-chlorid und Zink-II-chlorid, sowie organische Säuren, beispielsweise Carbonsäuren und Hydroxycarbonsäuren wie Ameisensäure, Essigsäure, Propionsäure, Oxalsäure, Zitronensäure und Trifluoressigsäure, sowie organische Sulfonsäuren wie Toluolsulfonsäure, Benzolsulfonsäure, Camphersulfonsäure und dergleichen, Verwendung finden. Selbstverständlich kann auch eine Mischung verschiedener Säuren eingesetzt werden.

In einer Ausführungsform des erfindungsgemäßen Verfahrens wird die Reaktion in Gegenwart von organischen Säuren durchgeführt, beispielsweise in Gegenwart von Carbonsäuren wie Ameisensäure, Essigsäure oder Trifluoressigsäure oder einer Mischung dieser Säuren. Bevorzugt wird nur eine dieser Säuren verwendet. In einer bevorzugten Ausführungsform wird die Reaktion in Essigsäure durchgeführt.

In einer besonders bevorzugten Ausführungsform erfolgt die saure Cyclisierung in einer Mischung umfassend n-Butanol oder ein Butanolisomerengemisch (z.B. ein Gemisch aus n-Butanol mit 2-Butanol und/oder lsobutanol), N-Methyl-Morpholin und Essigsäure, vorzugsweise unter Rückflussbedingungen.

In einer weiteren Ausführungsform der Erfindung wird die Umsetzung von V bzw. Va durch Behandlung mit einem Aktivierungsmittel in Gegenwart einer Base durchgeführt. R^{x} steht dann für Wasserstoff. Beispiel für ein geeignetes Aktivierungsmittel ist Di-(N-succinimidinyl)carbonat. Geeignete Aktivierungsmittel sind außerdem polystyrol- oder nicht polystyrolgebundenes Dicyclohexylcarbodiimid (DCC), Diisopropylcarbodiimid, 1-Ethyl-3-(dimethylaminopropyl)carbodiimid (EDAC), Carbonyldiimidazol, Chlorkohlensäureester wie Methylchloroformiat, Ethylchloroformiat, Isopropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid. Geeignete Basen sind die für die basische Cyclisierung zitierten Verbindungen. In einer Ausführungsform werden als Base Triethylamin oder N-Ethyldiisopropylamin oder deren Mischungen, besonders bevorzugt N-Ethyldiisopropylamin verwendet.

In einer weiteren Ausführungsform der Erfindung wird die Umsetzung von V bzw. Va ausschließlich durch Erhitzen der Reaktionsmischung durchgeführt (thermische Cyclisierung). Die Umsetzung erfolgt hierbei üblicherweise bei Temperaturen im Bereich von 10°C bis zum Siedepunkt der Reaktionsmischung, bevorzugt von 50°C bis zum Siedepunkt der Reaktionsmischung, insbesondere bevorzugt beim Siedepunkt der Reaktionsmischung unter Rückfluss. In der Regel wird die Umsetzung in einem Lösungsmittel vorgenommen, bevorzugt in einem inerten organischen Lösungsmittel.

Als Lösungsmittel kommen prinzipiell diejenigen in Betracht, die bei der basischen Cyclisierung verwendet werden. Bevorzugt werden polar aprotische Lösungsmittel, z.B. Dimethylsulfoxid oder Dimethylformamid oder deren Mischungen. In einer bevorzugten Ausführungsform wird die Reaktion in Dimethylsulfoxid durchgeführt.

Sofern in Verbindung V bzw. Va einer oder beide Reste R^{4a} und/oder R^{5b} für Wasserstoff stehen, können die Piperazinstickstoffe anschließend zur Einführung der Reste R^{4a} bzw. R^{5a} durch Umsetzung mit einem Alkylierungsmittel R^{4a}-X¹ bzw. R^{5a}-X¹ alkyliert oder durch Umsetzung mit einem Acylierungsmittel R^{4a}-X² bzw. R^{5a}-X² mit einer Schutzgruppe versehen werden. Hierbei haben R^{4a}, R^{5a}, X¹ und X² die zuvor angegebenen Bedeutungen.

Die Verbindungen der Formel V bzw. Va können ihrerseits nach der im folgenden Schema dargestellten Synthese in Analogie zu literaturbekannten Verfahren hergestellt werden, beispielsweise nach Wilford L. Mendelson et al., Int. J. Peptide & Protein Research 35(3), (1990), 249-57, Glenn L. Stahl et al., J. Org. Chem. 43(11), (1978), 2285-6. oder A. K. Ghosh et al., Org. Lett. 3(4), (2001), 635-638.

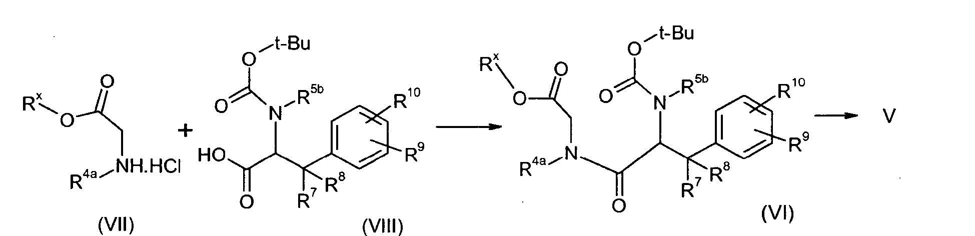

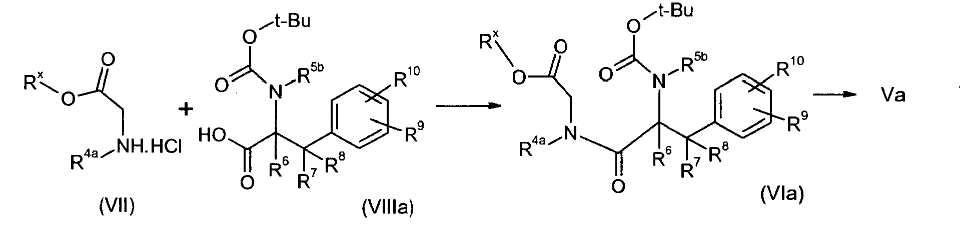

In dem Schema haben die Variablen R^{x}, R^{4a}, R^{5b}, R⁶, R⁷, R⁸, R⁹ und R¹⁰ die für Formel V angegebenen Bedeutungen. Die Synthese umfasst in einem ersten Schritt die Kupplung von Glycinesterverbindungen der Formel VII mit Boc-geschützten Phenylalaninverbindungen VIII bzw. VIIIa in Gegenwart eines Aktivierungsreagenz. Anstelle von Boc kann auch eine andere Aminoschutzgruppe eingesetzt werden.

Die Umsetzung einer Verbindung der Formel VII mit einer Verbindung der Formel VIII bzw. VIIIa erfolgt üblicherweise bei Temperaturen im Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel durchgeführt werden, bevorzugt in einem inerten organischen Lösungsmittel.

Im Allgemeinen erfordert die Umsetzung die Anwesenheit eines Aktivierungsreagenzes. Geeignete Aktivierungsreagenzien sind Kondensationsmittel wie z.B. polystyrol- oder nicht polystyrolgebundenes Dicyclohexylcarbodümid (DCC), Diisopropylcarbodiimid, Carbonyldiimidazol (CDI), 1-Ethyl-3-(dimethylaminopropyl)carbodiimid (EDAC), Chlorkohlensäureester wie Methylchloroformiat, Ethylchloroformiat, Isopropylchloroformiat, lsobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid. Nach einer Ausführungsform werden als Aktivierungsreagenz EDAC oder DCC bevorzugt.

Vorzugsweise erfolgt die Umsetzung von VII mit VIII bzw. VIIIa in Gegenwart einer Base. Geeignete Basen sind die Cyclisierung des Dipeptids V zum Piperazin IV zitierten Verbindungen. In einer Ausführungsform werden als Base Triethylamin oder N-Ethyldiisopropylamin oder deren Mischungen, besonders bevorzugt N-Ethyldiisopropylamin verwendet.

Das Entschützen der Verbindungen VI bzw. VIa zur Verbindung V bzw. Va kann nach üblichen Verfahren erfolgen, wie beispielsweise nach Glenn L. Stahl et al., J. Org. Chem. 43(11), (1978), 2285-6. oder A. K. Ghosh et al., Org. Lett. 3(4), (2001), 635-638. Das Entschützen erfolgt typischerweise durch Behandlung mit einer Säure. Als Säuren kommen sowohl Brönstedt- als auch Lewis-Säuren in Betracht, vorzugsweise organischen Carbonsäuren, beispielsweise Ameisensäure, Essigsäure oder Trifluoressigsäure oder deren Mischungen in betracht. In einer bevorzugten Ausführungsform wird die Reaktion in Gegenwart von Trifluoressigsäure durchgeführt.

Die Umsetzung erfolgt üblicherweise bei Temperaturen im Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel, bevorzugt in einem inerten organischen Lösungsmittel ausgeführt werden.

Als Lösungsmittel kommen prinzipiell die zuvor im Zusammenhang mit der basischen Cyclisierung von V zu IV genannten Lösungsmittel in Betracht, insbesondere Tetrahydrofuran oder Dichlormethan oder deren Mischungen. In einer bevorzugten Ausführungsform wird die Reaktion in Dichlormethan durchgeführt.

Wenn anstelle von Boc eine andere Schutzgruppe eingesetzt wird, so wird selbstverständlich ein für die jeweilige Schutzgruppe geeignetes Entschützungsverfahren angewendet.

Sofern in den Verbindungen IV bzw. IVa die Gruppen R^{4a} und R^{5a} bzw. R^{4c} und R^{5c} für Wasserstoff stehen, kann man die Verbindungen IV bzw. IVa auch durch intermolekulare Cyclisierung eines Glycinester-Derivats Vlla mit einer Phenylalanin-Verbindung VIIIb bzw. VIIIc gemäß den folgenden Schemata darstellen:

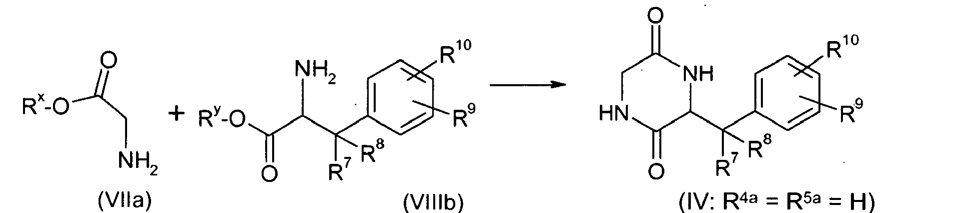

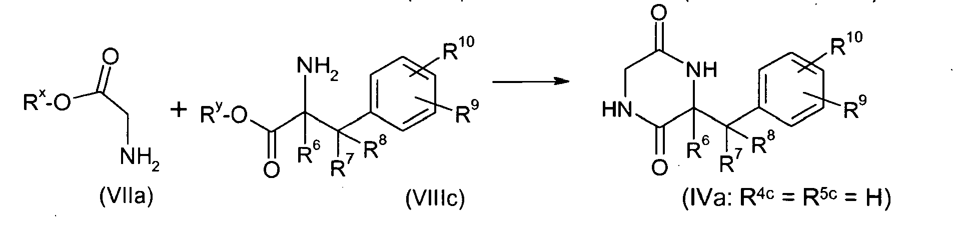

In den Schemata haben R^{x}, R⁶, R⁷, R⁸, R⁹ und R¹⁰ die zuvor angegebenen Bedeutungen. R^{y} steht für Alkyl, z.B. Methyl oder Ethyl. Die intermolekulare Cyclisierung kann z.B. durch eine Base, z.B. Ammoniak bewirkt werden. Die Verbindungen Vlla und/oder VIIIb bzw. Vlllc können auch in Form ihrer Säureadditionssalze, z.B. als Hydrochloride eingesetzt werden.

Gemäß einer anderen Ausführungsform (im Folgenden als Verfahren B bezeichnet) umfasst die Herstellung der Verbindungen I
i) die Bereitstellung einer Verbindung der allgemeinen Formel IX 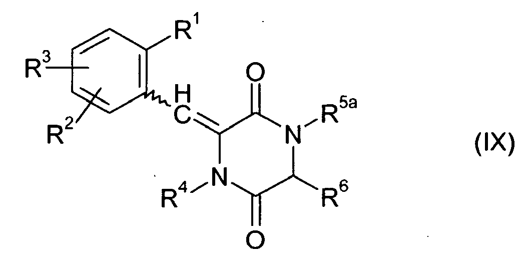 worin R¹, R²,R³, R⁴ und R⁶ die zuvor genannten Bedeutungen aufweisen, und R^{5a} eine der für R⁵ angegebenen, von Wasserstoff verschiedenen Bedeutungen aufweist oder für eine Schutzgruppe steht;
ii) Umsetzung der Verbindung IX mit einer Benzylverbindung der Formel X 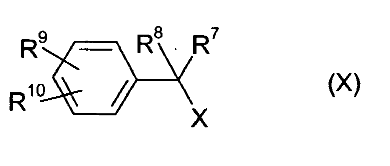 worin R⁷, R⁸, R⁹ und R¹⁰ die zuvor angegebenen Bedeutungen aufweisen und X für eine nucleophil verdrängbare Abgangsgruppe steht, in Gegenwart einer Base, wobei man eine Verbindung IIb erhält; 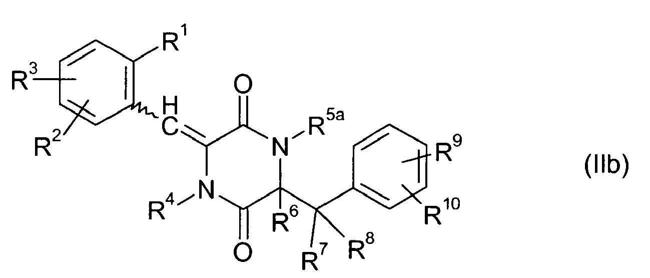
iii) Hydrieren der dabei erhaltenen Verbindung IIb
iv) wenn R^{5a} für einen Schutzgruppe steht, Entfernen der Schutzgruppe.

In Formel IX hat R^{5a} vorzugsweise eine für R⁵ angegebene, von Wasserstoff verschiedene Bedeutung. In Formel X hat die Variable X vorzugsweise eine der folgenden Bedeutungen: Halogen, insbesondere Chlor, Brom oder lod oder O-SO₂-R^{m} mit R^{m} in der Bedeutung von C₁-C₄-Alkyl oder Aryl, welche gegebenenfalls durch Halogen, C₁-C₄-Alkyl oder HalO-C₁-C₄-alkyl substituiert sind. Geeignete Schutzgruppen für die Stickstoffatome des Piperazinrings in IX sind insbesondere die zuvor genannten Reste C(O)R⁵², z.B. der Acetylrest.

Die Umsetzung der Verbindung IX mit der Verbindung X in Schritt ii) kann in Analogie zu der in Verfahren A, Schritt iv) beschriebenen Methode erfolgen oder z.B. nach der in J. Am. Chem. Soc. 105, 1983, 3214 beschriebenen Methode.

Die Hydrierung in Schritt ii) kann ebenfalls in der zuvor für die Hydrierung von Verbindung II bzw. IIa beschriebenen Weise erfolgen.

Die Bereitstellung der Verbindungen IX kann z.B. durch Umsetzung der Verbindung XI mit einer Benzaldehyd-Verbindung XII, wie in dem folgenden Schema erläutert, erfolgen.

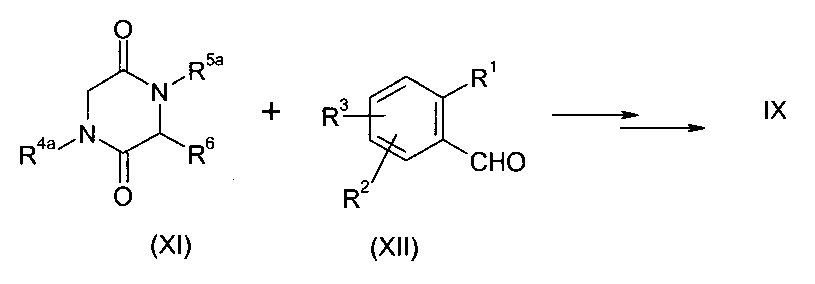

Hierin weisen R¹, R², R³, R^{5a} und R⁶ die zuvor genannten Bedeutungen auf. R^{4a} hat eine der für R⁴ angegebenen Bedeutungen oder steht für eine Schutzgruppe. Geeignete Schutzgruppen für die Stickstoffatome des Piperazinrings in XI sind insbesondere die zuvor genannten Reste C(O)R⁵¹, z.B. der Acetylrest. Insbesondere stehen R^{4a} und R^{5a} für einen der zuvor genannten Reste C(O)R⁵², z.B. für Acetylreste.

Die Umsetzung von XI mit XII kann unter den Bedingungen einer Aldolkondensation erfolgen, wie bereits für die Umsetzung von III mit IV bzw. IVa beschrieben. Solche Aldolkondensationen können analog zu den in J. Org. Chem. 2000, 65 (24), 8402-8405, Synlett 2006, 677, J. Heterocycl. Chem. 1988, 25, 591 beschriebenen Verfahren durchgeführt werden, worauf hiermit in vollem Umfang Bezug genommen wird.

Die Umsetzung erfolgt in der Regel in Gegenwart einer Base. Als Base verwendet man vorzugsweise ein Alkalimetall- oder Erdalkalimetallcarbonat, z.B. Natriumcarbonat, Kaliumcarbonat oder Cäsiumcarbonat oder deren Gemische.

Vorzugsweise erfolgt die Umsetzung in einem inerten, vorzugsweise aprotischen organischen Lösungsmittel. Beispiele für geeignete Lösungsmittel sind insbesondere Dichlormethan, Dichlorethan, Chlorbenzol, Ether wie Diethylether, Diisopropylether, tert.-Butylmethylether, Dioxan, Anisol und Tetrahydrofuran, Nitrile wie Acetonitril und Propionitril, sowie Dimethylsulfoxid, Dimethylformamid, N-Methylpyrrolidon und Dimethylacetamid

Vorzugsweise erfolgt die Umsetzung von solchen Verbindungen XI, worin R^{4a} und R^{5a} für eine Schutzgruppe und insbesondere einen Acylrest R⁵²C(O)- (R⁵² = C₁-C₄-Alkyl), z.B. einen Acetylrest, stehen. Dementsprechend schließt sich im Anschluss an die Kondensationreaktion in der Regel eine Entfernung der Schutzgruppen an. Die Entfernung einer Schutzgruppe R^{4a}, R^{5a} kann in Analogie zu bekannten Verfahren der Schutzgruppenchemie erfolgen, z.B. nach der in Green, Wuts, Protective Groups in Organic Synthesis, 3rd ed. 1999, John Wiley and Sons, S. 553 beschriebenen Methode. Auf diese Weise erhält man Verbindungen der Formel IX, worin R^{4a} und R^{5a} für Wasserstoff stehen.

Eine anschließende Alkylierung zur Einführung der Reste R⁴ bzw. R⁵ kann auf die zuvor bei Verfahren A für die Alkylierung von II angegebene Methode erfolgen, z.B. nach den in Heterocycles, 45, 1997, 1151, und Chem. Commun. 1998, 659 beschriebenen Methoden.

Die Verbindungen XI sind bekannt. Ihre Herstellung kann in Analogie zur Herstellung der oben beschriebenen Verbindungen V gemäß dem im Folgenden gezeigten Schema erfolgen:

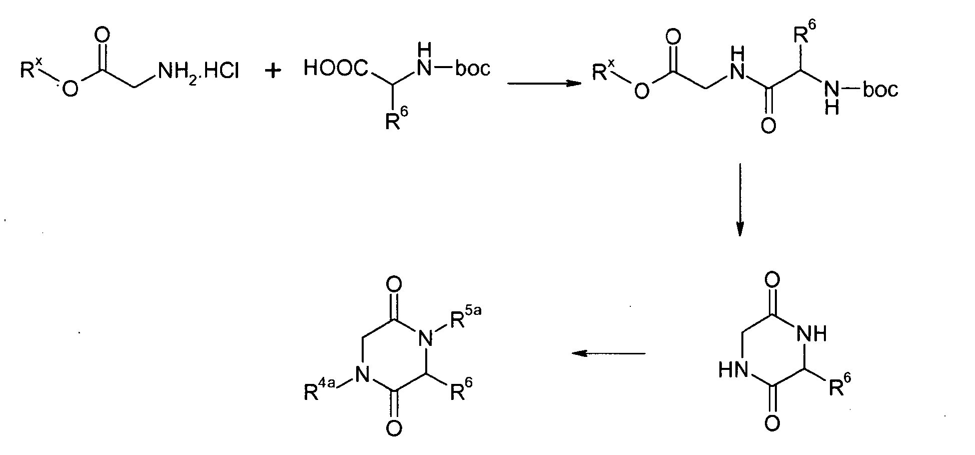

In diesem Schema haben R^{4a}, R^{5a} und R⁶ die zuvor genannten Bedeutungen. R^{x} steht vorzugsweise für C₁-C₄-Alkyl oder Benzyl. Boc steht für einen tert.-Butoxycarbonyl-Rest.

Bezüglich weiterer Details für den ersten Reaktionsschritt wird auf die Umsetzung von Verbindung VII mit der Verbindung VIII bzw. VIIIa bzw. auf die Umsetzung von VIIa mit Vlllb bwz. VIIIc verwiesen. Die anschließende Entfernung der boc-Schutzgruppe kann in Analogie zur Umsetzung der Verbindung VI zur Verbindung V erfolgen. Die Cyclisierung der dabei erhaltenen entschützten Verbindung kann nach den für die Cyclisierung der Verbindung V genannten Methoden erfolgen. Sofern R^{4a} und R^{5a} für eine Schutzgruppe, z.B. einen Rest C(O)R⁵¹ steht, kann die Einführung dieser Schutzgruppen in Analogie zu bekannten Verfahren der Schutzgruppenchemie erfolgen, z.B. durch Umsetzung mit Anhydriden der Formel (R⁵¹C(O))₂O, z.B. nach der in Green, Wuts, Protective Groups in Organic Synthesis, 3rd ed. 1999, John Wiley and Sons, S. 553 beschriebenen Methode.

Gemäß einem dritten Verfahren erfolgt die Herstellung der Verbindung 1 durch Cyclisierung entsprechender Dipeptid-Vorstufen der Formel XIII, beispielsweise in Analogie zu der von T. Kawasaki et al., Org. Lett. 2(19) (2000), 3027-3029, Igor L. Rodionov et al., Tetrahedron 58(42) (2002), 8515-8523 oder A. L. Johnson et al., Tetrahedron 60 (2004), 961-965 beschriebenen Methode. Die Cyclisierung von Dipeptiden der Formel XIII zu den erfindungsgemäßen Verbindungen wird im Folgenden auch als Verfahren C bezeichnet und ist in dem nachfolgenden Schema skizziert.

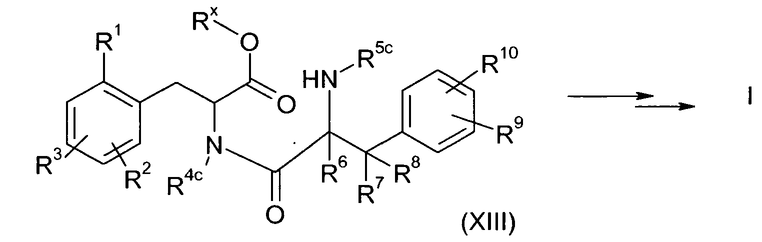

In Formel XIII haben die Variablen R¹, R², R³, R⁶, R⁷, R⁸, R⁹ und R¹⁰ die für Formel I angegebene Bedeutung. R^{4c} steht für Wasserstoff oder für R⁴. R^{5c} steht für Wasserstoff, C₁-C₄-Alkyl, C₃-C₄-Alkenyl oder C₃-C₄-Alkinyl. Die Gruppe OR^{x} steht für eine geeignete, über Sauerstoff gebundene Abgangsgruppe. Rₓ ist hierbei z.B. Wasserstoff, C₁-C₆-Alkyl, insbesondere Methyl, Ethyl oder Phenyl-C₁-C₆-alkyl, z.B. Benzyl. Dipeptide der allgemeinen Formel XIII sind neu und ebenfalls Gegenstand der vorliegenden Erfindung.

Die Cyclisierung kann beispielsweise durch Umsetzung eines Dipeptids der Formel XIII, entweder in Gegenwart von Säure oder Base (saure oder basische Cyclisierung) oder durch Erhitzen der Reaktionsmischung (thermische Cyclisierung) bewirkt werden. Bezüglich der Reaktionsbedingungen wird auf die für die Cyclisierung von V zur Verbindung IV gemachten Angaben verwiesen.

Vorzugsweise stehen R^{4c} und R^{5c} in Formel XIII für Wasserstoff. In diesem Fall erfolgt im Anschluss an die Cyclisierung eine Alkylierung bzw. Acylierung zur Einführung der Reste R⁴ bzw. R⁵.

Die Herstellung der Dipeptid-Verbindungen XIII kann in Analogie zur Herstellung der Verbindung V erfolgen. Die Herstellung ist in dem folgenden Schema skizziert:

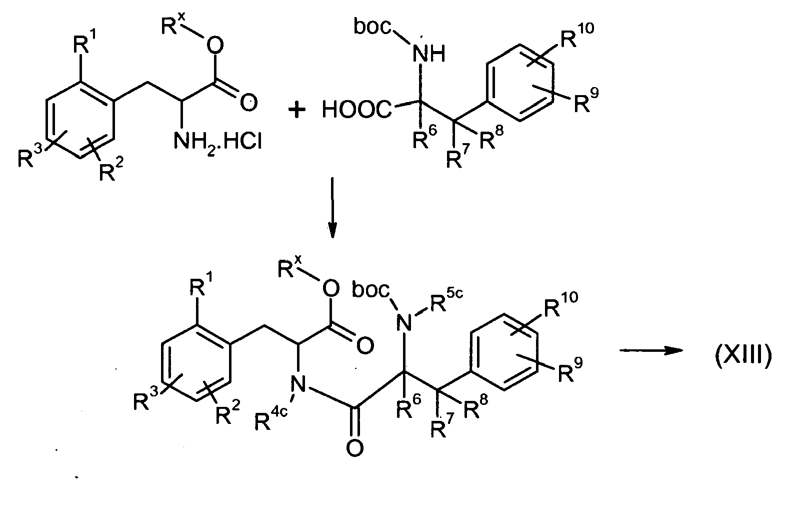

Bezüglich weiterer Details für den ersten Reaktionsschritt wird auf die Umsetzung von Verbindung VII mit der Verbindung VIII verwiesen. Die anschließende Entfernung der boc-Schutzgruppe kann in Analogie zur Umsetzung der Verbindung VI zur Verbindung V erfolgen. Die Cyclisierung der dabei erhaltenen entschützten Verbindung kann nach den für die Cyclisierung der Verbindung V genannten Methoden erfolgen.

Die Verbindungen der Formel I mit R⁵ ≠ H können auch dadurch hergestellt werden, dass man eine Piperazinverbindung der Formel I, worin R⁵ für Wasserstoff steht, mit einem Alkylierungsmittel oder Acylierungsmittel, das den von Wasserstoff verschiedenen Rest R⁵ enthält, umsetzt. Derartige Umsetzungen können in Analogie zu denjenigen Methoden durchgeführt werden, die im Zusammenhang mit Verfahren A Schritte ii), iiia) und iv) erläutert wurden.

Hierzu wird die Piperazinverbindung der Formel I mit R⁵ = Wasserstoff mit einem geeigneten Alkylierungsmittel, im Folgenden Verbindung X¹-R⁵, oder Acylierungsmittel, im Folgenden Verbindung X²-R⁵, umgesetzt, wobei man eine Piperazinverbindung der Formel I mit R⁵ ≠ Wasserstoff erhält.

In den Alkylierungsmitteln X¹-R⁵ kann X¹ Halogen oder O-SO₂-R^{m} mit R^{m} in der Bedeutung von C₁-C₄-Alkyl oder Aryl, welche gegebenenfalls durch Halogen, C₁-C₄-Alkyl oder Halo-C₁-C₄-alkyl substituiert sind, bedeuten. In Acylierungsmitteln X²-R⁵ kann X² Halogen, insbesondere Cl bedeuten. R⁵ steht dabei für einen Rest (CO)R⁵.

Die Umsetzung erfolgt üblicherweise bei Temperaturen im Bereich von -78°C bis zum Siedepunkt der Reaktionsmischung, bevorzugt von -50°C bis 65°C, insbesondere bevorzugt von -30°C bis 65°C. In der Regel wird die Umsetzung in einem Lösungsmittel vorgenommen, bevorzugt in einem inerten organischen Lösungsmittel.

Geeignete Lösungsmittel sind die bei der Cyclisierung des Dipeptids V zum Piperazin IV genannten Verbindungen, unter anderem Toluol, Dichlormethan, Tetrahydrofuran oder Dimethylformamid oder deren Mischungen.

In einer bevorzugten Ausführungsform wird die Verbindung I mit R⁵ = H mit dem Alkylierungs- bzw. Acylierungsmittel in Gegenwart einer Base umgesetzt. Geeignete Basen sind die bei der Cyclisierung des Dipeptids V zum Piperazin IV genannten Verbindungen. Die Basen werden im Allgemeinen äquimolar eingesetzt. Sie können auch im Überschuss oder selbst als Lösungsmittel verwendet werden. In einer bevorzugten Ausführungsform wird die Base in äquimolarer Menge oder im Wesentlichen äquimolarer Menge zugesetzt. In einer weiteren bevorzugten Ausführungsform wird Natriumhydrid als Base verwendet.

Die Alkylierung bzw. Acylierung der Gruppe NR⁵, worin R⁵ für H steht, kann alternativ auch in den Vorstufen erfolgen. So können z.B. Verbindungen II, IV, V, VI, VIII, in denen R^{5a} bzw. R^{5b} für H steht, wie zuvor beschrieben N-alkyliert oder N-acyliert werden. In gleicher Weise können Vorstufen II, IV, V, VI, VII, in denen der mit R⁴ bzw. R^{4a} bezeichnete Rest für Wasserstoff steht, alkyliert werden.

Die Verbindungen der Formel I können außerdem an der Gruppe R¹ modifiziert werden. So können beispielweise Verbindungen der Formel I, in denen R¹ für CN, gegebenenfalls substituiertes Phenyl oder für einen gegebenenfalls substituierten heterocyclischen Rest steht, ausgehend von Verbindungen I, worin R¹ für Halogen wie Chlor, Brom oder Jod steht, durch Umwandlung des Substituenten R¹ hergestellt werden, z.B. in Analogie zu den von J. Tsuji, Top. Organomet. Chem. (14) (2005), 332 pp., J. Tsuji, Organic Synthesis with Palladium Compounds, (1980), 207 pp., Tetrahedron Lett. 42, 2001, S.7473 oder Org. Lett. 5, 2003, 1785 beschriebenen Methoden.

Hierzu wird eine Piperazinverbindung der Formel I, die als Substituenten R¹ ein Halogenatom wie Chlor, Brom oder lod aufweist, durch Umsetzung mit einem Kupplungspartner, der eine Gruppe R¹ enthält (Verbindung R¹-X³), in ein anderes Piperazinderivat der Formel I überführt. In analoger Weise können auch Verbindungen la, II and IIa umgesetzt werden.

Die Umsetzung erfolgt üblicherweise in Gegenwart eines Katalysators, bevorzugt in Gegenwart eines Übergangsmetallkatalysators. In der Regel findet die Reaktion in Gegenwart einer Base statt.

Als Kupplungspartner X³-R¹ kommen insbesondere solche Verbindungen in Betracht, worin X³ im Falle von R¹ in der Bedeutung von Phenyl oder heterocyclischer Rest (Heterocyclyl) für eine der folgenden Gruppen steht:
- Zn-R¹ mit R¹ in der Bedeutung von Halogen, Phenyl oder Heterocyclyl;
- B(OR^{m})₂, mit R^{m} in der Bedeutung von H oder C₁-C₆-Alkyl, wobei zwei Alkylsubstituenten zusammen eine C₂-C₄-Alkylenkette bilden können; oder
- SnRn₃, mit Rₙ in der Bedeutung von C₁-C₆-Alkyl bedeutet.

Diese Umsetzungen erfolgt üblicherweise bei Temperaturen im Bereich von -78°C bis zum Siedepunkt der Reaktionsmischung, bevorzugt von -30°C bis 65°C, insbesondere bevorzugt bei Temperaturen von 30°C bis 65°C. In der Regel wird die Umsetzung in einem inerten organischen Lösungsmittel in Gegenwart einer Base durchgeführt.

Geeignete Lösungsmittel sind die bei der Cyclisierung des Dipeptids IV zum Piperazin V genannten Verbindungen. In einer Ausführungsform des erfindungsgemäßen Verfahrens wird Tetrahydrofuran mit einer katalytischen Menge Wasser verwendet; in einer anderen Ausführungsform wird nur Tetrahydrofuran eingesetzt.

Geeignete Basen sind die die bei der Cyclisierung des Dipeptids IV zum Piperazin V genannten Verbindungen.

Die Basen werden im Allgemeinen äquimolar eingesetzt. Sie können auch im Überschuss oder selbst als Lösungsmittel verwendet werden.

In einer bevorzugten Ausführungsform des erfindungsgemäßen Verfahrens wird die Base in äquimolarer Menge zugesetzt. In einer weiteren bevorzugten Ausführungsform werden Triethylamin oder Cäsiumcarbonat als Base verwendet, besonders bevorzugt Cäsiumcarbonat.

Als Katalysatoren für das erfindungsgemäße Verfahren sind prinzipiell Verbindungen der Übergangsmetalle Ni, Fe, Pd, Pt, Zr oder Cu geeignet. Es ist möglich, organische oder anorganische Verbindungen einzusetzen. Beispielhaft seien genannt : Pd(PPh₃)₂Cl₂, Pd(OAc)₂, PdCl₂, oder Na₂PdCl₄. Ph steht hierbei für Phenyl; Ac steht hierbei für Acetyl.

Die verschiedenen Katalysatoren können sowohl einzeln als auch als Gemische eingesetzt werden. In einer bevorzugten Ausführungsform der Erfindung wird Pd(PPh₃)₂Cl₂ verwendet.

Zur Herstellung der Verbindung I, worin R¹ für CN steht, kann man die Verbindung I, worin R¹ für Chlor, Brom oder lod steht, auch mit Kupfercyanid in Analogie zu bekannten Verfahren umsetzen (siehe beispielsweise Organikum, 21. Auflage, 2001, Wiley, S. 404, Tetrahedron Lett. 42, 2001, S.7473 oder Org. Lett. 5, 2003, 1785 und dort zitierte Literatur).

Diese Umsetzungen erfolgt üblicherweise bei Temperaturen im Bereich von 100°C bis zum Siedepunkt der Reaktionsmischung, bevorzugt von 100°C bis 250°C. In der Regel wird die Umsetzung in einem inerten organischen Lösungsmittel durchgeführt. Geeignete Lösungsmittel sind insbesondere aprotisch polare Lösungsmittel, z.B. Dimethylformamid, N-Methylpyrrolidon, N,N'-Dimethylimidazolidin-2-on und Dimethylacetamid.

Die Umwandlung der Gruppe R¹ kann alternativ auch bei den Vorstufen der Verbindung I erfolgen. So können z.B. Verbindungen II in denen R¹ für ein Halogenatom wie Chlor, Brom oder lod steht, der zuvor beschriebenen Umsetzung unterworfen werden.

Die Alkylierung bzw. Acylierung der Gruppe NR^{4a}, NR^{5a}, worin R^{4a} bzw. R^{5a} für H steht, kann alternativ auch in den Vorstufen erfolgen. So können z.B. Verbindungen II, IV, V, VI, VIII, in denen R^{5a} bzw. R^{5b} für H steht, wie zuvor beschrieben N-alkyliert oder N-acyliert werden. In gleicher Weise können Vorstufen II, IV, V, VI, VII, in denen der mit R⁴ bzw. R^{4a} bezeichnete Rest für Wasserstoff steht, alkyliert werden.

Die Verbindungen I und deren landwirtschaftlich brauchbaren Salze eignen sich - sowohl als Isomerengemische als auch in Form der reinen Isomeren - als Herbizide. Sie eignen sich als solche oder als entsprechend formuliertes Mittel. Die herbiziden Mittel, die die Verbindung I oder la enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I oder la bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, lpomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis und prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera, Zea mays.

Vorzugsweise kommen die folgenden Kulturen in Betracht: Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Darüber hinaus können die Verbindungen I auch in Kulturen, die durch Züchtung einschließlich gentechnischer Methoden gegen Insekten oder Pilzbefall tolerant sind, verwendet werden.

Des Weiteren wurde gefunden, dass die Verbindungen der Formel I auch zur Defoliation und/oder Desikkation von Pflanzenteilen geeignet ist, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und /oder Defoliation von Pflanzen, Verfahren zur Herstellung dieser Mittel und Verfahren zur Desikkation und/oder Defoliation von Pflanzen mit der Verbindungen der Formel I gefunden.

Als Desikkantien eignet sich die Verbindungen der Formel I insbesondere zur Austrocknung der oberirdischen Teile von Kulturpflanzen wie Kartoffel, Raps, Sonnenblume und Sojabohne aber auch Getreide. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, d.h., die Förderung der Ausbildung von Trenngewebe zwischen Frucht- oder Blatt- und Sprossteil der Pflanzen ist auch für ein gut kontrollierbares Entblättern von Nutzpflanzen, insbesondere. Baumwolle, wesentlich.

Außerdem führt die Verkürzung des Zeitintervalls, in dem die einzelnen Baumwollpflanzen reif werden, zu einer erhöhten Qualität der Faser nach der Ernte.

Die Verbindungen I bzw. die sie enthaltenden herbiziden Mittel können beispielsweise in Form von direkt versprühbaren wässrigen Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen, Gießen oder Behandlung des Saatgutes bzw. Mischen mit dem Saatgut angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsstoffe.

Beispiele für die Formulierung von Pflanzenschutzmitteln übliche Hilfsmittel sind inerte Hilfsstoffe, feste Trägerstoffe, oberflächenaktive Stoffe (wie Dispergiermittel Schutzkolloide, Emulgatoren, Netzmittel und Haftmittel), organische und anorganische Verdicker, Bakterizide, Frostschutzmittel, Entschäumer ggf. Farbstoffe und für Saatgutformulierungen Kleber.

Beispiele für Verdicker (d.h. Verbindungen, die der Formulierung ein modifiziertes Fließverhalten verleihen, d.h. hohe Viskosität im Ruhezustand und niedrige Viskosität im bewegten Zustand) sind Polysaccharide wie Xanthan Gum (Kelzan® der Fa. Kelco), Rhodopol® 23 (Rhone Poulenc) oder Veegum® (Firma R.T. Vanderbilt) sowie organische und anorganische Schichtmineralienwie Attaclay® (Firma Engelhardt).

Beispiele für Antischaummittel sind Silikonemulsionen (wie z.Bsp. Silikon® SRE, Firma Wacker oder Rhodorsil® der Firma Rhodia ), langkettige Alkohole, Fettsäuren, Salze von Fettsäuren, fluororganische Verbindungen und deren Gemische.

Bakterizide können zur Stabilisierung der wässrigen Herbizid-Formulierung zugesetzt werden. Beispiele für Bakterizide sind Bakterizide basierend auf Diclorophen und Benzylalkoholhemiformal (Proxel® der Fa. ICI oder Acticide® RS der Fa. Thor Chemie und Kathon® MK der Firma Rohm & Haas) sowie Isothiazolinonderivaten wie Alkylisothiazolinonen und Benzisothiazolinonen (Acticide MBS der Fa. Thor Chemie)

Beispiele für Frostschutzmittel sind Ethylenglycol, Propylenglycol, Harnstoff oder Glycerin.

Beispiele für Farbmittel sind sowohl in Wasser wenig lösliche Pigmente als auch in Wasser lösliche Farbstoffe. Als Beispiele genannt seien die unter den Bezeichnungen Rhodamin B, C.I. Pigment Red 112 und C.I. Solvent Red 1 bekannten Farbstoffe, sowie pigment blue 15:4, pigment blue 15:3, pigment blue 15;2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Beispiele für Kleber sind Polyvinylpyrrolidon, Polyvinylacetat, Polyvinylalkohol und Tylose.

Als inerte Zusatzstoffe kommen beispielsweise in Betracht:
Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nussschalenmehl, Cellulosepulver oder andere feste Trägerstoffe.

Als oberflächenaktive Stoffe (Adjuvantien, Netz-, Haft-, Dispergier- sowie Emulgiermittel) kommen die Alkali-, Erdalkali-, Ammoniumsalze von aromatischen Sulfonsäuren, z.B. Ligninsulfonsäuren (z.B. Borrespers-Typen, Borregaard), Phenolsulfonsäuren, Naphthalinsulfonsäuren (Morwet-Typen, Akzo Nobel) und Dibutylnaphthalinsulfonsäure (Nekal-Typen, BASF AG), sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol typen Clariant), Polycarboxylate (BASF AG, Sokalan-Typen), Polyalkoxylate, Polyvinylamin (BASF AG, Lupamin-Typen), Polyethylenimin (BASF AG, Lupasol-Typen), Polyvinylpyrrolidon und deren Copolymere in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, lmprägnierungs- und Homogengranulate können durch Bindung der Wirkstoffe an feste Trägerstoffe hergestellt werden.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen der Formel I oder la als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Die Konzentrationen der Verbindungen der Formel I in den anwendungsfertigen Zubereitungen können in weiten Bereichen variiert werden. Die Formulierungen enthalten im Allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die erfindungsgemäßen Verbindungen I können beispielsweise wie folgt formuliert werden:

### 1. Produkte zur Verdünnung in Wasser

### A Wasserlösliche Konzentrate

10 Gew.-Teile Wirkstoff werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

### B Dispergierbare Konzentrate

20 Gew.-Teile Wirkstoff werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%

### C Emulgierbare Konzentrate

15 Gew.-Teile Wirkstoff werden in 75 Gew.-Teilen eines organisches Lösungsmittel (z.B. Alkylaromaten)-unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

### D Emulsionen

25 Gew.-Teile Wirkstoff werden in 35 Gew.-Teilen eines organisches Lösungsmittel (z.B. Alkylaromaten) unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.-Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E Suspensionen

20 Gew.-Teile Wirkstoff werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-%.

### F Wasserdispergierbare und wasserlösliche Granulate

50 Gew.-Teile Wirkstoff werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.

### G Wasserdispergierbare und wasserlösliche Pulver

75 Gew.-Teile Wirkstoff werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### H Gelformulierungen

In einer Kugelmühle werden 20 Gew.-Teile Wirkstoff, 10 Gew.-Teile Dispergiermittel, 1 Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

### 2. Produkte für die Direktapplikation

### I Stäube

5 Gew.-Teile Wirkstoff werden fein gemahlen und mit 95 Gew.-Teilen feinteiligem Kaolin innig vermischt. Man erhält dadurch ein Stäubemittel mit 5 Gew.-% Wirkstoffgehalt.

### J Granulate (GR, FG, GG, MG)

0,5 Gew.-Teile Wirkstoff werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.

### K ULV- Lösungen (UL)

10 Gew.-Teile Wirkstoff werden in 90 Gew.-Teilen eines organischen Lösungsmittel z.B. Xylol gelöst. Dadurch erhält man ein Produkt für die Direktapplikation mit 10 Gew.-% Wirkstoffgehalt.

Die Applikation der Verbindungen I oder der sie enthaltenden herbiziden Mittel kann im Vorauflauf-, im Nachauflaufverfahren oder zusammen mit dem Saatgut einer Kulturpflanze erfolgen. Es besteht auch die Möglichkeit, die herbiziden Mittel bzw. Wirkstoffe dadurch zu applizieren, dass mit den herbiziden Mitteln bzw. Wirkstoffen vorbehandeltes Saatgut einer Kulturpflanze ausgebracht wird. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

In einer weiteren Ausführungsform kann die Applikation der Verbindungen der Formel I bzw. der herbiziden Mittel durch Behandlung von Saatgut erfolgen.

Die Behandlung von Saatgut umfasst im Wesentlichen alle dem Fachmann geläufigen Techniken (seed dressing, seed coating, seed dusting, seed soaking, seed film coating, seed multilayer coating, seed encrusting, seed dripping, und seed pelleting" ) basierend auf den erfindungsgemäßen Verbindungen der Formel I bzw. daraus hergestellten Mitteln. Hierbei können die herbiziden Mittel verdünnt oder unverdünnt aufgetragen werden.

Der Begriff Saatgut umfasst Saatgut aller Arten, wie z.B. Körner, Samen, Früchte, Knollen, Stecklinge und ähnliche Formen. Bevorzugt beschreibt der Begriff Saatgut hier Körner und Samen.

Als Saatgut kann Saatgut der oben erwähnten Nutzpflanzen aber auch das Saatgut transgener oder durch herkömmliche Züchtungsmethoden erhaltener Pflanzen eingesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.). Zur Saatgutbehandlung werden die Verbindungen I üblicherweise in Mengen von 0,001 bis 10 kg pro 100 kg Saatgut eingesetzt.

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3- -cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile sowie Phenylpyrazoline und Isoxazoline und deren Derivate in Betracht.

Außerdem kann es von Nutzen sein, die Verbindungen I allein oder in Kombination mit anderen Herbiziden oder auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch weitere Additive wie nicht phytotoxische Öle und Ölkonzentrate zugesetzt werden.

Es kann auch von Vorteil sein, die Verbindungen der Formel I in Kombination mit Safenern zu verwenden. Safener sind chemische Verbindungen, die Schaden an Nutzpflanzen verhindern oder reduzieren, ohne die herbizide Wirkung der Verbindungen der Formel I auf unerwünschte Pflanzen wesentlich zu beeinflussen. Sie können sowohl vor der Aussaat (beispielsweise bei Saatgutbehandlungen, bei Stecklingen, oder bei Setzlingen) als auch im Vor- oder Nachauflauf der Nutzpflanze verwendet werden. Die Safener und die Verbindungen der Formel I können gleichzeitig oder nacheinander verwendet werden. Geeignete Safener sind beispielsweise (Chinolin-8-oxy)essigsäuren, 1-Phenyl-5-haloalkyl-1H-1,2,4-triazol-3-carbonsäuren, 1-Phenyl-4,5-dihydro-5-alkyl-1 H-pyrazol-3,5-dicarbonsäuren, 4,5-Dihydro-5,5-diaryl-3-isoxazolcarbonsäuren, Dichloroacetamide, alpha-Oximinophenylacetonitrile, Acetophenonoxime, 4,6-Dihalo-2-phenylpyrimidine, N-[[4-(Aminocarbonyl)phenyl]sulfonyl]-2-benzoesäureamide, 1,8-Naphthalsäureanhydrid, 2-Halo-4-(haloalkyl)-5-thiazolcarbonsäuren, Phosphorthiolate und N-Alkyl-O-phenylcarbamate sowie ihre landwirtschaftlich brauchbaren Salze, und vorausgesetzt sie haben eine Säurefunktion, ihre landwirtschaftlich brauchbaren Derivate, wie Amide, Ester und Thioester.

Im Folgenden wird die Herstellung von Piperazinverbindungen der Formel I anhand von Beispielen erläutert ohne dabei den Gegenstand der vorliegenden Erfindung auf die gezeigten Beispiele zu begrenzen.

### Beispiele

Die Charakterisierung der im Folgenden gezeigten Produkte erfolgte durch Bestimmung des Schmelzpunktes, durch NMR-Spektroskopie oder anhand der durch HPLC-MS-Spektrometrie ermittelten Massen ([m/z]) oder Retentionszeit (RT; [min.]).

[HPLC-MS = High Performance Liquid Chromatographie kombiniert mit Massen Spektrometrie; HPLC-Säule: RP-18 Säule (Chromolith Speed ROD von Merck KgaA, Deutschland), 50*4,6 mm; Eluent: Acetonitril + 0,1 % Trifluoressigsäure (TFA)/ Wasser + 0,1 % TFA, mit einem Gradienten von 5 : 95 bis 100 : 0 in 5 Minuten bei 40°C, Flussrate 1,8 ml/min;
MS: Quadrupol Elektrospray-lonisation, 80 V (Positiv-Modus).]

### Beispiel 1: 2-[5-Benzyl-1,4,5-trimethyl-3,6-dioxopiperazin-2-ylmethyl]-benzonitril

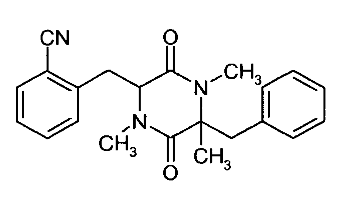

### 1.1 Herstellung von (2-tert-Butoxycarbonylamino-3-phenyl-propionylamino)-essigsäuremethylester

Zu einer Lösung von Glycinmethylester-Hydrochlorid (100 g, 0,8 mol) in Tetrahydrofuran (THF, 1000 ml) wurde bei 0 °C Ethyldiisopropylamin (259 g, 2,0 mol), N-tert-Butoxycarbonyl-L-phenylalanin (212 g, 0,8 mol) und 1-Ethyl-3-(3'-dimethylaminopropyl)carbodiimid (EDAC, 230 g, 1,2 mol) gegeben. Anschließend wurde das Reaktionsgemisch 24 h bei Raumtemperatur gerührt. Das erhaltene Reaktionsgemisch wurde unter vermindertem Druck von flüchtigen Bestandteilen befreit und der so erhaltene Rückstand wurde in Wasser (1000 ml) aufgenommen. Die wässrige Phase wurde mehrmals mit CH₂Cl₂ extrahiert. Die so erhaltenen organischen Phasen wurden vereint, mit Wasser gewaschen, über Na₂SO₄ getrocknet, filtriert und unter vermindertem Druck vom Lösungsmittel befreit. (2-tert-Butoxycarbonylamino-3-phenyl-propionylamino)-essigsäuremethylester wurde als gelbes Öl in einer Menge von 300 g erhalten. Das erhaltene Rohprodukt wurde ohne weitere Aufreinigung weiter umgesetzt.

### 1.2 Herstellung von 3-Benzylpiperazin-2,5-dion

Zu einer Lösung von (2-tert-Butoxycarbonylamino-3-phenyl-propionylamino)-essigsäuremethylester (300 g, ca. 0,8 mol) in CH₂Cl₂ wurde bei Raumtemperatur Trifluoressigsäure (342 g, 3 mol) getropft. Das erhaltene Reaktionsgemisch wurde für 24 h bei Raumtemperatur gerührt und anschließend unter vermindertem Druck eingeengt. Der erhaltene Rückstand wurde in THF (500 ml) aufgenommen und langsam mit einer wässrigen Ammoniak-Lösung (25 %ig, 500 ml) versetzt. Das Reaktionsgemisch wurde weitere 72 h bei Raumtemperatur gerührt. Der ausgefallene Feststoff wurde durch Filtrieren isoliert und mit Wasser gewaschen. 3-Benzylpiperazin-2,5-dion wurde in einer Menge von 88 g (Ausbeute 54 %) erhalten.

### 1.3 Herstellung von 1,4-Diacetyl-3-benzyl-piperazin-2,5-dion

Eine Lösung von 3-Benzyl-piperazin-2,5-dion (20,4 g, 0,1 mol) in Essigsäureanhydrid (200 ml) wurde unter Rückflussbedingungen für 4 h gerührt. Das erhaltene Reaktionsgemisch wurde unter vermindertem Druck eingeengt. Der Rückstand wurde in CH₂Cl₂ aufgenommen, nacheinander mit einer wässrigen NaHCO₃-Lösung und Wasser gewaschen, über Na₂SO₄ getrocknet, filtriert und unter vermindertem Druck vom Lösungsmittel befreit. 1,4-Diacetyl-3-benzyl-piperazin-2,5-dion wurde als gelbes Öl in einer Menge von 28,5 g (quantitativ) erhalten und als Rohprodukt weiter umgesetzt.
HPLC-MS [m/z]: 289.1 [M+1]⁺.

### 1.4 Herstellung von 1-Acetyl-6-benzyl-3-(2-brom-benzyliden)-piperazin-2,5-dion

Zu einer Lösung von 1,4-Diacetyl-3-benzyl-piperazin-2,5-dion (17,4 g, 0,06 mol) in Dimethylformamid (DMF, 100 ml) wurde Brombenzaldehyd (5,55 g, 0,03 mol) und Cs₂CO₃ (9,8 g, 0,03 mol) gegeben. Das Reaktionsgemisch wurde für 36 h bei Raumtemperatur gerührt, anschließend mit Wasser (500 ml) und Zitronensäure (10 g) versetzt und mehrmals mit CH₂Cl₂ extrahiert. Die so erhaltenen organischen Phasen wurden vereint, mit Wasser gewaschen, über Na₂SO₄ getrocknet, filtriert und unter vermindertem Druck vom Lösungsmittel befreit. 1-Acetyl-6-benzyl-3-(2-brom-benzyliden)-piperazin-2,5-dion wurde nach säulechromatographischer Reinigung (CH₂Cl₂ als Eluent) als gelbes Öl in einer Menge von 12 g (Ausbeute 48 %) erhalten.
HPLC-MS [m/z]: 413.9 [M+1]⁺.

### 1.5 Herstellung von 3-Benzyl-6-(2-brom-benzyliden)-piperazin-2,5-dion

Zu einer Lösung von 1-Acetyl-6-benzyl-3-(2-brom-benzyliden)-piperazin-2,5-dion (12 g, 0,03 mol) in THF (50 ml) wurde verdünnte wässrige HCl-Lösung (5 %ig, 250 ml) gegeben. Das Reaktionsgemisch wurde 8 h unter Rückflussbedingungen gerührt. Nach Abkühlen der Reaktionslösung wurde der ausgefallene Feststoff durch Filtrieren isoliert. Der so erhaltene Feststoff wurde mit Wasser und THF gewaschen. 3-Benzyl-6-(2-brom-benzyliden)-piperazin-2,5-dion wurde als farbloser Feststoff in einer Menge von 8.3 g (Ausbeute 75 %) erhalten.
HPLC-MS [m/z]: 371.2 [M]⁺.

### 1.6 3-Benzyl-6-(2-brom-benzyliden)-1,3,4-trimethyl-piperazin-2,5-dion

Zu einer Lösung von 3-Benzyl-6-(2-brombenzyliden)-piperazin-2,5-dion (2,00 g, 5,4 mmol) in DMF (50 ml) wurde bei 0 °C NaH (0,85 g, 60 %ig, 21 mmol) gegeben. Das Reaktionsgemisch wurde 2 h bei 0 °C gerührt und anschließend mit Mel (5.0 g, 35 mmol) versetzt. Das Reaktionsgemisch wurde für weitere 18 h bei Raumtemperatur gerührt und anschließend mit Wasser versetzt. Es wurde mehrfach mit Methyl-tert-butylether extrahiert. Die so erhaltenen organischen Phasen wurden vereint, mit Wasser gewaschen, über Na₂SO₄ getrocknet, filtriert und unter vermindertem Druck vom Lösungsmittel befreit. 3-Benzyl-6-(2-brombenzyliden)-1,3,4-trimethyl-piperazin-2,5-dion wurde nach säulenchromatographischer Reinigung in einer Menge von 1,6 g (Ausbeute 72 %) erhalten. HPLC-MS [m/z]: 413.0 [M]⁺.

### 1.7 Herstellung von 2-(5-Benzyl-1,4,5-trimethyl-3,6-dioxo-piperazin-2-ylidenmethyl)-benzonitril

Zu einer Lösung von 3-Benzyl-6-(2-brom-benzyliden)-1,3,4-trimethyl-piperazin-2,5-dion (1,5 g, 3,6 mmol) in N-Methylpyrrolidin (NMP, 25 ml) wurde CuCN (0,7 g, 7,8 mmol) gegeben. Das Reaktionsgemisch wurde 16 h bei 155 °C gerührt und nach Abkühlung auf Raumtemperatur in Essigester eingebracht. Das Reaktionsgemisch wurde mit Methyl-tert-butylether verdünnt. Die so erhaltene organische Phase wurde mit Wasser gewaschen, über Na₂SO₄ getrocknet, filtriert und unter vermindertem Druck vom Lösungsmittel befreit. Nach säulenchromatographischer Reinigung wurde 2-(5-Benzyl-1,4,5-trimethyl-3,6-dioxo-piperazin-2-ylidenmethyl)-benzonitril in einer Menge von 0,79 g (Ausbeute 61 %) erhalten. HPLC-MS [m/z]: 360.5 [M+1]⁺.

### 1.8 Herstellung von 2-[5-Benzyl-1,4,5-trimethyl-3,6-dioxopiperazin-2-ylmethyl]-benzonitril

Zu einer Lösung von 2-(5-Benzyl-1,4,5-trimethyl-3,6-dioxo-piperazin-2-ylidenmethyl)-benzonitril (0,5 g, 1,4 mmol) in MeOH (Methanol, 40 ml) wurde unter Stickstoff Pd auf Aktivkohle (0,1 g) als Suspension in MeOH (2 ml) gegeben. Die erhaltene Suspension wurde für 7 h unter H₂-Atmosphäre hydriert. Das erhaltene Reaktionsgemisch wurde über Celite filtriert. Das Filtrat wurde unter vermindertem Druck vom Lösungsmittel befreit. Das so erhaltene Rohprodukt wurde säulenchromatographischer aufgereinigt. Es wurden 2 Isomere erhalten, die mittels HPLC-MS untersucht wurden.
Hauptisomer 1: HPLC-MS: [m/z] = 362,1 [M+H]⁺; RT = 2,834 min;
Nebenisomer 2: HPLC-MS: [m/z] = 362,1 [M+H]+; RT = 2,657 min.

### Beispiel 2: Alternative Herstellung von 2-[5-Benzyl-1,4,5-trimethyl-3,6-dioxopiperazin-2-ylmethyl]-benzonitril

### 2.1 Herstellung von N-(Diphenylmethylen)ethylglycinat

Ethylglycinat Hydrochlorid (37 g, 0,27 mol) wurde in einer Lösung von K₂CO₃ (74,4 g, 0,54 mol) in Wasser (186 ml) gelöst. Die Lösung wurde 15 min gerührt und anschließend mit Dichlormethan (10*150 ml) extrahiert. Die so erhaltenen organischen Phasen wurden vereint, über MgSO₄ getrocknet und unter vermindertem Druck (500 mbar) vom Lösungsmittel befreit (Ausbeute - 50 %). Der Rückstand (9,5 g, 0,092 mol) wurde zusammen mit Benzophenon (14,03g, 0,077 mol) in Xylol (76 ml) gelöst. Nach Zugabe einiger Tropfen BF₃*Et₂O wurde das Reaktionsgemisch 5 h unter Rückflussbedingungen am Wasserabscheider gerührt. Nach Abkühlen des Reaktionsgemisches auf Raumtemperatur wurde das Lösungsmittel unter vermindertem Druck entfernt. N-(Diphenylmethylen)ethylglycinat wurde aus dem erhaltenen Rückstand durch Destillation (80 °C bei 5,5*10⁻² mbar) in einer Ausbeute von 48 % isoliert.

### 2.2 Herstellung von N-(Diphenylmethylen)- α-(2-cyanophenyl)ethylalaninat

Eine Lösung von N-(Diphenylmethylen)ethylglycinat (5 g, 18,7 mmol; aus Beispiel 2.1), 2-Cyanobenzylbromid (4,1 g, 20,7 mmol) und Tetrabutylammoniumsulfat (320 mg, 0,9 mmol) in Dichlormethan (40 ml) wurde mit wässriger Natronlauge NaOH (10 %ig, 40 ml) versetzt und über Nacht bei Raumtemperatur gerührt. Nach Trennung der Phasen wurde die wässrige Phase mit Dichlormethan (2 mal 50ml) extrahiert. Die erhaltenen organischen Phasen wurden vereint, bis zur Neutralität der Waschphase mit Wasser gewaschen, über MgSO₄ getrocknet, filtriert und unter vermindertem Druck vom Lösungsmittel befreit. N-(Diphenylmethylen)-α-(2-cyanophenyl)ethylalaninat wurde aus dem erhaltenen Rückstand durch Flash-Chromatographie (SiO₂ Cyclohexan/Ethylacetat) in einer Ausbeute von 83% isoliert.

### 2.3 Herstellung von α-(2-Cyanophenyl)ethylalaninat Hydrochlorid

Zu einer Lösung von N-(Diphenylmethylen)-α-(2-cyanophenyl)ethylalaninat (11,4 g, 29,8 mmol; aus Beispiel 2.2) in Aceton (95 ml) wurde wässrige HCl (1 M, 95 ml) gegeben. Das Gemisch wurde für 3 h bei Raumtemperatur gerührt und anschließend unter vermindertem Druck vom Lösungsmittel befreit. Der erhaltene Rückstand wurde mit Diethylether (2 mal 50 ml) versetzt. Die überstehende Flüssigkeit wurde abdekantiert. Bei dem zurückbleibenden Feststoff handelt es sich um α-(2-Cyanophenyl)ethylalaninat Hydrochloride, das ohne weitere Aufreinigung im darauffolgenden Schritt verwendet werden kann (Ausbeute 87 %).

### 2.4 Herstellung von N-(tert-Butoxycarbonyl)-α-methylphenylalanin

Zu einer Suspension von α-Methylphenylalanin (20 g, 0,11 mol) in Dioxan/Wasser (2 : 1, 300 ml) wurde wässrige Natronlauge (1 M, 170 ml) zugegeben. Zu diesem Reaktionsgemisch wurde eine Lösung von Di-tert-Butyldicarbonat (29,2 g, 0,134 mol) in Dioxan (50 ml) bei einer Temperatur von 0 °C langsam zugetropft. Nach beendeter Zugabe wurde das Reaktionsgemisch über Nacht bei Raumtemperatur gerührt. Die Umsetzung wurde mittels LC-MS-Analytik überwacht. Jeweils ein halbes Äquivalent Di-tert-Butyldicarbonat wurde solange zugegeben bis kein Ausgangsmaterial mehr nachweisbar war. Dabei wurde jeweils der pH-Wert mit wässriger Natronlauge NaOH (1 M) auf 9 eingestellt. Anschließend wurde das Reaktionsgemisch mit 10%iger wässriger Salzsäure auf einen pH-Wert von 2 eingestellt und mit Ethylacetat extrahiert. Die erhaltenen organischen Phasen wurden vereint, mit Wasser gewaschen, über MgSO₄ getrocknet, filtriert und unter vermindertem Druck vom Lösungsmittel befreit. Das als Rückstand in einer Ausbeute von 88 % erhaltene N-(tert-Butoxycarbonyl)-α-methylphenylalanin kann ohne weitere Aufreinigung im darauffolgenden Schritt verwendet werden.

### 2.5 Herstellung von (N-Boc-α-CHs-Phe)-(o-CN-Phe)-OC₂H₅

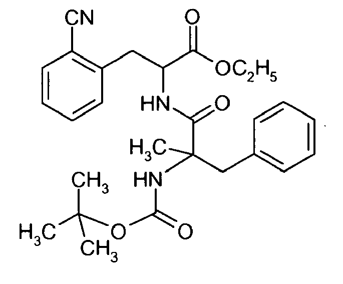

Zu einer Suspension von N,N'-Carbonyldiimidazol (CDI, 3,7 g, 27,1 mmol) in Tetrahydrofuran (THF, 34 ml) wurde eine Lösung von N-(tert-Butoxycarbonyl)-α-methylphenylalanin (6,3 g, 22,6 mmol) in THF (13 ml) bei 0 °C unter N₂-Atmosphäre gegeben. Das Reaktionsgemisch wurde bei Raumtemperatur für 8 h gerührt. Anschließend wurde α-(2-Cyanophenyl)ethylalaninat Hydrochlorid (8,6 g, 33,8 mmol) portionsweise zugegeben und anschließend wurde Diisopropylethylamin (DIPEA, 8,7 g, 67,6 mmol). Das Reaktionsgemisch wurde über Nacht bei 45°C und anschließend für 2 h unter Rückflussbedingungen gerührt. Das Reaktionsgemisch wurde auf wässrige 5%ige Zitronensäure gegossen und anschließend mit Ethylacetat extrahiert. Die erhaltenen organischen Phasen wurden vereint, mit ges. wässriger NaHCO₃-Lösung gewaschen, über MgSO₄ getrocknet, filtriert und unter vermindertem Druck vom Lösungsmittel befreit. (N-Boc-α-CH₃-Phe)-(o-CN-Phe)-OC₂H₅ wurde aus dem Rückstand durch Flash-Chromatographie (SiO₂ Cyclohexan/Ethylacetat) in einer Ausbeute von etwa 40 % erhalten.

### 2.6 Herstellung von (α-CH₃-Phe)-(o-CN-Phe)-OH

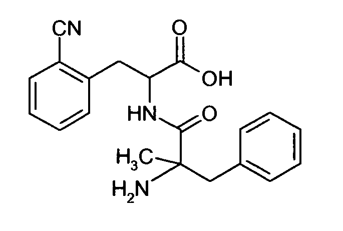

Zu einer Lösung von (N-Boc-α-CH₃-Phe)-(o-CN-Phe)-OC₂H₅ (4,1 g, 8,5 mmol) in Dichlormethan (14 ml) wurde Trifluoressigsäure (TFA, 8,20 g, 71,9 mmol) gegeben. Das Reaktionsgemisch wurde für 2 h bei Raumtemperatur gerührt und anschließend unter vermindertem Druck von flüchtigen Bestandteilen befreit. Der Rückstand wurde in Chloroform aufgenommen. Das Reaktionsgemisch wurde mit ges. wässriger Na₂CO₃-Lösung gewaschen. Die organische Phase wurde über MgSO₄ getrocknet, filtriert und unter vermindertem Druck vom Lösungsmittel befreit. Der erhaltene Rückstand (~1 g) wurde in einem Gemisch von Tetrahydrofuran/Natronlauge (2M) (1 : 1, 10 ml) bei einer Temperatur von 0 °C aufgenommen. Das Gemisch wurde 2 h bei dieser Temperatur gerührt. Anschließend wurde der pH-Wert mit Salzsäure (10%ig) auf 7 eingestellt. Das Gemisch wurde mit Ethylacetat gewaschen. Die erhaltene wässrige Phase wurde unter vermindertem Druck getrocknet. Der Rückstand bestand aus (α-CH₃-Phe)-(o-CN-Phe)-OH und Salzen aus der Neutralisation. Ausbeute: 1.2 g (<40%).

### 2.7 Herstellung von 2-(5-Benzyl-3,6-dioxo-5-methylpiperazin-2-ylmethyl)-benzonitril

Eine Suspension von (α-CH₃-Phe)-(o-CN-Phe)-OH (0,92 g, 2,6 mmol) und di(N-succinimidyl)carbonat (0,8 g, 3,1 mmol) in trockenem Acetonitril (35 ml) wurde unter N₂-Atmosphäre für 12 h bei Raumtemperatur gerührt. Anschließend wurde zu dem Reaktionsgemisch Diisopropylethylamin (DIPEA, 0,47 ml, 2,6 mmol) gegeben. Das Reaktionsgemisch wurde weitere 12 h bei Raumtemperatur gerührt. Das Lösungsmittel wurde unter vermindertem Druck entfernt. Der Rückstand wurde in Wasser (2x5 ml) aufgenommen und gerührt. Der ausfallende Feststoff wurde durch Filtrieren isoliert. 2-(5-Benzyl-3,6-dioxo-5-methylpiperazin-2-ylmethyl)-benzonitril wurde durch präparative HPLC-Chromatographie (RP; Eluent: Wasser/Acetonitril) aus dem Feststoff in einer Menge von 315 mg (Ausbeute 36 %) isoliert.

### 2.8 Herstellung von 2-(5-Benzyl-1,4,5-trimethyl-3,6-dioxopiperazin-2-ylmethyl)-benzonitril

Eine Lösung von 2-(5-Benzyl-3,6-dioxo-5-methylpiperazin-2-ylmethyl)-benzonitril (0,3 g, 0,9 mmol) in trockenem Dimethylformamid (DMF) wurde bei 0 °C unter N2-Atmosphäre mit NaH (144 mg, 3,6 mmol) versetzt und 1 h bei dieser Temperatur gerührt. Anschließend wurde Mel (0,77 g, 5,4 mmol) zugegeben. Nach einstündigem Rühren des Reaktionsgemisches bei Raumtemperatur wurde das Lösungsmittel unter vermindertem Druck entfernt. Der erhaltene Rückstand wurde durch präparative HPLC-Chromatographie (RP; Eluent: Wasser/Acetonitril) aufgetrennt. 2-(5-Benzyl-1,4,5-trimethyl-3,6-dioxopiperazin-2-ylmethyl)-benzonitril wurde in einer Menge von 77 mg als Gemisch zweier Diastereomere erhalten. Die Diasteromere wurden durch präparative Dünnschicht-Chromatographie (SiO₂, Cyclohexan/Ethylacetat 1 : 3) isoliert. Das erste Diastereomer wurde in einer Menge von 6 mg erhalten (R_{f} = 0,25). Das zweite Diastereomer wurde in einer Menge von 24 mg erhalten (R_{f} = 0,12). Dies entspricht einer Ausbeute von10 %.

### Beispiel 3: 3-Benzyl-6-(2,3-dichlorbenzyl)-1,3,4-trimethylpiperazin-2,5-dion

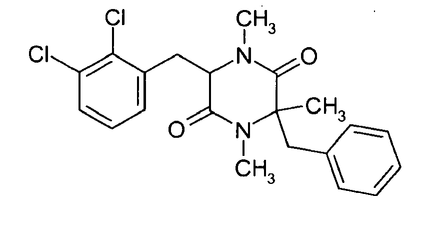

### 3.1 Herstellung von 3-Benzyl-1,3,4-trimethylpiperazin-2,5-dion

Zu einer Lösung von 3-Benzyl-3-methyl-piperazin-2,5-dion (9,8 g, 0,045 mol) in Dimethylformamid (450 mL) gab man portionsweise NaH (3,96 g, 60%ig, 0,1 mol) bei 0°C. Das Reaktionsgemisch wurde 30 min bei 0°C gerührt und anschließend mit Methyliodid (14,05 g, 0,1 mol) versetzt. Das Reaktionsgemisch wurde bei Raumtemperatur eine weitere Stunde gerührt und anschließend mit einer gesättigten Lösung von NH₄OH versetzt. Man extrahierte mehrmals mit CH₂Cl₂. Die organischen Phasen wurden vereint, mit Wasser gewaschen, über Na₂SO₄ getrocknet, filtriert und unter vermindertem Druck von Lösungsmittel befreit. Der erhaltene Feststoff wurde mit kaltem CH₂Cl₂ gewaschen. Man erhielt 3-Benzyl-1,3,4-trimethylpiperazin-2,5-dion als hellen Feststoff in einer Menge von 9,6 g (Ausbeute 87 %).
HPLC-MS [m/z]: 247,1 [M+H]+

### 3.2 Herstellung von 3-Benzyl-6-(2,3-dichlorbenzyl)-1,3,4-trimethylpiperazin-2,5-dion

Unter Argon wurden zu einer Lösung von 170 mg (0,7 mmol) 3-Benzyl-1,3,4-trimethyl-piperazin-2,5-dion in 2 ml Dimethylformamid (DMF) 35 mg NaH (60%ig) gegeben. Man rührte die Reaktionsmischung 10 min bei Raumtemperatur. Danach gab man 167 mg (0,7 mmol) 1-Brommethyl-2,3-dichlorbenzol zu und rührte weitere 48 Stunden bei Raumtemperatur. Man versetzte die Reaktionsmischung mit Wasser und extrahierte danach mit Dichlormethan. Das rohe Produkt wurde mittels präparativer HPLC gereinigt, wobei man die Titelverbindung in 20%iger Ausbeute erhielt.

Die Herstellung der in Tabelle 1 zusammengestellten Verbindungen der Formel I (Beispiele 4 bis 39) erfolgte in Analogie zu den zuvor gezeigten Beispielen 1, 2 und 3.

**Tabelle 1: Verbindungen der allgemeinen Formel I, worin R⁴ für CH₃ steht und R⁷, R⁸, R⁹ und R¹⁰ jeweils für Wasserstoff stehen**

| | | | | | | | |
|---|---|---|---|---|---|---|---|
| 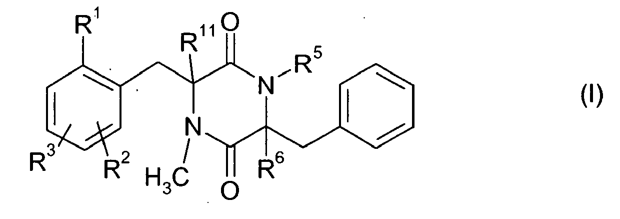 | | | | | | | |

| Nr. | R¹ | R² | R³ | R⁵ | R⁶ | R¹¹ | Schmp. [°C] und/oder RT, [m/z] |
|---|---|---|---|---|---|---|---|
| 1*) | CN | H | H | CH₃ | CH₃ | H | 2,834 min m/z= 362,1 [M+H] |
| 1**) | CN | H | H | CH₃ | CH₃ | H | 2,657 min m/z= 362,1 [M+H] |
| 4 | CN | H | H | CH₃ | CH₃ | CH₃ | 130-132°C 2,968 min m/z= 376,1 [M+H] |
| 5 | NO₂ | H | H | CH₃ | CH₃ | H | 3,245 min m/z= 382,8 [M+H] |
| 6 | Cl | H | H | CH₃ | CH₃ | H | 3,635 min m/z= 372,3 [M+H] |
| 7 | Cl | 6-Cl | H | CH₃ | CH₃ | H | 3,785 min m/z= 406,1 [M+H] |
| 8 | NO₂ | 6-Cl | H | CH₃ | CH₃ | H | 3,606 min m/z= 416,12 [M+H] |
| 9 | Cl | 5-CF₃ | H | CH₃ | CH₃ | H | 3,580 min m/z= 439,3 [M+H] |
| 10 | F | 5-Cl | H | CH₃ | CH₃ | H | 3,695 min m/z= 390,9 [M+H] |
| 11 | Br | 5-F | H | CH₃ | CH₃ | H | 3,733 min m/z= 436,12 [M+2] |
| 12 | F | 3-Cl | H | CH₃ | CH₃ | H | 3,700 min m/z= 390,2 [M+H] |
| 13 | F | 5-F | 6-Cl | CH₃ | CH₃ | H | 3,642 min m/z= 408,1 [M+H] |
| 14 | F | 5-CH₃ | 6-F | CH₃ | CH₃ | H | 3,686 min m/z= 388,3 [M+2] |
| 15 | F | 5-CF₃ | H | CH₃ | CH₃ | H | 3,758 min m/z= 424,2 [M+2] |
| 16 | F | 3-F | H | CH₃ | CH₃ | H | 3,501 min m/z= 474,2 [M+2] |
| 17 | F | 5-CF₃ | 3-Cl | CH₃ | CH₃ | H | 4,041 min m/z= 458,3 [M+H] |
| 18 | F | 3-CF₃ | H | CH₃ | CH₃ | H | 3,754 min m/z= 424,2 [M+2] |
| 19 | F | 5-CH₃ | 6-Cl | CH₃ | CH₃ | H | 3,901 min m/z= 404,2 [M+2] |
| 20 | Br | H | H | CH₃ | CH₃ | H | 3,777 min m/z= 415,8 [M+H] |
| 21 | F | H | H | CH₃ | CH₃ | H | 3,641 min m/z= 355,9 [M+H] |
| 22 | F | 4-F | H | CH₃ | CH₃ | H | 3,189 min m/z= 372,9 [M+H] |
| 23 | NO₂ | 3-Cl | H | CH₃ | CH₃ | H | 3,663 min m/z= 416,9 [M+H] |
| 24 | Cl | 3-CH(CH₃)₂ | H | CH₃ | CH₃ | H | 4,346 min m/z= 413,9 [M+H] |
| 25 | Cl | 3-CF₃ | H | CH₃ | CH₃ | H | 4,024 min m/z= 439,8 [M+H] |
| 26 | Cl | 4-F | H | CH₃ | CH₃ | H | 3,629 min m/z= 390,2 [M+2] |
| 27 | F | 5-F | H | CH₃ | CH₃ | H | 3,014 min m/z= 373,1 [M+H] |
| 28 | F | 6-F | H | CH₃ | CH₃ | H | 3,384 min m/z= 373,8 [M+H] |
| 29 | Cl | 4-OCH₃ | 3-Cl | CH₃ | CH₃ | H | 3,161 min m/z= 437,7 [M+2] |
| 30 | F | 4-F | 6-F | CH₃ | CH₃ | H | 3,483 min m/z= 391,7 [M+H] |
| 31 | F | 3-F | 4-F | CH₃ | CH₃ | H | 3,539 min m/z= 391,7 [M+H] |
| 32 | Cl | 6-F | H | CH₃ | CH₃ | H | 3,062 min m/z= 388,8 [M+] |
| 33 | F | 4-CF₃ | H | CH₃ | CH₃ | H | 3,746 min m/z= 423,7 [M+H] |
| 34 | I | H | H | CH₃ | CH₃ | H | 3,235 min m/z= 462,6 [M+H] |
| 35 | Cl | 4-NO₂ | 5-Cl | CH₃ | CH₃ | H | 3,638 min m/z= 452,0 [M+2] |
| 36 | Cl | 6-F | H | CH₃ | CH₃ | H | 2,926 min m/z= 388,7 [M] |
| 37 | Cl | 6-CH₃ | 5-Cl | CH₃ | CH₃ | H | 3,134 min m/z= 421,5 [M+H] |
| 38 | Cl | 5-Cl | H | CH₃ | CH₃ | H | 4,006 min m/z= 405,8 [M+H] |
| 39 | C₆H₅ | H | H | CH₃ | CH₃ | H | 4,033 min m/z= 413,9 [M+H] |

| | | | | | | | |
|---|---|---|---|---|---|---|---|
| Mit Ausnahme der mit *) und **) gekennzeichneten Verbindungen erhielt man jeweils ein Isomerengemisch, das nicht aufgetrennt wurde. *) (S,S)-1 (Hauptisomer 1) **) (R,S)-I (Nebenisomer 2) Schmp. Schmelzpunkt RT Retentionszeit, HPLC | | | | | | | |

### Anwendungsbeispiele

Die herbizide Wirkung der Verbindungen der Formel 1 ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf. Eine gute herbizide Aktivität ist bei Werten von wenigstens 70 und eine sehr gute herbizide Aktivität ist bei Werten von wenigstens 85 gegeben.

Die in den Gewächshausversuchen verwendeten Pflanzen setzten sich aus folgenden Arten zusammen:

| Bayer Code | Lateinischer Name | Deutscher Name | Englischer Name |
|---|---|---|---|
| AMARE | Amaranthus retofle xus | Zurückgekrümmter Fuchsschwanz | redroot pigweed |
| ALOMY | Alopecurus myosu roides | Ackerfuchsschwanzgras | black grass |
| APESV | Apera spica-venti | Gemeiner Windhalm | windgrass |
| CHEAL | Chenopodium album | Weißer Gänsefuß | common lambs-quarters |
| ECHCG | Echinochloa crus galli | Hühnerhirse | barnyard grass |
| SETFA | Setaria faberi | Große Borstenhirse | giant foxtail |
| SETVI | Setaria viridis | Grüne Borstenhirse | green foxtail |

Die erfindungsgemäßen Verbindungen zeigen eine sehr gute herbizide Wirkung im Vorauflaufverfahren.

Bei einer Aufwandmenge von 1,0 kg/ha wies die Verbindung des Beispiels 1 (Hauptisomer 1) im Vorauflauf gegen AMARE, ALOMY, APESV, ECHCG und SETFA eine sehr gute herbizide Wirkung auf.

Bei einer Aufwandmenge von 0,5 kg/ha wies die Verbindung des Beispiels 1 (Nebenisomer 2) im Vorauflauf gegen ALOMY, APESV und ECHCG eine sehr gute herbizide Wirkung auf.

Die erfindungsgemäßen Verbindungen zeigen eine sehr gute herbizide Wirkung im Nachauflaufverfahren.

Bei einer Aufwandmenge von 1,0 kg/ha wies die Verbindung des Beispiels 1 (Hauptisomer 1) im Nachauflauf gegen AMARE, CHEAL, ECHCG und SETVI eine sehr gute herbizide Wirkung auf.

Bei einer Aufwandmenge von 0,5 kg/ha wies die Verbindung des Beispiels 1 (Nebenisomer 2) im Nachauflauf gegen ALOMY, APESV und ECHCG eine sehr gute herbizide Wirkung auf.

## Patentansprüche

1. Piperazinverbindungen der Formel I 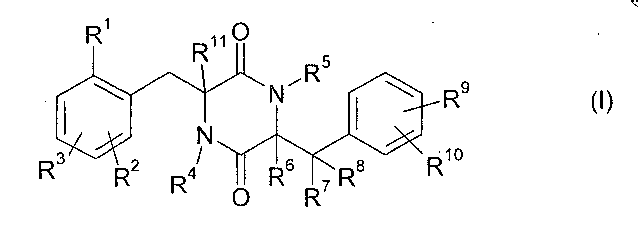 worin
R¹ ausgewählt ist unter Halogen, Cyano, Nitro, Z-C,(=O)-R¹², Phenyl und ei- nem 5- oder 6-gliedrigen heterocyclischen Rest, der 1, 2, 3 oder 4 Hetero- atom, ausgewählt unter O, N und S als Ringatome aufweist, worin Phenyl und der heterocyclische Rest unsubstituiert sind oder 1, 2, 3 oder 4 Substi- tuenten R^{1a} aufweisen können, die unabhängig voneinander unter Halogen, CN, NO₂, C₁-C₄-Alkyl, C₁-C₄-Haloalkyl, C₁-C₄-Alkoxy und C₁-C₄-Haloalkoxy ausgewählt sind, und worin
Z für eine kovalente Bindung oder eine CH₂-Gruppe steht;
R¹² Wasserstoff, C₁-C₆-Alkyl, C₃-C₆-Cycloalkyl, C₂-C₆-Alkenyl, C₅-C₆-
Cycloalkenyl, C₂-C₆-Alkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, C₁-C₆-Alkylsulfonylamino, C₁-C₆- Alkylaminosulfonylamino, [Di-(C₁-C₆)-alkylamino]sulfonylamino, C₃- C₆-Alkenylamino, C₃-C₆-Alkinylamino, N-(C₂-C₆-Alkenyl)-N-(C₁-C₆- alkyl)-amino, N-(C₂-C₆-Alkinyl)-N-(C₁-C₆-alkyl)-amino, N-(C₁-C₆- Alkoxy)-N-(C₁-C₆-alkyl)-amino, N-(C₂-C₆-Alkenyl)-N-(C₁-C₆-alkoxy)- amino, N-(C₂-C₆-Alkinyl)-N-(C₁-C₆-alkoxy)-amino, Phenyl, Phenoxy oder Phenylamino bedeutet;
wobei die Alkylteile in den unter R¹² aufgeführten Resten teilweise oder vollständig halogeniert sein können und die Phenylteile in den unter R¹² aufgeführten Resten 1, 2, 3 oder 4 Substituenten R^{12a} tragen können, die unter Halogen, CN, NO₂, C₁-C₄-Alkyl, C₁-C₄-Haloalkyl, C₁-C₄-Alkoxy und C₁-C₄-Haloalkoxy ausgewählt sind;
R² Wasserstoff, Halogen, Nitro, Cyano, C₁-C₄-Alkyl, C₁-C₄-Haloalkyl, C₂-C₄- Alkenyl, C₁-C₄-Alkoxy, C₁-C₄-Haloalkoxy, Benzyl oder eine Gruppe S(O)ₙR²¹ bedeutet, worin R²¹ für C₁-C₄-Alkyl oder C₁-C₄-Haloalkyl steht und n für 0, 1 oder 2 steht;
R³ Wasserstoff oder Halogen bedeutet;
R⁴ C₁-C₄-Alkyl, C₃-C₄-Alkenyl oder C₃-C₄-Alkinyl bedeutet;
R⁵ Wasserstoff, C₁-C₄-Alkyl, C₃-C₄-Alkenyl, C₃-C₄-Alkinyl oder eine Gruppe C(=O)R⁵¹ bedeutet, worin R⁵¹ für Wasserstoff, C₁-C₄-Alkyl, C₁-C₄-Haloalkyl, C₁-C₄-Alkoxy oder C₁-C₄-Haloalkoxy steht;
R⁶ für C₁-C₄-Alkyl, C₁-C₄-Hydroxyalkyl oder C₁-C₄-Haloalkyl steht;
R⁷, R⁸ unabhängig voneinander für Wasserstoff, OH, C₁-C₄-Alkoxy, C₁-C₄- Haloalkyoxy, C₁-C₄-Alkyl oder C₁-C₄-Haloalkyl stehen;
R⁹, R¹⁰ unabhängig voneinander ausgewählt sind unter Wasserstoff, Halogen, CN, NO₂, C₁-C₄-Alkyl, C₁-C₄-Haloalkyl, C₂-C₄-Alkenyl, C₁-C₄-Alkoxy und C₁- C₄-Haloalkoxy; und
R¹¹ Wasserstoff oder C₁-C₄-Alkyl bedeutet;
sowie die landwirtschaftlich geeigneten Salze dieser Verbindungen.

2. Piperazinverbindungen gemäß Anspruch 1, worin R¹ für Cyano, Nitro oder einen 5- oder 6-gliedrigen heteroaromatischen Rest steht, der entweder 1, 2 oder 3 Stickstoffatome oder 1 Sauerstoff oder 1 Schwefelatom und gegebenenfalls 1 oder 2 Stickstoffatome als Ringlieder aufweist und der unsubstituiert ist oder 1 oder 2 unter R^{1a} ausgewählte Substituenten aufweisen kann.

3. Piperazinverbindungen gemäß Anspruch 1, worin R¹ für Halogen, insbesondere für Chlor oder Brom, steht.

4. Piperazinverbindungen gemäß einem der vorhergehenden Ansprüche, worin R⁴ für Methyl steht.

5. Piperazinverbindungen gemäß einem der vorhergehenden Ansprüche, worin R⁵ für Wasserstoff, Methyl oder Ethyl steht.

6. Piperazinverbindungen gemäß einem der Ansprüche 1 bis 5, worin R⁵ C(=O)R⁵¹ bedeutet, worin R⁵¹ für Wasserstoff, C₁-C₄-Alkyl oder C₁-C₄-Haloalkyl steht.

7. Piperazinverbindungen gemäß einem der vorhergehenden Ansprüche, worin R⁶ für Methyl oder Ethyl steht.

8. Piperazinverbindungen gemäß einem der vorhergehenden Ansprüche, worin R⁷ und R⁸ Wasserstoff bedeuten.

9. Piperazinverbindungen gemäß einem der vorhergehenden Ansprüche, worin R¹⁰ Wasserstoff bedeutet.

10. Piperazinverbindungen gemäß einem der vorhergehenden Ansprüche, worin R¹¹ Wasserstoff bedeutet.

11. Piperazinverbindungen gemäß einem der vorhergehenden Ansprüche der allgemeinen Formel la, worin R¹, R², R³, R⁴, R⁵, R⁶ und R⁹ eine der zuvor angegebenen Bedeutungen aufweisen: 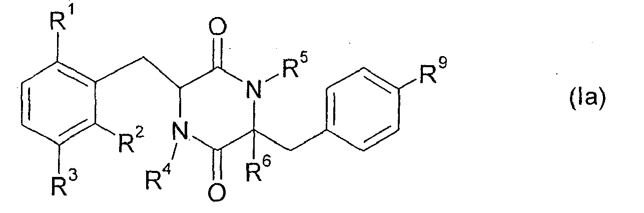 sowie die landwirtschaftlich geeigneten Salze dieser Verbindungen.

12. Piperazinverbindungen gemäß Anspruch 11, worin
R¹ für Cyano oder Nitro steht;
R² für Wasserstoff, Fluor, Chlor, C₁-C₂-Alkyl, Ethenyl oder C₁-C₂-Alkoxy steht;
R³ Fluor oder Wasserstoff bedeutet;
R⁴ für Methyl steht;
R⁵ für Wasserstoff, Methyl oder Ethyl steht;
R⁶ für Methyl oder Ethyl steht; und
R⁹ Wasserstoff oder Halogen bedeutet.

13. Piperazinverbindungen gemäß Anspruch 1, ausgewählt unter:
2-[5-Benzyl-1,4,5-trimethyl-3,6-dioxopiperazin-2-ylmethyl]-benzonitril,
2-[5-Benzyl-1,4,5-trimethyl-3,6-dioxopiperazin-2-ylmethyl]-3-fluorbenzonitril,
2-[5-Benzyl-1,4,5-trimethyl-3,6-dioxopiperazin-2-ylmethyl]-3-methoxybenzonitril,
2-[5-Benzyl-1,4,5-trimethyl-3,6-dioxopiperazin-2-ylmethyl]-3,4-difluorbenzonitril,
2-[5-Benzyl-1,4,5-trimethyl-3,6-dioxopiperazin-2-ylmethyl]-3-methylbenzonitril,
2-[5-Benzyl-1,4,5-trimethyl-3,6-dioxopiperazin-2-ylmethyl]-3-ethenylbenzonitril,
2-[5-Benzyl-1,5-dimethyl-3,6-dioxopiperazin-2-ylmethyl]-benzonitril,
2-[5-Benzyl-1,5-dimethyl-3,6-dioxopiperazin-2-ylmethyl]-3-fluorbenzonitril,
2-[5-Benzyl-1,5-dimethyl-3,6-dioxopiperazin-2-ylmethyl]-3-methoxybenzonitril,
2-[5-Benzyl-1,5-dimethyl-3,6-dioxopiperazin-2-ylmethyl]-3,4-difluorbenzonitril,
2-[5-Benzyl-1,5-dimethyl-3,6-dioxopiperazin-2-ylmethyl]-3-methylbenzonitril,
2-[5-Benzyl-1,5-dimethyl-3,6-dioxopiperazin-2-ylmethyl]-3-ethenylbenzonitril,
2-[5-Benzyl-5-ethyl-1,4-dimethyl-3,6-dioxopiperazin-2-ylmethyl]-benzonitril
2-[5-Benzyl-5-ethyl-1,4-dimethyl-3,6-dioxopiperazin-2-ylmethyl]-3-fluorbenzonitril
2-[5-Benzyl-5-ethyl-1,4-dimethyl-3,6-dioxopiperazin-2-ylmethyl]-3-methoxybenzonitril
2-[5-Benzyl-5-ethyl-1,4-dimethyl-3,6-dioxopiperazin-2-ylmethyl]-3,4-difluorbenzonitril,
2-[5-Benzyl-5-ethyl-1,4-dimethyl-3,6-dioxopiperazin-2-ylmethyl]-3-methylbenzonitril,
2-[5-Benzyl-5-ethyl-1,4-dimethyl-3,6-dioxopiperazin-2-ylmethyl]-3-ethenyl-benzonitril,
2-[5-Benzyl-5-ethyl-1-methyl-3,6-dioxopiperazin-2-ylmethyl]-benzonitril,
2-[5-Benzyl-5-ethyl-1-methyl-3,6-dioxopiperazin-2-ylmethyl]-3-fluorbenzonitril,
2-[5-Benzyl-5-ethyl-1-methyl-3,6-dioxopiperazin-2-ylmethyl]-3-methoxybenzonitril,
2-[5-Benzyl-5-ethyl-1-methyl-3,6-dioxopiperazin-2-ylmethyl]-3,4-difluorbenzonitril,
2-[5-Benzyl-5-ethyl-1-methyl-3,6-dioxopiperazin-2-ylmethyl]-3-methylbenzonitril,
2-[5-Benzyl-5-ethyl-1-methyl-3,6-dioxopiperazin-2-ylmethyl]-3-ethenylbenzonitril,
2-[5-(4-Fluorbenzyl)-1,4,5-trimethyl-3,6-dioxopiperazin-2-ylmethyl]-benzonitril,
2-[5-(4-Fluorbenzyl)-1,4,5-trimethyl-3,6-dioxopiperazin-2-ylmethyl]-3-fluorbenzonitril,
2-[5-(4-Fluorbenzyl)-1,4,5-trimethyl-3,6-dioxopiperazin-2-ylmethyl]-3-methoxybenzonitril,
2-[5-(4-Fluorbenzyl)-1,4,5-trimethyl-3,6-dioxopiperazin-2-ylmethyl]-3,4-difluorbenzonitril,
2-[5-(4-Fluorbenzyl)-1,4,5-trimethyl-3,6-dioxopiperazin-2-ylmethyl]-3-methylbenzonitril,
2-[5-(4-Fluorbenzyl)-1,4,5-trimethyl-3,6-dioxopiperazin-2-ylmethyl]-3-ethenylbenzonitril,
2-[5-(4-Fluorbenzyl)-1,5-dimethyl-3,6-dioxopiperazin-2-ylmethyl]-benzonitril,
2-[5-(4-Fluorbenzyl)-1,5-dimethyl-3,6-dioxopiperazin-2-ylmethyl]-3-fluorbenzonitril,
2-[5-(4-Fluorbenzyl)-1,5-dimethyl-3,6-dioxopiperazin-2-ylmethyl]-3-methoxybenzonitril,
2-[5-(4-Fluorbenzyl)-1,5-dimethyl-3,6-dioxopiperazin-2-ylmethyl]-3,4-difluorbenzonitril,
2-[5-(4-Fluorbenzyl)-1,5-dimethyl-3,6-dioxopiperazin-2-ylmethyl]-3-methylbenzonitril,
2-[5-(4-Fluorbenzyl)-1,5-dimethyl-3,6-dioxopiperazin-2-ylmethyl]-3-ethenylbenzonitril,
2-[5-(4-Fluorbenzyl)-5-ethyl-1,4-dimethyl-3, 6-dioxopiperazin-2-ylmethyl]-benzonitril
2-[5-(4-Fluorbenzyl)-5-ethyl-1,4-dimethyl-3,6-dioxopiperazin-2-ylmethyl]-3-fluorbenzonitril
2-[5-(4-Fluorbenzyl)-5-ethyl-1,4-dimethyl-3,6-dioxopiperazin-2-ylmethyl]-3-methoxybenzonitril
2-[5-(4-Fluorbenzyl)-5-ethyl-1,4-dimethyl-3, 6-dioxopiperazin-2-ylmethyl]-3,4-difluorbenzonitril,
2-[5-(4-Fluorbenzyl)-5-ethyl-1,4-dimethyl-3,6-dioxopiperazin-2-ylmethyl]-3-methylbenzonitril,
2-[5-(4-Fluorbenzyl)-5-ethyl-1,4-dimethyl-3,6-dioxopiperazin-2-ylmethyl]-3-ethenylbenzonitril,
2-[5-(4-Fluorbenzyl)-5-ethyl-1-methyl-3,6-dioxopiperazin-2-ylmethyl]-benzonitril,
2-[5-(4-Fluorbenzyl)-5-ethyl-1-methyl-3,6-dioxopiperazin-2-ylmethyl]-3-fluorbenzonitril,
2-[5-(4-Fluorbenzyl)-5-ethyl-1-methyl-3,6-dioxopiperazin-2-ylmethyl]-3-methoxybenzonitril,
2-[5-(4-Fluorbenzyl)-5-ethyl-1-methyl-3,6-dioxopiperazin-2-ylmethyl]-3,4-difluorbenzonitril,
2-[5-(4-Fluorbenzyl)-5-ethyl-1-methyl-3,6-dioxopiperazin-2-ylmethyl]-3-methylbenzonitril,
2-[5-(4-Fluorbenzyl)-5-ethyl-1-methyl-3,6-dioxopiperazin-2-ylmethyl]-3-ethenylbenzonitril,
3-Benzyl-6-(2-nitrobenzyl)-1,3,4-trimethylpiperazin-2,5-dion,
3-Benzyl-6-(2-fluor-6-nitrobenzyl)-1,3,4-trimethylpiperazin-2,5-dion,
3-Benzyl-6-(2,3-difluor-6-nitrobenzyl)-1,3,4-trimethylpiperazin-2,5-dion,
3-Benzyl-6-(2-methoxy-6-nitrobenzyl)-1,3,4-trimethylpiperazin-2,5-dion,
3-Benzyl-6-(2-methyl-6-nitrobenzyl)-1,3,4-trimethylpiperazin-2,5-dion,
3-Benzyl-6-(2-ethenyl-6-nitrobenzyl)-1,3,4-trimethylpiperazin-2,5-dion,
3-Benzyl-6-(2-nitrobenzyl)-1,3-dimethylpiperazin-2,5-dion,
3-Benzyl-6-(2-fluor-6-nitrobenzyl)-1,3-dimethylpiperazin-2,5-dion,
3-Benzyl-6-(2,3-difluor-6-nitrobenzyl)-1,3-dimethylpiperazin-2,5-dion,
3-Benzyl-6-(2-methoxy-6-nitrobenzyl)-1,3-dimethylpiperazin-2,5-dion,
3-Benzyl-6-(2-methyl-6-nitrobenzyl)-1,3-dimethylpiperazin-2,5-dion,
3-Benzyl-6-(2-ethenyl-6-nitrobenzyl)-1,3-dimethylpiperazin-2,5-dion,
3-Benzyl-6-(2-nitrobenzyl)-3-ethyl-1,4-dimethylpiperazin-2,5-dion,
3-Benzyl-6-(2-fluor-6-nitrobenzyl)-3-ethyl-1,4-dimethylpiperazin-2,5-dion,
3-Benzyl-6-(2,3-difluor-6-nitrobenzyl)-3-ethyl-1,4-dimethylpiperazin-2,5-dion,
3-Benzyl-6-(2-methoxy-6-nitrobenzyl)-3-ethyl-1,4-dimethylpiperazin-2,5-dion,
3-Benzyl-6-(2-methyl-6-nitrobenzyl)-3-ethyl-1,4-dimethylpiperazin-2,5-dion,
3-Benzyl-6-(2-ethenyl-6-nitrobenzyl)-3-ethyl-1,4-dimethylpiperazin-2,5-dion,
3-Benzyl-6-(2-nitrobenzyl)-3-ethyl-1-methylpiperazin-2,5-dion,
3-Benzyl-6-(2-fluor-6-nitrobenzyl)-3-ethyl-1-methylpiperazin-2,5-dion,
3-Benzyl-6-(2,3-difluor-6-nitrobenzyl)-3-ethyl-1-methylpiperazin-2,5-dion,
3-Benzyl-6-(2-methoxy-6-nitrobenzyl)-3-ethyl-1-methylpiperazin-2,5-dion,
3-Benzyl-6-(2-methyl-6-nitrobenzyl)-3-ethyl-1-methylpiperazin-2,5-dion,
3-Benzyl-6-(2-ethenyl-6-nitrobenzyl)-3-ethyl-1-methylpiperazin-2,5-dion,
3-(4-Fluorbenzyl)-6-(2-nitrobenzyl)-1,3,4-trimethylpiperazin-2,5-dion,
3-(4-Fluorbenzyl)-6-(2-fluor-6-nitrobenzyl)-1,3,4-trimethylpiperazin-2,5-dion,
3-(4-Fluorbenzyl)-6-(2,3-difluor-6-nitrobenzyl)-1,3,4-trimethylpiperazin-2,5-dion,
3-(4-Fluorbenzyl)-6-(2-methoxy-6-nitrobenzyl)-1,3,4-trimethylpiperazin-2,5-dion,
3-(4-Fluorbenzyl)-6-(2-methyl-6-nitrobenzyl)-1,3,4-trimethylpiperazin-2,5-dion,
3-(4-Fluorbenzyl)-6-(2-ethenyl-6-nitrobenzyl)-1,3,4-trimethylpiperazin-2,5-dion,
3-(4-Fluorbenzyl)-6-(2-nitrobenzyl)-1,3-dimethylpiperazin-2,5-dion,
3-(4-Fluorbenzyl)-6-(2-fluor-6-nitrobenzyl)-1,3-dimethylpiperazin-2,5-dion,
3-(4-Fluorbenzyl)-6-(2,3-difluor-6-nitrobenzyl)-1,3-dimethylpiperazin-2,5-dion,
3-(4-Fluorbenzyl)-6-(2-methoxy-6-nitrobenzyl)-1,3-dimethylpiperazin-2,5-dion,
3-(4-Fluorbenzyl)-6-(2-methyl-6-nitrobenzyl)-1,3-dimethylpiperazin-2,5-dion,
3-(4-Fluorbenzyl)-6-(2-ethenyl-6-nitrobenzyl)-1,3-dimethylpiperazin-2,5-dion,
3-(4-Fluorbenzyl)-6-(2-nitrobenzyl)-3-ethyl-1,4-dimethylpiperazin-2,5-dion,
3-(4-Fluorbenzyl)-6-(2-fluor-6-nitrobenzyl)-3-ethyl-1,4-dimethylpiperazin-2,5-dion,
3-(4-Fluorbenzyl)-6-(2,3-difluor-6-nitrobenzyl)-3-ethyl-1,4-dimethylpiperazin-2,5-dion,
3-(4-Fluorbenzyl)-6-(2-methoxy-6-nitrobenzyl)-3-ethyl-1,4-dimethylpiperazin-2,5-dion,
3-(4-Fluorbenzyl)-6-(2-methyl-6-nitrobenzyl)-3-ethyl-1,4-dimethylpiperazin-2,5-dion,
3-(4-Fluorbenzyl)-6-(2-ethenyl-6-nitrobenzyl)-3-ethyl-1,4-dimethylpiperazin-2,5-dion,
3-(4-Fluorbenzyl)-6-(2-nitrobenzyl)-3-ethyl-1-methylpiperazin-2,5-dion,
3-(4-Fluorbenzyl)-6-(2-fluor-6-nitrobenzyl)-3-ethyl-1-methylpiperazin-2,5-dion,
3-(4-Fluorbenzyl)-6-(2,3-difluor-6-nitrobenzyl)-3-ethyl-1-methylpiperazin-2,5-dion,
3-(4-Fluorbenzyl)-6-(2-methoxy-6-nitrobenzyl)-3-ethyl-1-methylpiperazin-2,5-dion,
3-(4-Fluorbenzyl)-6-(2-methyl-6-nitrobenzyl)-3-ethyl-1-methylpiperazin-2,5-dion und
3-(4-Fluorbenzyl)-6-(2-ethenyl-6-nitrobenzyl)-3-ethyl-1-methylpiperazin-2,5-dion.

14. Verwendung von Piperazinverbindungen der Formel I bzw. la gemäß einem der Ansprüche 1 bis 13 und deren landwirtschaftlich geeignete Salze als Herbizide.

15. Mittel, enthaltend eine herbizid wirksame Menge mindestens einer Piperazinverbindung der Formel I bzw. la oder eines landwirtschaftlich geeigneten Salzes von I bzw. la nach einem der Ansprüche 1 bis 13 und für die Formulierung von Pflanzenschutzmitteln übliche Hilfsmittel.

16. Verfahren zur Bekämpfung von unerwünschtem Pflanzenwuchs, **dadurch gekennzeichnet, dass** man eine herbizid wirksame Menge mindestens einer Piperazinverbindung der Formel I bzw. la oder eines landwirtschaftlich brauchbaren Salzes von I bzw. la nach einem der Ansprüche 1 bis 13 auf Pflanzen, deren Samen und/oder deren Lebensraum einwirken lässt.

## Claims

1. A piperazine compound of the formula I 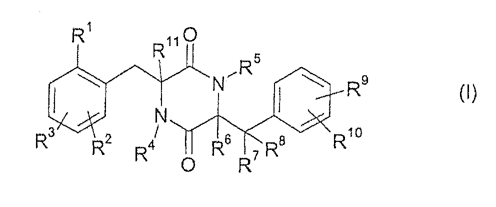 in which
R¹ is selected from the group consisting of halogen, cyano, nitro, Z- C(=O)-R¹², phenyl and a 5- or 6-membered heterocyclic radical which has 1, 2, 3 or 4 heteroatoms selected from the group consisting of O, N and S as ring atoms, where phenyl and the heterocyclic radical are unsubstituted or may have 1, 2, 3 or 4 substituents R^{1a} independently of one another selected from the group consisting of halogen, CN, NO₂, C₁-C₄-alkyl, C₁-C₄-haloalkyl, C₁-C₄-alkoxy and C₁-C₄-haloalkoxy, and in which
Z is a covalent bond or a CH₂ group;
R¹² is hydrogen, C₁-C₆-alkyl, C₃-C₆-cycloalkyl, C₂-C₆-alkenyl, C₅-C₆- cycloalkenyl, C₂-C₆-alkynyl, hydroxyl, C₁-C₆-alkoxy, C₃-C₆- alkenyloxy, C₃-C₆-alkynyloxy, amino, C₁-C₆-alkylamino, [di-(C₁- C₆)-alkyl]amino, C₁-C₆-alkoxyamino, C₁-C₆-alkylsulfonylamino, C₁-C₆-alkylaminosulfonylamino, [di-(C₁-C₆)- alkylamino]sulfonylamino, C₃-C₆-alkenylamino, C₃-C₆- alkynylamino, N-(C₂-C₆-alkenyl)-N-(C₁-C₆-alkyl)-amino, N-(C₂-C₆- alkynyl)-N-(C₁-C₆-alkyl)-amino, N-(C₁-C₆-alkoxy)-N-(C₁-C₆-alkyl)- amino, N-(C₂-C₆-alkenyl)-N-(C₁-C₆-alkoxy)-amino, N-(C₂-C₆- alkynyl)-N-(C₁-C₆-alkoxy)-amino, phenyl, phenoxy or phenylamino; where the alkyl moieties in the radicals listed under R¹² may be partially or fully halogenated and the phenyl moieties in the radicals listed under R¹² may carry 1, 2, 3 or 4 substituents R^{12a} selected from the group consisting of halogen, CN, NO₂, C₁-C₄-alkyl, C₁-C₄-haloalkyl, C₁-C₄-alkoxy and C₁-C₄-haloalkoxy;
R² is hydrogen, halogen, nitro, cyano, C₁-C₄-alkyl, C₁-C₄-haloalkyl, C₂-C₄- alkenyl, C₁-C₄-alkoxy, C₁-C₄-haloalkoxy, benzyl or a group S(O)ₙR²¹ in which R²¹ is C₁-C₄-alkyl or C₁-C₄-haloalkyl and n is 0, 1 or 2;
R³ is hydrogen or halogen;
R⁴ is C₁-C₄-alkyl, C₃-C₄-alkenyl or C₃-C₄-alkynyl;
R⁵ is hydrogen, C₁-C₄-alkyl, C₃-C₄-alkenyl, C₃-C₄-alkynyl or a group C(=O)R⁵¹ in which R⁵¹ is hydrogen, C₁-C₄-alkyl, C₁-C₄-haloalkyl, C₁-C₄- alkoxy or C₁-C₄-haloalkoxy;
R⁶ is C₁-C₄-alkyl, C₁-C₄-hydroxyalkyl or C₁-C₄-haloalkyl;
R⁷, R⁸ independently of one another are hydrogen, OH, C₁-C₄-alkoxy, C₁-C₄- haloalkyloxy, C₁-C₄-alkyl or C₁-C₄-haloalkyl;
R⁹, R¹⁰ independently of one another are selected from the group consisting of hydrogen, halogen, CN, NO₂, C₁-C₄-alkyl, C₁-C₄-haloalkyl, C₂-C₄- alkenyl, C₁-C₄-alkoxy and C₁-C₄-haloalkoxy; and
R¹¹ is hydrogen or C₁-C₄-alkyl;
or an agriculturally useful salt of this compound.

2. A piperazine compound according to claim 1 in which R¹ is cyano, nitro or a 5- or 6-membered heteroaromatic radical which has either 1, 2 or 3 nitrogen atoms or 1 oxygen or 1 sulfur atom and, if appropriate, 1 or 2 nitrogen atoms as ring members and which is unsubstituted or may have 1 or 2 substituents selected from R^{1a}.

3. A piperazine compound according to claim 1 in which R¹ is halogen, in particular chlorine or bromine.

4. A piperazine compound according to any of the preceding claims in which R⁴ is methyl.

5. A piperazine compound according to any of the preceding claims in which R⁵ is hydrogen, methyl or ethyl.

6. A piperazine compound according to any of claims 1 to 5 in which R⁵ is C(=O)R⁵¹ in which R⁵¹ is hydrogen, C₁-C₄-alkyl or C₁-C₄-haloalkyl.

7. A piperazine compound according to any of the preceding claims in which R⁶ is methyl or ethyl.

8. A piperazine compound according to any of the preceding claims in which R⁷ and R⁸ are hydrogen.

9. A piperazine compound according to any of the preceding claims in which R¹⁰ is hydrogen.

10. A piperazine compound according to any of the preceding claims in which R¹¹ is hydrogen.

11. A piperazine compound according to any of the preceding claims of the general formula la in which R¹,R², R³, R⁴, R⁵, R⁶ und R⁹ have one of the meanings given above: 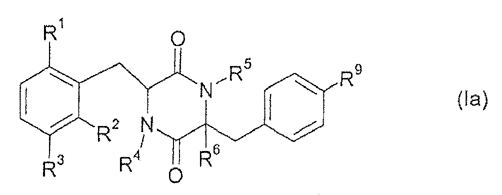 or an agriculturally useful salt of this compound.

12. A piperazine compound according to claim 11 in which
R¹ is cyano or nitro;
R² is hydrogen, fluorine, chlorine, C₁-C₂-alkyl, ethenyl or C₁-C₂-alkoxy;
R³ is fluorine or hydrogen;
R⁴ is methyl;
R⁵ is hydrogen, methyl or ethyl;
R⁶ is methyl or ethyl; and
R⁹ is hydrogen or halogen.

13. A piperazine compound according to claim 1, selected from the group consisting of:
2-[5-benzyl-1,4,5-trimethyl-3,6-dioxopiperazin-2-ylmethyl]benzonitrile,
2-[5-benzyl-1,4,5-trimethyl-3,6-dioxopiperazin-2-ylmethyl]-3-fluorobenzonitrile,
2-[5-benzyl-1,4,5-trimethyl-3,6-dioxopiperazin-2-ylmethyl]-3-methoxybenzonitrile,
2-[5-benzyl-1,4,5-trimethyl-3,6-dioxopiperazin-2-ylmethyl]-3,4-difluorobenzonitrile,
2-[5-benzyl-1,4,5-trimethyl-3,6-dioxopiperazin-2-ylmethyl]-3-methylbenzonitrile,
2-[5-benzyl-1,4,5-trimethyl-3,6-dioxopiperazin-2-ylmethyl]-3-ethenylbenzonitrile,
2-[5-benzyl-1,5-dimethyl-3,6-dioxopiperazin-2-ylmethyl]benzonitrile,
2-[5-benzyl-1,5-dimethyl-3,6-dioxopiperazin-2-ylmethyl]-3-fluorobenzonitrile,
2-[5-benzyl-1,5-dimethyl-3,6-dioxopiperazin-2-ylmethyl]-3-methoxybenzonitrile,
2-[5-benzyl-1,5-dimethyl-3,6-dioxopiperazin-2-ylmethyl]-3,4-difluorobenzonitrile,
2-[5-benzyl-1,5-dimethyl-3,6-dioxopiperazin-2-ylmethyl]-3-methylbenzonitrile,
2-[5-benzyl-1,5-dimethyl-3,6-dioxopiperazin-2-ylmethyl]-3-ethenylbenzonitrile,
2-[5-benzyl-5-ethyl-1,4-dimethyl-3,6-dioxopiperazin-2-ylmethyl]benzonitrile,
2-[5-benzyl-5-ethyl-1,4-dimethyl-3,6-dioxopiperazin-2-ylmethyl]-3-fluorobenzonitrile,
2-[5-benzyl-5-ethyl-1,4-dimethyl-3,6-dioxopiperazin-2-ylmethyl]-3-methoxybenzonitrile,
2-[5-benzyl-5-ethyl-1,4-dimethyl-3,6-dioxopiperazin-2-ylmethyl]-3,4-difluorobenzonitrile,
2-[5-benzyl-5-ethyl-1,4-dimethyl-3,6-dioxopiperazin-2-ylmethyl]-3-methylbenzonitrile,
2-[5-benzyl-5-ethyl-1,4-dimethyl-3,6-dioxopiperazin-2-ylmethyl]-3-ethenylbenzonitrile,
2-[5-benzyl-5-ethyl-1-methyl-3,6-dioxopiperazin-2-ylmethyl]benzonitrile,
2-[5-benzyl-5-ethyl-1-methyl-3,6-dioxopiperazin-2-ylmethyl]-3-fluorobenzonitrile,
2-[5-benzyl-5-ethyl-1-methyl-3,6-dioxopiperazin-2-ylmethyl]-3-methoxybenzonitrile,
2-[5-benzyl-5-ethyl-1-methyl-3,6-dioxopiperazin-2-ylmethyl]-3,4-difluorobenzonitrile,
2-[5-benzyl-5-ethyl-1-methyl-3,6-dioxopiperazin-2-ylmethyl]-3-methylbenzonitrile,
2-[5-benzyl-5-ethyl-1-methyl-3,6-dioxopiperazin-2-ylmethyl]-3-ethenylbenzonitrile,
2-[5-(4-fluorobenzyl)-1,4,5-trimethyl-3,6-dioxopiperazin-2-ylmethyl]benzonitrile,
2-[5-(4-fluorobenzyl)-1,4,5-trimethyl-3,6-dioxopiperazin-2-ylmethyl]-3-fluorobenzonitrile,
2-[5-(4-fluorobenzyl)-1,4,5-trimethyl-3,6-dioxopiperazin-2-ylmethyl]-3-methoxybenzonitrile,
2-[5-(4-fluorobenzyl)-1,4,5-trimethyl-3,6-dioxopiperazin-2-ylmethyl]-3,4-difluorobenzonitrile,
2-[5-(4-fluorobenzyl)-1,4,5-trimethyl-3,6-dioxopiperazin-2-ylmethyl]-3-methylbenzonitrile,
2-[5-(4-fluorobenzyl)-1,4,5-trimethyl-3,6-dioxopiperazin-2-ylmethyl]-3-ethenylbenzonitrile,
2-[5-(4-fluorobenzyl)-1,5-dimethyl-3,6-dioxopiperazin-2-ylmethyl]benzonitrile,
2-[5-(4-fluorobenzyl)-1,5-dimethyl-3,6-dioxopiperazin-2-ylmethyl]-3-fluorobenzonitrile,
2-[5-(4-fluorobenzyl)-1,5-dimethyl-3,6-dioxopiperazin-2-ylmethyl]-3-methoxybenzonitrile,
2-[5-(4-fluorobenzyl)-1,5-dimethyl-3,6-dioxopiperazin-2-ylmethyl]-3,4-difluorobenzonitrile,
2-[5-(4-fluorobenzyl)-1,5-dimethyl-3,5-dioxopiperazin-2-ylmethyl]-3-methylbenzonitrile,
2-[5-(4-fluorobenzyl)-1,5-dimethyl-3,6-dioxopiperazin-2-ylmethyl]-3-ethenylbenzonitrile,
2-[5-(4-fluorobenzyl)-5-ethyl-1,4-dimethyl-3,6-dioxopiperazin-2-ylmethyl]-benzonitrile,
2-[5-(4-fluorobenzyl)-5-ethyl-1,4-dimethyl-3,6-dioxopiperazin-2-ylmethyl]-3-fluorobenzonitrile,
2-[5-(4-fluorobenzyl)-5-ethyl-1,4-dimethyl-3,6-dioxopiperazin-2-ylmethyl]-3-methoxybenzonitrile,
2-[5-(4-fluorobenzyl)-5-ethyl-1,4-dimethyl-3,6-dioxopiperazin-2-ylmethyl]-3,4-difluorobenzonitrile,
2-[5-(4-fluorobenzyl)-5-ethyl-1,4-dimethyl-3,6-dioxopiperazin-2-ylmethyl]-3-methylbenzonitrile,
2-[5-(4-fluorobenzyl)-5-ethyl-1,4-dimethyl-3,6-dioxopiperazin-2-ylmethyl]-3-ethenylbenzonitrile,
2-[5-(4-fluorobenzyl)-5-ethyl-1-methyl-3,6-dioxopiperazin-2-ylmethyl]benzonitrile,
2-[5-(4-fluorobenzyl)-5-ethyl-1-methyl-3,6-dioxopiperazin-2-ylmethyl]-3-fluorobenzonitrile,
2-[5-(4-fluorobenzyl)-5-ethyl-1-methyl-3,6-dioxopiperazin-2-ylmethyl]-3-methoxybenzonitrile,
2-[5-(4-fluorobenzyl)-5-ethyl-1-methyl-3,6-dioxopiperazin-2-ylmethyl]-3,4-difluorobenzonitrile,
2-[5-(4-fluorobenzyl)-5-ethyl-1-methyl-3,6-dioxopiperazin-2-ylmethyl]-3-methylbenzonitrile,
2-[5-(4-fluorobenzyl)-5-ethyl-1-methyl-3,6-dioxopiperazin-2-ylmethyl]-3-ethenylbenzonitrile,
3-benzyl-6-(2-nitrobenzyl)-1,3,4-trimethylpiperazine-2,5-dione,
3-benzyl-6-(2-fluoro-6-nitrobenzyl)-1,3,4-trimethylpiperazine-2,5-dione,
3-benzyl-6-(2,3-difluoro-6-nitrobenzyl)-1,3,4-trimethylpiperazine-2,5-dione,
3-benzyl-6-(2-methoxy-6-nitrobenzyl)-1,3,4-trimethylpiperazine-2,5-dione,
3-benzyl-6-(2-methyl-6-nitrobenzyl)-1,3,4-trimethylpiperazine-2,5-dione,
3-benzyl-6-(2-ethenyl-6-nitrobenzyl)-1,3,4-trimethylpiperazine-2,5-dione,
3-benzyl-6-(2-nitrobenzyl)-1,3-dimethylpiperazine-2,5-dione,
3-benzyl-6-(2-fluoro-6-nitrobenzyl)-1,3-dimethylpiperazine-2,5-dione,
3-benzyl-6-(2,3-difluoro-6-nitrobenzyl)-1,3-dimethylpiperazine-2,5-dione,
3-benzyl-6-(2-methoxy-6-nitrobenzyl)-1,3-dimethylpiperazine-2,5-dione,
3-benzyl-6-(2-methyl-6-nitrobenzyl)-1,3-dimethylpiperazine-2,5-dione,
3-benzyl-6-(2-ethenyl-6-nitrobenzyl)-1,3-dimethylpiperazine-2,5-dione,
3-benzyl-6-(2-nitrobenzyl)-3-ethyl-1,4-dimethylpiperazine-2,5-dione,
3-benzyl-6-(2-fluoro-6-nitrobenzyl)-3-ethyl-1,4-dimethylpiperazine-2,5-dione,
3-benzyl-6-(2,3-difluoro-6-nitrobenzyl)-3-ethyl-1,4-dimethylpiperazine-2,5-dione,
3-benzyt-6-(2-methoxy-6-nitrobenzyl)-3-ethyl-1,4-dimethylpiperazine-2,5-dione,
3-benzyl-6-(2-methyl-6-nitrobenzyl)-3-ethyl-1,4-dimethylpiperazine-2,5-dione,
3-benzyl-6-(2-ethenyl-6-nitrobenzyl)-3-ethyl-1,4-dimethylpiperazine-2,5-dione,
3-benzyl-6-(2-nitrobenzyl)-3-ethyl-1-methylpiperazine-2,5-dione,
3-benzyl-6-(2-fluoro-6-nitrobenzyl)-3-ethyl-1-methylpiperazine-2,5-dione,
3-benzyl-6-(2,3-difluoro-6-nitrobenzyl)-3-ethyl-1-methylpiperazine-2,5-dione,
3-benzyl-6-(2-methoxy-6-nitrobenzyl)-3-ethyl-1-methylpiperazine-2,5-dione,
3-benzyl-6-(2-methyl-6-nitrobenzyl)-3-ethyl-1-methylpiperazine-2,5-dione,
3-benzyl-6-(2-ethenyl-6-nitrobenzyl)-3-ethyl-1-methylpiperazine-2,5-dione,
3-(4-fluorobenzyl)-6-(2-nitrobenzyl)-1,3,4-trimethylpiperazine-2,5-dione,
3-(4-fluorobenzyl)-6-(2-fluoro-6-nitrobenzyl)-1,3,4-trimethylpiperazine-2,5-dione,
3-(4-fluorobenzyl)-6-(2,3-difluoro-6-nitrobenzyl)-1,3,4-trimethylpiperazine-2,5-dione,
3-(4-fluorobenzyl)-6-(2-methoxy-6-nitrobenzyl)-1,3,4-trimethylpiperazine-2,5-dione,
3-(4-fluorobenzyl)-6-(2-methyl-6-nitrobenzyl)-1,3,4-trimethylpiperazine-2,5-dione,
3-(4-fluorobenzyl)-6-(2-ethenyl-6-nitrobenzyl)-1,3,4-trimethylpiperazine-2,5-dione,
3-(4-fluorobenzyl)-6-(2-nitrobenzyl)-1,3-dimethylpiperazine-2,5-dione,
3-(4-fluorobenzyl)-6-(2-fluoro-6-nitrobenzyl)-1,3-dimethylpiperazine-2,5-dione,
3-(4-fluorobenzyl)-6-(2,3-difluoro-6-nitrobenzyl)-1,3-dimethylpiperazine-2,5-dione,
3-(4-fluorobenzyl)-6-(2-methoxy-6-nitrobenzyl)-1,3-dimethylpiperazine-2,5-dione,
3-(4-fluorobenzyl)-6-(2-methyl-6-nitrobenzyl)-1,3-dimethylpiperazine-2,5-dione,
3-(4-fluorobenzyl)-6-(2-ethenyl-6-nitrobenzyl)-1,3-dimethylpiperazine-2,5-dione,
3-(4-fluorobenzyl)-6-(2-nitrobenzyl)-3-ethyl-1,4-dimethylpiperazine-2,5-dione,
3-(4-fluorobenzyl)-6-(2-fluoro-6-nitrobenzyl)-3-ethyl-1,4-dimethylpiperazine-2,5-dione,
3-(4-fluorobenzyl)-6-(2,3-difluoro-6-nitrobenzyl)-3-ethyl-1,4-dimethylpiperazine-2,5-dione,
3-(4-fluorobenzyl)-6-(2-methoxy-6-nitrobenzyl)-3-ethyl-1,4-dimethylpiperazine-2,5-dione,
3-(4-fluorobenzyl)-6-(2-methyl-6-nitrobenzyl)-3-ethyl-1,4-dimethylpiperazine-2,5-dione,
3-(4-fluorobenzyl)-6-(2-ethenyl-6-nitrobenzyl)-3-ethyl-1,4-dimethylpiperazine-2,5-dione,
3-(4-fluorobenzyl)-6-(2-nitrobenzyl)-3-ethyl-1-methylpiperazine-2,5-dione,
3-(4-fl uorobenzyl)-6-(2-fluoro-6-nitrobenzyl)-3-ethyl-1-methylpiperazine-2, 5-dione,
3-(4-fluorobenzyl)-6-(2,3-difluoro-6-nitrobenzyl)-3-ethyl-1-methylpiperazine-2,5-dione,
3-(4-fluorobenzyl)-6-(2-methoxy-6-nitrobenzyl)-3-ethyl-1-methylpiperazine-2,5-dione,
3-(4-fluorobenzyl)-6-(2-methyl-6-nitrobenzyl)-3-ethyl-1-methylpiperazine-2,5-dione, and
3-(4-fluorobenzyl)-6-(2-ethenyl-6-nitrobenzyl)-3-ethyl-1-methylpiperazine-2,5-dione.

14. The use of a piperazine compound of the formula I or la according to any of claims 1 to 13 or an agriculturally useful salt thereof as a herbicide.

15. A composition comprising a herbicidally effective amount of at least one piperazine compound of the formula I or la or an agriculturally useful salt of I or la according to any of claims 1 to 13 and auxiliaries customary for formulating crop protection agents.

16. A method for controlling unwanted vegetation wherein a herbicidally effective amount of at least one piperazine compound of the formula I or la or an agriculturally useful salt of I or la according to any of claims 1 to 13 is allowed to act on plants, their seed and/or their habitat.

## Revendications

1. Composés de pipérazine de formule I 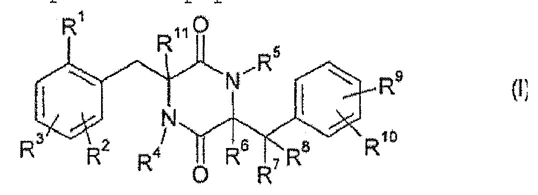 où
R¹ est choisi parmi halogène, cyano, nitro, Z-C(=O)- R¹², phényle et un radical hétérocyclique de 5 ou 6 chaînons, qui présente 1, 2, 3 ou 4 hétéroatomes, choisis parmi O, N et S comme atomes de cycle, où phényle et le radical hétérocyclique sont non substitués ou peuvent présenter 1, 2, 3 ou 4 substituants R^{1a} qui sont choisis, indépendamment l'un de l'autre, parmi halogène, CN, NO₂, C₁-C₄- alkyle, C₁-C₄-halogénoalkyle, C₁-C₄-alcoxy et C₁-C₄- halogénoalcoxy, et où
Z représente une liaison covalente ou un groupe CH₂ ;
R¹² signifie hydrogène, C₁-C₆-alkyle, C₃-C₆-cycloalkyle, C₂-C₆-alcényle, C₅-C₆-cycloalcényle, C₂-C₆-alcynyle, hydroxy, C₁-C₆-alcoxy, C₃-C₆-alcényloxy, C₃-C₆- alcynyloxy, amino, C₁-C₆-alkylamino, [di-(C₁-C₆)- alkyl]amino, C₁-C₆-alcoxyamino, C₁-C₆- alkylsulfonylamino, C₁-C₆-alkylaminosulfonylamino, [di- (C₁-C₆) -alkylamino] sulfonylamino, C₃-C₆- alcénylamino, C₃-C₆-alcynylamino, N-(C₂-C₆-alcényl)- N- (C₁-C₆-alkyl) -amino, N-(C₂-C₆-alcynyl) -N-(C₁-C₆- alkyl)-amino, N-(C₁-C₆-alcoxy) -N- (C₁-C₆-alkyl) - amino, N-(C₂-C₆-alcényl)-N-(C₁C₆-alcoxy)-amino, N- (C₂C₆-alcynyl)-N-(C₁-C₆-alcoxy)-amino, phényle, phénoxy ou phénylamino ; où les parties alkyle dans les radicaux indiqués pour R¹² peuvent être partiellement ou totalement halogénés et les parties phényle dans les radicaux indiqués pour R¹²peuvent porter 1, 2, 3 ou 4 substituants R^{12a}, qui sont choisis parmi halogène, CN, NO₂, C₁-C₄-alkyle, C₁-C₄-halogénoalkyle, C₁-C₄- alcoxy et C₁-C₄-halogénoalcoxy ;
R² signifie hydrogène, halogène, nitro, cyano, C₁-C₄- alkyle, C₁-C₄-halogénoalkyle, C₂-C₄-alcényle, C₁-C₄- alcoxy, C₁-C₄-halogénoalcoxy, benzyle ou un groupe S (O) ₙR²¹, où R²¹ représente _{C1}-_{C4}-alkyle ou C₁-C₄- halogénoalkyle et n vaut 0, 1 ou 2 ;
R³ signifie hydrogène ou halogène ;
R⁴ signifie C₁-C₄-alkyle, C₃-C₄-alcényle ou C₃-C₄- alcynyle ;
R⁵ signifie hydrogène, C₁-C₄-alkyle, C₃-C₄-alcényle, C₃-C₄-alcynyle ou un groupe C(=O)R⁵¹, où R⁵¹ représente hydrogène, C₁-C₄-alkyle, C₁-C₄- halogénoalkyle, C₁-C₄-alcoxy ou C₁-C₄- halogénoalcoxy ;
R⁶ représente C₁-C₄-alkyle, C₁-C₄-hydroxyalkyle ou C₁- C₄-halogénoalkyle ;
R⁷, R⁸ représentent, indépendamment l'un de l'autre, hydrogène, OH, C₁-C₄-alcoxy, C₁-C₄-halogénoalkyloxy, C₁-C₄-alkyle ou C₁-C₄-halogénoalkyle ;
R⁹, R¹⁰ sont choisis indépendamment l'un de l'autre parmi hydrogène, halogène, CN, NO₂, C₁-C₄-alkyle, C₁-C₄-halogénoalkyle, C₂-C₄-alcényle, C₁-C₄-alcoxy et C₁-C₄-halogénoalcoxy ; et
R¹¹ signifie hydrogène ou C₁-C₄-alkyle ;
ainsi que les sels appropriés en agriculture de ces composés.

2. Composés de pipérazine selon la revendication 1, où R¹ représente cyano, nitro ou un radical hétéroaromatique de 5 ou 6 chaînons, qui contient soit 1, 2 ou 3 atomes d'azote, soit 1 atome d'oxygène soit 1 atome de soufre et le cas échéant 1 ou 2 atomes d'azote comme chaînons de cycle et qui est non substitué ou qui peut présenter 1 ou 2 substituants choisis parmi R^{1a}.

3. Composés de pipérazine selon la revendication 1, où R¹ représente halogène, en particulier chlore ou brome.

4. Composés de pipérazine selon l'une quelconque des revendications précédentes, où R⁴ représente méthyle.

5. Composés de pipérazine selon l'une quelconque des revendications précédentes, où R⁵ représente hydrogène, méthyle ou éthyle.

6. Composés de pipérazine selon l'une quelconque des revendications 1 à 5, où R⁵ signifie C(=O)R⁵¹, où R⁵¹ représente hydrogène, C₁-C₄-alkyle ou C₁-C₄-halogénoalkyle.

7. Composés de pipérazine selon l'une quelconque des revendications précédentes, où R⁶ représente méthyle ou éthyle.

8. Composés de pipérazine selon l'une quelconque des revendications précédentes, où R⁷ et R⁸ signifient hydrogène.

9. Composés de pipérazine selon l'une quelconque des revendications précédentes, où R¹⁰ signifie hydrogène.

10. Composés de pipérazine selon l'une quelconque des revendications précédentes, où R¹¹ signifie hydrogène.

11. Composés de pipérazine selon l'une quelconque des revendications précédentes de formule générale Ia, où R¹, R² , R³ , R⁴ , R⁵ , R⁶ et R⁹ présentent une des significations indiquées ci-dessus : 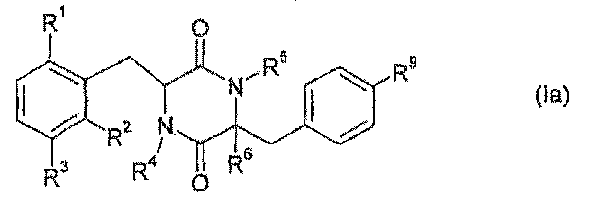 ainsi que les sels appropriés en agriculture de ces composés.

12. Composés de pipérazine selon la revendication 11, où
R¹ représente cyano ou nitro ;
R² représente hydrogène, fluor, chlore, C₁-C₂-alkyle, éthényle ou C₁-C₂-alcoxy ;
R³ signifie fluor ou hydrogène ;
R⁴ représente méthyle ;
R⁵ représente hydrogène, méthyle ou éthyle ;
R⁶ représente méthyle ou éthyle ; et
R⁹ signifie hydrogène ou halogène.

13. Composés de pipérazine selon la revendication 1, choisis parmi :
le 2-[5-benzyl-1,4,5-triméthyl-3,6-dioxopipérazin-2-ylméthyl]-benzonitrile,
le 2-[5-benzyl-1,4,5-triméthyl-3,6-dioxopipérazin-2-ylméthyl]-3-fluorobenzonitrile,
le 2-[5-benzyl-1,4,5-triméthyl-3,6-dioxopipérazin-2-ylméthyl]-3-méthoxybenzonitrile,
le 2-[5-benzyl-1,4,5-triméthyl-3,6-dioxopipérazin-2-ylméthyl]-3,4-difluorobenzonitrile,
le 2-[5-benzyl-1,4,5-triméthyl-3,6-dioxopipérazin-2-ylméthyl]-3-méthylbenzonitrile,
le 2-[5-benzyl-1,4,5-triméthyl-3,6-dioxopipérazin-2-ylméthyl]-3-éthénylbenzonitrile,
le 2-[5-benzyl-1,5-diméthyl-3,6-dioxopipérazin-2-ylméthyl]-benzonitrile,
le 2-[5-benzyl-1,5-diméthyl-3,6-dioxopipérazin-2-ylméthyl]-3-fluorobenzonitrile,
le 2-[5-benzyl-1,5-diméthyl-3,6-dioxopipérazin-2-ylméthyl]-3-méthoxybenzonitrile,
le 2-[5-benzyl-1,5-diméthyl-3,6-dioxopipérazin-2-ylméthyl]-3,4-difluorobenzonitrile,
le 2-[5-benzyl-1,5-diméthyl-3,6-dioxopipérazin-2-ylméthyl]-3-méthylbenzonitrile,
le 2-[5-benzyl-1,5-diméthyl-3,6-dioxopipérazin-2-ylméthyl]-3-éthénylbenzonitrile,
le 2-[5-benzyl-5-éthyl-1,4-diméthyl-3,6-dioxopipérazin-2-ylméthyl]-benzonitrile,
le 2-[5-benzyl-5-éthyl-1,4-diméthyl-3,6-dioxopipérazin-2-ylméthyl]-3-fluorobenzonitrile,
le 2-[5-benzyl-5-éthyl-1,4-diméthyl-3,6-dioxopipérazin-2-ylméthyl]-3-méthoxybenzonitrile,
le 2-[5-benzyl-5-éthyl-1,4-diméthyl-3,6-dioxopipérazin-2-ylméthyl]-3,4-difluorobenzonitrile,
le 2-[5-benzyl-5-éthyl-1,4-diméthyl-3,6-dioxopipérazin-2-ylméthyl]-3-méthylbenzonitrile,
le 2-[5-benzyl-5-éthyl-1,4-diméthyl-3,6-dioxopipérazin-2-ylméthyl]-3-éthénylbenzonitrile,
le 2-[5-benzyl-5-éthyl-1-méthyl-3,6-dioxopipérazin-2-ylméthyll-benzonitrile,
le 2-[5-benzyl-5-éthyl-1-méthyl-3,6-dioxopipérazin-2-ylméthyl]-3-fluorobenzonitrile,
le 2-[5-benzyl-5-éthyl-1-méthyl-3,6-dioxopipérazin-2-ylméthyl]-3-méthoxybenzonitrile,
le 2-[5-benzyl-5-éthyl-1-méthyl-3,6-dioxopipérazin-2-ylméthyl]-3,4-difluorobenzonitrile,
le 2-[5-benzyl-5-éthyl-1-méthyl-3,6-dioxopipérazin-2-ylméthyl]-3-méthylbenzonitrile,
le 2-[5-benzyl-5-éthyl-l-méthyl-3,6-dioxopipérazin-2-ylméthyl]-3-éthénylbenzonitrile,
le 2-[5-(4-fluorobenzyl)-1,4,5-triméthyl-3,6-dioxopipérazin-2-ylméthyl]-benzonitrile,
le 2-[5-(4-fluorobenzyl)-1,4,5-triméthyl-3,6-dioxopipérazin-2-ylméthyl]-3-fluorobenzonitrile,
le 2-[5-(4-fluorobenzyl)-1,4,5-triméthyl-3,6-dioxopipérazin-2-ylméthyl]-3-méthoxybenzonitrile,
le 2-[5-(4-fluorobenzyl)-1,4,5-triméthyl-3,6-dioxopipérazin-2-ylméthyl]-3,4-difluorobenzonitrile,
le 2-[5-(4-fluorobenzyl)-1,4,5-triméthyl-3,6-dioxopipérazin-2-ylméthyl]-3-méthylbenzonitrile,
le 2-[5-(4-fluorobenzyl)-1,4,5-triméthyl-3,6-dioxopipérazin-2-ylméthyl]-3-éthénylbenzonitrile,
le 2-[5-(4-fluorobenzyl)-1,5-diméthyl-3,6-dioxopipérazin-2-ylméthyl]-benzonitrile,
le 2-[5-(4-fluorobenzyl)-1,5-diméthyl-3,6-dioxopipérazin-2-ylméthyl]-3-fluorobenzonitrile,
le 2-[5-(4-fluorobenzyl)-1,5-diméthyl-3,6-dioxopipérazin-2-ylméthyl]-3-méthoxybenzonitrile,
le 2-[5-(4-fluorobenzyl)-1,5-diméthyl-3,6-dioxopipérazin-2-ylméthyl]-3,4-difluorobenzonitrile,
le 2-[5-(4-fluorobenzyl)-1,5-diméthyl-3,6-dioxopipérazin-2-ylméthyl]-3-méthylbenzonitrile,
le 2-[5-(4-fluorobenzyl)-1,5-diméthyl-3,6-dioxopipérazin-2-ylméthyl]₋3-éthénylbenzonitrile,
le 2-[5-(4-fluorobenzyl)-5-éthyl-1,4-diméthyl-3,6-dioxopipérazin-2-ylméthyl]-benzonitrile,
le 2-[5-(4-fluorobenzyl)-5-éthyl-1,4-diméthyl-3,6-dioxopipérazin-2-ylméthyl]-3-fluorobenzonitrile,
le 2-[5-(4-fluorobenzyl)-5-éthyl-1,4-diméthyl-3,6-dioxopipérazin-2-ylméthyl]-3-méthoxybenzonitrile,
le 2-[5-(4-fluorobenzyl)-5-éthyl-1,4-diméthyl-3,6-dioxopipérazin-2-ylméthyl]-3,4-difluorobenzonitrile,
le 2-[5-(4-fluorobenzyl)-5-éthyl-1,4-diméthyl-3,6-dioxopipérazin-2-ylméthyl]-3-méthylbenzonitrile,
le 2-[5-(4-fluorobenzyl)-5-éthyl-1,4-diméthyl-3,6-dioxopipérazin-2-ylméthyl]-3-éthénylbenzonitrile,
le 2-[5-(4-fluorobenzyl)-5-éthyl-1-méthyl-3,6-dioxopipérazin-2-ylméthyl]-benzonitrile,
le 2-[5-(4-fluorobenzyl)-5-éthyl-1-méthyl-3,6-dioxopipérazin-2-ylméthyl]-3-fluorobenzonitrile,
le 2-[5-(4-fluorobenzyl)-5-éthyl-1-méthyl-3,6-dioxopipérazin-2-ylméthyl]-3-méthoxybenzonitrile,
le 2-[5-(4-fluorobenzyl)-5-éthyl-1-méthyl-3,6-dioxopipérazin-2-ylméthyl]-3,4-difluorobenzonitrile,
le 2-[5-(4-fluorobenzyl)-5-éthyl-1-méthyl-3,6-dioxopipérazin-2-ylméthyl]-3-méthylbenzonitrile,
le 2-[5-(4-fluorobenzyl)-5-éthyl-1-méthyl-3,6-dioxopipérazin-2-ylméthyl]-3-éthénylbenzonitrile,
la 3-benzyl-6-(2-nitrobenzyl)-1,3,4-triméthylpipérazine-2,5-dione,
la 3-benzyl-6-(2-fluoro-6-nitrobenzyl)-1,3,4-triméthylpipérazine-2,5-dione,
la 3-benzyl-6-(2,3-difluoro-6-nitrobenzyl)-1,3,4-triméthylpipérazine-2,5-dione,
la 3-benzyl-6-(2-méthoxy-6-nitrobenzyl)-1,3,4-triméthylpipérazine-2,5-dione,
la 3-benzyl-6-(2-méthyl-6-nitrobenzyl)-1,3,4-triméthylpipérazine-2,5-dione,
la 3-benzyl-6-(2-éthényl-6-nitrobenzyl)-1,3,4-triméthylpipérazine-2,5-dione,
la 3-benzyl-6-(2-nitrobenzyl)-1,3-diméthylpipérazine-2,5-dione,
la 3-benzyl-6-(2-fluoro-6-nitrobenzyl)-1,3-diméthylpipérazine-2,5-dione,
la 3-benzyl-6-(2,3-difluoro-6-nitrobenzyl)-1,3-diméthylpipérazine-2,5-dione,
la 3-benzyl-6-(2-méthoxy-6-nitrobenzyl)-1,3-diméthylpipérazine-2,5-dione,
la 3-benzyl-6-(2-méthyl-6-nitrobenzyl)-1,3-diméthylpipérazine-2,5-dione,
la 3-benzyl-6-(2-éthényl-6-nitrobenzyl)-1,3-diméthylpipérazine-2,5-dione,
la 3-benzyl-6-(2-nitrobenzyl)-3-éthyl-1,4-diméthylpipérazine-2,5-dione,
la 3-benzyl-6-(2-fluoro-6-nitrobenzyl)-3-éthyl-1,4-diméthylpipérazine-2,5-dione,
la 3-benzyl-6-(2,3-difluoro-6-nitrobenzyl)-3-éthyl-1,4-diméthylpipérazine-2,5-dione,
la 3-benzyl-6-(2-méthoxy-6-nitrobenzyl)-3-éthyl-1,4-diméthylpipérazine-2,5-dione,
la 3-benzyl-6-(2-méthyl-6-nitrobenzyl)-3-éthyl-1,4-diméthylpipérazine-2,5-dione,
la 3-benzyl-6-(2-éthényl-6-nitrobenzyl)-3-éthyl-1,4-diméthylpipérazine-2,5-dione,
la 3-benzyl-6-(2-nitrobenzyl)-3-éthyl-1-méthylpipérazine-2,5-dione,
la 3-benzyl-6-(2-fluoro-6-nitrobenzyl)-3-éthyl-1-méthylpipérazine-2,5-dione,
la 3-benzyl-6-(2,3-difluoro-6-nitrobenzyl)-3-éthyl-1-méthylpipérazine-2,5-dione,
la 3-benzyl-6-(2-méthoxy-6-nitrobenzyl)-3-éthyl-1-méthylpipérazine-2,5-dione,
la 3-benzyl-6-(2-méthyl-6-nitrobenzyl)-3-éthyl-1-méthylpipérazine-2,5-dione,
la 3-benzyl-6-(2-éthényl-6-nitrobenzyl)-3-éthyl-1-méthylpipérazine-2,5-dione,
la 3-(4-fluorobenzyl)-6-(2-nitrobenzyl)-1,3,4-triméthylpipérazine-2,5-dione,
la 3-(4-fluorobenzyl)-6-(2-fluoro-6-nitrobenzyl)-1,3,4-triméthylpipérazine-2,5-dione,
la 3-(4-fluorobenzyl)-6-(2,3-difluoro-6-nitrobenzyl)-1,3,4-triméthylpipérazine-2,5-dione,
la 3-(4-fluorobenzyl)-6-(2-méthoxy-6-nitrobenzyl)-1,3,4-triméthylpipérazine-2,5-dione,
la 3-(4-fluorobenzyl)-6-(2-méthyl-6-nitrobenzyl)-1,3,4-triméthylpipérazine-2,5-dione,
la 3-(4-fluorobenzyl)-6-(2-éthényl-6-nitrobenzyl)-1,3,4-triméthylpipérazine-2,5-dione,
la 3-(4-fluorobenzyl)-6-(2-nitrobenzyl)-1,3-diméthylpipérazine-2,5-dione,
la 3-(4-fluorobenzyl)-6-(2-fluoro-6-nitrobenzyl)-1,3-diméthylpipérazine-2,5-dione,
la 3-(4-fluorobenzyl)-6-(2,3-difluoro-6-nitrobenzyl)-1,3-diméthylpipérazine-2,5-dione,
la 3-(4-fluorobenzyl)-6-(2-méthoxy-6-nitrobenzyl)-1,3-diméthylpipérazine-2,5-dione,
la 3-(4-fluorobenzyl)-6-(2-méthyl-6-nitrobenzyl)-1,3-diméthylpipérazine-2,5-dione,
la 3-(4-fluorobenzyl)-6-(2-éthényl-6-nitrobenzyl)-1,3-diméthylpipérazine-2,5-dione,
la 3-(4-fluorobenzyl)-6-(2-nitrobenzyl)-3-éthyl-1,4-diméthylpipérazine-2,5-dione,
la 3-(4-fluorobenzyl)-6-(2-fluoro-6-nitrobenzyl)-3-éthyl-1,4-diméthylpipérazine-2,5-dione,
la 3-(4-fluorobenzyl)-6-(2,3-difluoro-6-nitrobenzyl)-3-éthyl-1,4-diméthylpipérazine-2,5-dione,
la 3-(4-fluorobenzyl)-6-(2-méthoxy-6-nitrobenzyl)-3-éthyl-1,4-diméthylpipérazine-2,5-dione,
3-(4-fluorobenzyl)-6-(2-méthyl-6-nitrobenzyl)-3-éthyl-1,4-diméthylpipérazine-2,5-dione,
la 3-(4-fluorobenzyl)-6-(2-éthényl-6-nitrobenzyl)-3-éthyl-1,4-diméthylpipérazine-2,5-dione,
la 3-(4-fluorobenzyl)-6-(2-nitrobenzyl)-3-éthyl-1-méthylpipérazine-2,5-dione,
la 3-(4-fluorobenzyl)-6-(2-fluoro-6-nitrobenzyl)-3-éthyl-1-méthylpipérazine-2,5-dione,
la 3-(4-fluorobenzyl)-6-(2,3-difluoro-6-nitrobenzyl)-3-éthyl-1-méthylpipérazine-2,5-dione,
la 3-(4-fluorobenzyl)-6-(2-méthoxy-6-nitrobenzyl)-3-éthyl-1-méthylpipérazine-2,5-dione,
la 3-(4-fluorobenzyl)-6-(2-méthyl-6-nitrobenzyl)-3-éthyl-1-méthylpipérazine-2,5-dione et
la 3-(4-fluorobenzyl)-6-(2-éthényl-6-nitrobenzyl)-3-éthyl-1-méthylpipérazine-2,5-dione.

14. Utilisation de composés de pipérazine de formule I ou Ia selon l'une quelconque des revendications 1 à 13 et leurs sels appropriés en agriculture comme herbicides.

15. Agent, contenant une quantité active en tant qu'herbicide d'au moins un composé de pipérazine de formule I ou Ia ou d'un sel approprié en agriculture de I ou la selon l'une quelconque des revendications 1 à 13 et des adjuvants usuels pour la formulation d'agents phytosanitaires.

16. Procédé pour lutter contre une croissance non souhaitée de plantes, **caractérisé en ce qu'**on laisse agir une quantité active en tant qu'herbicide d'au moins un composé de pipérazine de formule I ou la ou d'un sel utilisable en agriculture de I ou Ia selon l'une quelconque des revendications 1 à 13 sur des plantes, leurs graines et/ou leur espace de vie.
